(19) Europäisches Patentamt
European Patent Office
Office européen des brevets

(11) **EP 2 674 744 A1**

(12) **EUROPEAN PATENT APPLICATION**
published in accordance with Art. 153(4) EPC

(43) Date of publication:
**18.12.2013 Bulletin 2013/51**

(51) Int Cl.:
**G01N 21/27** *(2006.01)*     **B82Y 30/00** *(2011.01)*
**G01N 21/01** *(2006.01)*     **G01N 33/532** *(2006.01)*
**G01N 33/543** *(2006.01)*

(21) Application number: **12744603.7**

(22) Date of filing: **02.02.2012**

(86) International application number:
**PCT/JP2012/052331**

(87) International publication number:
**WO 2012/108322 (16.08.2012 Gazette 2012/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**09.02.2011    JP 2011025951**
**13.06.2011    JP 2011131610**
**13.09.2011    JP 2011199909**
**14.10.2011    JP 2011226818**
**19.01.2012    JP 2012008773**

(71) Applicant: **Nippon Steel & Sumikin Chemical Co., Ltd.**
**Chiyoda-ku**
**Tokyo 101-0021 (JP)**

(72) Inventors:
• **MATSUMURA Yasufumi**
  **Kisarazu-shi, Chiba 292-0835 (JP)**
• **ENOMOTO Yasushi**
  **Kisarazu-shi, Chiba 292-0835 (JP)**
• **SHINTA Ryuzo**
  **Kisarazu-shi, Chiba 292-0835 (JP)**

(74) Representative: **Becker Kurig Straus**
**Bavariastrasse 7**
**80336 München (DE)**

(54) **COMPOSITE HAVING METAL MICROPARTICLES DISPERSED THEREIN AND PROCESS FOR PRODUCTION THEREOF, AND SUBSTRATE CAPABLE OF GENERATING LOCALIZED SURFACE PLASMON RESONANCE**

(57)    A nanocomposite (10) comprises: a matrix layer (1) which comprises solid backbone parts (1a) and voids (1b) formed by the solid backbone parts (1a); and metal microparticles (3) which are fixed on the solid backbone parts (1a) of the matrix layer (1). The solid backbone parts (1a) comprise an aluminum oxyhydroxide or an alumina hydrate and form a three-dimensional network structure. In the metal microparticles (3), metal microparticles having an average particle diameter falling within the range of 3-100 nm and particle diameters falling within the range of 1-100 nm make up 60% or more of the metal microparticles (3). The metal microparticles (3) exist in such a manner that the metal microparticles (3) are not in contact with one another and any two adjacent metal microparticles (3) are apart from each other by a distance that is equal to or greater than the particle diameter ($D_L$) of one of the two adjacent metal microparticles (3) which has a larger particle diameter (D) than that of the other one. Each of the metal microparticles (3) has a part that is exposed to the voids (1b) of the matrix layer (1), and the metal microparticles (3) exist in a state dispersed three-dimensionally in the matrix layer (1).

FIG. 2

**Description**

TECHNICAL FIELD

The invention relates to a metal fine-particle dispersed composite, a method for fabricating the same, and a localized surface plasmon resonance inducing substrate. The metal fine-particle dispersed composite includes a matrix having a three-dimensional network structure, and metal fine-particles, and is applicable to, for example, various devices utilizing localized surface plasmon resonance.

BACKGROUND ART

Nanometer-sized fine-particles have a geometrically high specific surface area, and in addition, exhibit changes in optical properties, lowering of melting point, high catalytic properties, high magnetic properties and so on as a result of quantum size effects. Hence they are expected to offer new functions which could not be achieved with bulk materials, such as improvement of catalytic reaction, luminescence properties and other chemical and physical conversion characteristics, and have become a very important material in various fields such as electronic material, catalyst material, phosphor material, luminous body material, medical supplies and so on. In particularly, for metal fine-particles having a size of approximately several nm to 100 nm, there is a phenomenon called localized surface plasmon resonance (LSPR) in which electrons in the fine-particles interact and resonate with light of a specific wavelength. In recent years, application to various devices has been studied to take advantage of this phenomenon. This localized surface plasmon resonance is sensitive to variation in the dielectric constant $\varepsilon_m(\lambda)$ $[=(n_m(\lambda))^2]$ ($n_m$ is the refractive index) of the medium surrounding the metal fine-particles, and thus have a characteristic that the resonance wavelength varies with a variation in the dielectric constant (or refractive index) of the medium surrounding the metal fine-particles. Based on this characteristic, applications of LSPR in the field of sensing such as frost sensors, moisture sensors, bio-sensors and chemical sensors have been enthusiastically discussed.

In a case of applying the LSPR of metal fine-particles to a device, from viewpoints of handleability of the material, stability, diversity of the application field, and so on, it is necessary for the metal fine-particles to be immobilized to a support. However, the metal fine-particles are different from bulk metal in agglomeration and dispersion properties. Therefore, even if dispersed in an aqueous solution or an organic solvent as in a colloidal solution, when the metal fine-particles are to be immobilized to a support, aggregation occurs due to lowering of dispersion stability caused by electrostatic repulsion and so on, and LSPR is decreased in intensity, or disappears. In order to produce such material, special methods are used, and there were various problems such as low yield and low productivity in addition to requirement of costly equipment and complicated operations. Accordingly, there is demand for techniques capable of easily and inexpensively fixing metal fine-particles to a support while maintaining LSPR. In addition, to improve the device performance, a material providing high intensity of LSPR and capable of highly sensitively detecting the variation in the environment surrounding the metal fine-particles is strongly expected.

In regard to a metal fine-particle dispersed composite for solving the above problems, to further improve its performance, development of new technologies, such as those described in Patent Documents 1 and 2, has proceeded.

Patent Document 1 discloses a metal fine-particle dispersed composite including metal fine-particles that are mono-dispersed and immobilized to a glass substrate surface-modified by 3-aminopropyltrimethoxysilane. In addition, Patent Document 2 discloses a metal fine-particle layer including metal fine-particles that are regularly immobilized to a substrate composed of porous alumina having micropores result from anodic oxidation. In Patent Document 1, however, there is a problem that when the metal fine-particles are immobilized, the degree of dispersion varies due to variation in the concentration, so that in-plane variation becomes larger. Further, as the metal fine-particles are chemically immobilized on the glass substrate, their distribution on the substrate is likely to be non-uniform due to falling-off. In addition, in Patent Document 2, since the metal fine-particles are buried in the holes formed on the surface of the alumina, their portions related to variation in the medium surrounding the metal fine-particles is small so that it is impossible to sensitively detect the variation in the surrounding environment. Further, in the technologies of Patent Documents 1 and 2, since the metal fine-particles are immobilized two-dimensionally, there is a limitation on the amount of the immobilized metal fine-particles, and increasing the intensity of LSPR any further is difficult. That is to say, to produce the aforementioned "material providing high intensity of LSPR and capable of highly sensitively detecting variation in the environment surrounding the metal fine-particles," a structure in which the metal fine-particles are distributed uniformly not only in the surface portion of the support but also in a thickness direction thereof is required.

A composite including metal fine-particles that are present three-dimensionally inside a matrix is disclosed in, e.g., Non-Patent Document 1 and Patent Documents 3 and 4. In Non-Patent Document 1, it is discovered and proposed that a structure in which metal fine-particles are dispersed inside a porous matrix may be obtained by impregnating a porous silica with an $HAuCl_4$ acid solution or $NaCuAl_4$ solution and then heat-reducing the same in a hydrogen atmosphere. In addition, in Patent Document 3, it is proposed that a uniformly 3D-dispersed structure is produced in a matrix by radiolysis reduction after impregnating a precursor compound of the metal fine-particles or metal oxide fine-particles in a microporous or mesoporous solid matrix. Further, in Patent Document 4, it is discovered and proposed that after metal fine-particles covered by a protein such as ferritin or a polymeric dendrimer are mixed with a raw material for forming a porous body, an organic composite porous medium, in which nano-particles do not aggregate but are contained three-dimensionally, may be obtained by a sol-gel method.

However, in Non-Patent Document 1, since the precursor solution of the metal fine-particles has a low degree of impregnation inside the porous matrix and are in a non-uniform state in the solid matrix, the content of the reduced metal fine-particles in the matrix remains relatively low. Moreover, the metal fine-particles are basically present in a condensed state close to the surface of the porous matrix.

In addition, in Patent Document 3, the metal fine-particles formed in the porous matrix have the same size as voids in the matrix material. Thus, most of the surface of the metal fine-particles is in contact with the matrix, namely covered by the matrix component, and it is difficult to use the wavelength variation of LSPR caused by the variation in the medium surrounding the metal fine-particles. In addition, as for the fabrication method, to form metal nanoparticles in deep portions of the porous matrix, the deep portions of the porous matrix must also be impregnated with the precursor solution of the metal nanoparticles. However, as the matrix has a small pore diameter, the deep portions cannot be impregnated with the precursor solution if being immersed only, and a special apparatus equipped with an impregnation chamber and a pump system is thus required. Further, because this impregnation process requires a vacuum condition, it takes a long time. In addition, to reduce the precursor of the metal fine-particles, a special reducing element useful in radiolysis reduction, such as a γ-ray source, an X-ray source or an accelerated electron source, is required. Also, to suppress oxidation of the metal fine-particles caused by oxygen radical generated during the reduction, a primary alcohol, a secondary alcohol or a formate salt must be added as an oxygen radical blocker.

In Patent Document 4, in addition to requiring very complicated operations, in the resulting composite porous body containing metal fine-particles, since the surface of the contained metal fine-particles is covered by an organic compound, it is difficult to utilize the wavelength variation of LSPR caused by the variation in the medium surrounding the metal fine-particles. Although this patent document also describes removal of the organic compound having the function of supporting the metal nano-particles, in this case, there is a fear of the metal fine-particles moving or falling off inside the matrix. Further, depending on the type of the metal, there is a problem that a metal oxide is generated so that LSPR of the metal fine-particles is no longer exhibited.

Prior-Art Documents

Patent Documents

Patent Document 1: Japanese Patent Application Publication No. 2000-356587
Patent Document 2: Japanese Patent Application Publication No. 2005-171306
Patent Document 3: Japanese Translation of PCT Publication No. 2008-531447
Patent Document 4: Re-publication of PCT Publication No. 04-110930

Non-Patent Document

Non-Patent Document 1: Kuei-Jung Chao et al. ["Preparation and characterization of highly dispersed gold fine-particles within channels of mesoporous silica," Catalysis Today (2004), Vol. 97, No. 1, pp. 49-53]

SUMMARY

Problems to Be Solved by the Invention

In a case that a metal fine-particle dispersed composite including metal fine-particles dispersed in a matrix is applied to devices utilizing LSPR of the metal fine-particles and so on, it is necessary to stabilize the metal fine-particles by immobilizing them to a solid framework of the matrix. It is important at least that the absorption spectrum thereof has a large intensity. In addition, generally, the sharper the absorption spectrum, the more possible it is to perform highly-sensitive detection. To achieve a sharp absorption spectrum with large intensity, the metal fine-particle dispersed composite is required to have structural characteristics such as:

1) the size of the metal fine-particles being controlled within a predetermined range;

2) the shape of the metal fine-particles being uniform;

3) neighboring metal fine-particles being separated from each other while maintaining an inter-particle spacing equal to or larger than a constant value;

4) the volume filling proportion of the metal fine-particles relative to the metal fine-particle dispersed composite being controlled within a constant range; and

5) the metal fine-particles being present from a surface portion of the matrix, and also being distributed uniformly in the thickness direction of the same while maintaining a predetermined inter-particle distance.

In addition, to be applicable for sensors to highly sensitively detect the wavelength variation of LSPR caused by the variation in the environment outside the metal fine-particles, the metal fine-particle dispersed composite is further required to have, in addition to the above characteristics, the following structural characteristic:

6) the metal fine-particles being exposed to the outside environment.

An object of the invention is to provide a metal fine-particle dispersed composite and a method for fabricating the same. The metal fine-particle dispersed composite has been invented to address the above problems that could not be solved by the prior art, and is suitable for use in, for example, various devices utilizing LSPR.

Technical Means for Solving the Technical Problems

As a result of intensive studies for the aforementioned situations, the present inventors discovered that a metal fine-particle dispersed material meets the above requirements if it is fabricated by a method of performing a heating treatment to a mixture of a precursor capable of forming a 3D matrix and a precursor of metal fine-particles to form a 3D network structure and also precipitate the metal fine-particles by reducing the aforementioned precursor of metal fine-particles. The invention is thus accomplished.

Specifically, the metal fine-particle dispersed composite of the invention is provided with a matrix layer including a solid framework and voids defined by the solid framework, and metal fine-particles immobilized to the solid framework, and has the following features a to d:

a) the solid framework containing an aluminum oxyhydroxide or an alumina hydrate and forming a 3D network structure;

b) the metal fine-particles having a mean particle diameter in the range of 3 to 100 nm, with a proportion of 60% or more having particle diameters in the range of 1 to 100 nm;

c) the metal fine-particles being present in a manner that they are not in contact with one another and neighboring metal fine-particles are apart from each other by a distance equal to or larger than the particle diameter of the larger one of the neighboring metal fine-particles; and

d) the metal fine-particles are 3D-dispersed in the matrix layer, wherein each metal fine-particle has a portion exposed in the voids of the matrix layer.

The metal fine-particle dispersed composite of the invention may have a void proportion in the range of 15 to 95%. The volume fraction of the metal fine-particles relative to the metal fine-particle dispersed composite may be in the range of 0.05 to 30%. The metal fine-particles may include Au, Ag or Cu. The metal fine-particles may induce LSPR when interacting with light of a wavelength of 380 nm or more. Furthermore, a binding species having a functional group interacting with a specific substance may be immobilized on the surface of the metal fine-particles.

A LSPR inducing substrate of the invention is provided with any above-described metal fine-particle dispersed composite and a light reflecting member disposed on one side of the metal fine-particle dispersed composite.

In the LSPR inducing substrate of the invention, the metal fine-particle dispersed composite may be provided with a first surface receiving light emitted from a light source and a second surface formed opposite to the first surface. The light reflecting member may be disposed connected to the second surface.

In addition, in the LSPR inducing substrate of the invention, the light reflecting member may be provided with a light transmission layer and a metal layer laminated on the light transmission layer.

In addition, in the LSPR inducing substrate of the invention, the light reflecting member may further include a protection layer covering the metal layer. In this case, the protection layer may be include a Ni-Cr alloy.

In addition, a method for fabricating a metal fine-particle dispersed composite in a first aspect of the invention is for fabricating a metal fine-particle dispersed composite provided with a matrix layer including a solid framework and voids defined by the solid framework, and metal fine-particles immobilized to the solid framework. The method may include the following steps Ia to Id:

Ia) preparing a slurry containing an aluminum oxyhydroxide or an alumina hydrate for forming the solid framework;

Ib) mixing the slurry with a metal compound as a raw material of the metal fine-particles to prepare a coating liquid, wherein the metal compound has an amount, in terms of the metal element, in the range of 0.5 to 480 weight parts relative to 100 weight parts of the solid content of the slurry;

Ic) coating the coating liquid on a substrate and drying the same to form a coated film; and

Id) subjecting the coated film to a heating treatment to form, from the coated film, a matrix layer including a solid framework with a 3D network structure and voids defined by the solid framework, and simultaneously to heat-reduce the metal ion of the metal compound to precipitate particle-like metal as the metal fine-particles.

In this case, after the step Id, a step Ie) of immobilizing, on the surface of the metal fine-particles, a binding species having a functional group interacting with a specific substance may be further included.

In addition, a method for fabricating a metal fine-particle dispersed composite in a second aspect of the invention is for fabricating a metal fine-particle dispersed composite provided with a matrix layer including a solid framework and voids defined by the solid framework, and metal fine-particles immobilized to the solid framework. The method may include the following steps IIa to IId:

IIa) preparing a slurry containing an aluminum oxyhydroxide or an alumina hydrate for forming the solid framework;

IIb) coating the slurry on a substrate, drying and then subjecting the coated slurry to a heating treatment to form a matrix layer including a solid framework having a 3D network structure and voids defined by the solid framework;

IIc) impregnating the matrix layer with a solution containing a metal ion as a raw material of the metal fine-particles, wherein the metal ion has an amount, in terms of the metal element, in the range of 0.5 to 480 weight parts relative to 100 weight parts of the solid content of the slurry; and

IId) reducing the metal ion to precipitate particle-like metal as the metal fine-particles through a heating treatment after the step IIc.

In this case, after the step IId, a step Ie) of immobilizing, on the surface of the metal fine-particles, a binding species having a functional group interacting with a specific substance may further be included.

In addition, a method for fabricating a metal fine-particle dispersed composite in a third aspect of the invention is for fabricating a metal fine-particle dispersed composite provided with a matrix layer including a solid framework and voids defined by the solid framework, and metal fine-particles immobilized to the solid framework. The fabrication method includes the following steps IIIa to IIId:

IIIa) preparing a slurry containing a metal hydroxide or a metal oxide as a raw material of the solid framework;

IIIb) mixing the slurry with a metal compound as a raw material of the metal fine-particles to prepare a coating liquid, wherein the metal compound has an amount, in terms of the metal element, in the range of 0.5 to 480 weight parts relative to 100 weight parts of the solid content of the slurry;

IIIc) coating the coating liquid on a substrate and drying the same to form a coated film; and

IIId) subjecting the coated film to a heating treatment to form, from the coated film, a matrix layer including a solid framework having a 3D network structure and voids defined by the solid framework, and simultaneously to heat-reduce the metal ion of the metal compound to precipitate particle-like metal as the metal fine-particles, so as to obtain the metal fine-particle dispersed composite.

The fabrication method is characterized in that the step IIId is performed in the presence of a polyvinyl alcohol.

In the method for fabricating a metal fine-particle dispersed composite in the third aspect of the invention, the polyvinyl alcohol may be added in the step IIIa of preparing the slurry. Alternatively, the polyvinyl alcohol may be added in the step IIIb of preparing the coating liquid.

In addition, in the method for fabricating a metal fine-particle dispersed composite in the third aspect of the invention, the polyvinyl alcohol may be used in the range of 0.1 to 50 weight parts relative to 1 weight part of the metal compound.

In addition, in the method for fabricating a metal fine-particle dispersed composite in the third aspect of the invention, the polyvinyl alcohol may have a polymerization degree in the range of 10 to 5000.

In addition, in the method for fabricating a metal fine-particle dispersed composite in the third aspect of the invention, the polyvinyl alcohol may have a saponification degree of 30% or more.

In addition, the method for fabricating a metal fine-particle dispersed composite in the third aspect of the invention may further include the following step IIIe:

IIIe) heating the metal fine-particle dispersed composite at a temperature equal to or higher than the temperature at which thermal decomposition of the polyvinyl alcohol starts.

In addition, the metal fine-particle dispersed composite in the third aspect of the invention is fabricated by any above-described method for fabricating a metal fine-particle dispersed composite.

A method for fabricating a metal fine-particle dispersed composite in a fourth aspect of the invention is for fabricating a metal fine-particle dispersed composite provided with a matrix layer including a solid framework and voids defined by the solid framework, and metal fine-particles immobilized to the solid framework. Moreover, the method for fabricating a metal fine-particle dispersed composite of the invention includes the following steps IVa to IVd:

IVa) preparing a slurry containing a metal hydroxide or a metal oxide as a raw material of the solid framework;
IVb) coating the slurry on a substrate, drying and then subjecting the coated slurry to a heating treatment to form a matrix layer including a solid framework having a 3D network structure and voids defined by the solid framework by;
IVc) impregnating the matrix layer with a solution containing a metal ion as a raw material of the metal fine-particles, wherein the metal ion has an amount, in terms of the metal element, in the range of 0.2 to 1100 weight parts relative to 100 weight parts of the solid content of the slurry;
IVd) reducing the metal ion through a heating treatment after the step IVc to precipitate particle-like metal as the metal fine-particles.
The fabrication method is characterized in that a polyvinyl alcohol is mixed in the solution containing the metal ion in the step IVc and the step IVd is performed in the presence of the polyvinyl alcohol.

In the method for fabricating a metal fine-particle dispersed composite in the fourth aspect of the invention, the polyvinyl alcohol may be used in the range of 0.1 to 50 weight parts relative to 1 weight part of a metal compound which is a raw material of the metal ion.

In addition, in the method for fabricating a metal fine-particle dispersed composite in the fourth aspect of the invention, the polyvinyl alcohol may have a polymerization degree in the range of 10 to 5000.

In addition, in the method for fabricating a metal fine-particle dispersed composite in the fourth aspect of the invention, the polyvinyl alcohol may have a saponification degree of 30% or more.

In addition, the method for fabricating a metal fine-particle dispersed composite in the fourth aspect of the invention may further include the following step IVe:

IVe) heating the metal fine-particle dispersed composite at a temperature equal to or higher than the temperature at which thermal decomposition of the polyvinyl alcohol starts.

In addition, in the method for fabricating a metal fine-particle dispersed composite in the fourth aspect of the invention, the slurry may contain a silane compound in the range of 10 to 200 weight parts relative to 100 weight parts of the solid content of the slurry.

In addition, the metal fine-particle dispersed composite in the fourth aspect of the invention is fabricated by any above-described method for fabricating a metal fine-particle dispersed composite.

Effects of the Invention

The metal fine-particle dispersed composite of the invention includes a matrix of a 3D network structure including a solid framework and voids defined by the solid framework. Because the metal fine-particles are 3D-dispersed in this matrix, it is possible to increase the intensity of the absorption spectrum of LSPR. Furthermore, since the metal fine-particles present inside the matrix are controlled to have particle diameters in a predetermined range and are dispersed uniformly while maintaining an inter-particle distance, the absorption spectrum of LSPR is sharp. Further, since each metal fine-particle has a portion exposed in the voids inside the matrix having a network structure, it is possible to make the most of the characteristic that the resonant wavelength varies with the variation in the dielectric constant (or the refractive index) of the medium surrounding the metal fine-particles, and applications to devices taking advantage of this characteristic also become possible.

The metal fine-particle dispersed composite of the invention having the above structural features is suitable for use not only in the field utilizing LSPR effect, but also in, for example, catalysts and electrodes. Its application to electrochemical devices using the foregoing is possible, and thus fuel cells, air cells, water electrolysis devices, electric double layer capacitors, gas sensors, pollutant gas removal devices and so on may be provided. In addition, because the metal fine-particles do not aggregate but are homogeneously dispersed, development in various devices, such as optical devices of light emission, light modulation or the like and electronic devices, that take advantage of the characteristics becomes possible.

BRIEF DESCRIPTION OF THE DRAWINGS

FIG. 1 schematically illustrates the structure of the matrix in a nano-composite according to the first embodiment of the invention.

FIG. 2 schematically illustrates the dispersed state of metal fine-particles in a cross section in the thickness direction of the nano-composite according to the first embodiment of the invention.

FIG. 3 schematically illustrates the dispersed state of metal fine-particles in a cross section parallel to the surface of the nano-composite in FIG. 2.

FIG. 4 illustrates the structure of metal fine-particles.

FIG. 5 illustrates, in a magnified view, a nano-composite having a binding species according to a variant example.

FIG. 6 illustrates a specific binding by means of a binding species.

FIG. 7 illustrates a schematic structure of a LSPR-inducing substrate according to an embodiment of the invention.

FIG. 8 schematically illustrates the structure of the matrix in a nano-composite according to the second embodiment of the invention.

FIG. 9 schematically illustrates the dispersed state of metal fine-particles at a cross section in the thickness direction of the nano-composite according to the second embodiment of the invention.

FIG. 10 schematically illustrates the dispersed state of metal fine-particles at a cross section parallel to the surface of the nano-composite in FIG. 9.

FIG. 11 is an image obtained by observing a surface of the nano-composite in Examples 1-2 of the invention using a scanning electron microscope (SEM).

FIG. 12 is an image obtained by observing a cross section of the nano-composite in Examples 1-2 of the invention using a transmission electron microscope (TEM).

FIG. 13 shows the absorption spectra of the nano-composite measured in air and in water, respectively, in Examples 1-2 of the invention.

DESCRIPTION OF THE EMBODIMENTS

Embodiments of the invention are hereinafter described in details with reference to appropriate drawings.

[First Embodiment]

A metal fine-particle dispersed composite of the first embodiment of the invention is provided with a matrix layer including a solid framework and voids defined by the solid framework, and metal fine-particles immobilized to the solid framework, and having the following features a to d:

a) the solid framework containing an aluminum oxyhydroxide or an alumina hydrate and forming a 3D network structure;

b) the metal fine-particles having a mean particle diameter in the range of 3 to 100 nm, with a proportion of 60% or more having particle diameters in the range of 1 to 100 nm;

c) the metal fine-particles being present in a manner that they are not in contact with one another and neighboring metal fine-particles are apart from each other by a distance equal to or larger than the particle diameter of the larger one of the neighboring metal fine-particles; and

d) the metal fine-particles are 3D-dispersed in the matrix layer, wherein each metal fine-particle has a portion exposed in the voids in the matrix layer.

<Metal Fine-particle Dispersed Composite>

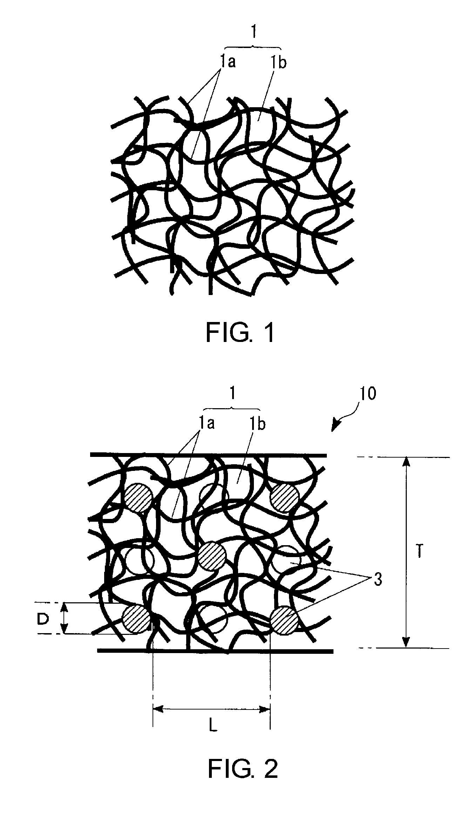

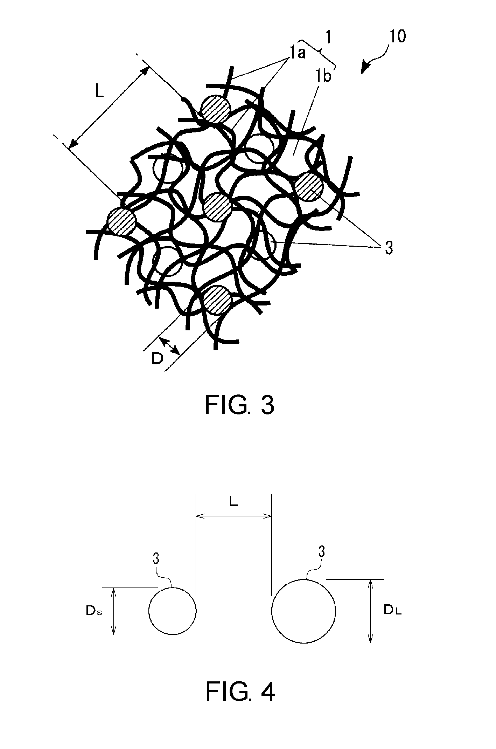

FIG. 1 schematically illustrates the structure of the matrix layer 1 in a metal fine-particle dispersed composite 10 (hereinafter simply called "nano-composite") according to the present embodiment. FIG. 2 schematically illustrates the dispersed state of the metal fine-particles 3 at a cross section in the thickness direction of the nano-composite 10. FIG. 3 schematically illustrates the dispersed state of the metal fine-particles 3 at a cross section in the surface direction of the nano-composite 10. FIG. 4 illustrates the metal fine-particles 3 in a magnified view. Furthermore, between neighboring metal fine-particles 3 in FIG. 4, the particle diameter of the larger metal fine-particle 3 is denoted as $D_L$ while that of the smaller one as $D_S$. However, in a case that no distinction is made between the two, a particle diameter is just denoted as D.

The nano-composite 10 is provided with the matrix layer 1 including a solid framework 1a and voids 1b defined by the solid framework 1a, and metal fine-particles 3 immobilized to the solid framework 1a of the matrix layer 1. In addition, the nano-composite 10 has the following features a to d:

a) the solid framework 1a containing an aluminum oxyhydroxide or an alumina hydrate and forming a 3D network structure;

b) the metal fine-particles 3 having a mean particle diameter in the range of 3 to 100 nm, with a proportion of 60% or more having particle diameters D in the range of 1 to 100 nm;

c) the metal fine-particles 3 being present in a manner that they are not in contact with one another and neighboring metal fine-particles 3 are apart from each other by a distance equal to or larger than the particle diameter $D_L$ of the larger one of the neighboring metal fine-particles; and

d) the metal fine-particles 3 being 3D-dispersed in the matrix layer 1, wherein each metal fine-particle 3 has a portion exposed in the voids 1b of the matrix layer 1.

Further, the nano-composite 10 may also include a substrate not shown. Such substrate includes, e.g., glass, ceramics, silicon wafer, semiconductor, paper, metal, metal alloy, metal oxide, synthetic resin, organic/inorganic composite material and so on, and is applicable in shape of, e.g., plate, sheet, film, mesh, geometric pattern, convex and concave, fiber, snake belly, multilayer, ball, and so on. Further, the surface of these substrates may be subjected to, e.g., silane coupling agent treatment, chemical etching treatment, plasma treatment, alkali treatment, acid treatment, ozone treatment, ultraviolet treatment, electric grinding treatment, abrasive grinding treatment, and so on.

(Matrix Layer)

The matrix layer 1, as shown in FIG. 1, includes the solid framework 1a and the voids 1b defined by the solid framework 1a. As shown in the above feature a), the solid framework 1a includes an aluminum oxyhydroxide or an alumina hydrate and forms a 3D network structure. The solid framework 1a is an aggregate of fine inorganic filler (or crystals) of a metal oxide containing an aluminum oxyhydroxide or an alumina hydrate, and the inorganic filler is in shape of particle, scale, plate, needle, fiber, or cubic, etc. A 3D network structure including an aggregate of such inorganic filler is preferably obtained with a heating treatment to a slurry obtained by dispersing the inorganic filler of the metal oxide containing an aluminum oxyhydroxide or an alumina hydrate in a solution. In addition, the metal oxide containing an aluminum oxyhydroxide or an alumina hydrate is advantageous as a material having thermal resistance even at the heat-reduction of metal ion forming the metal fine-particles 3, and is also preferable from the viewpoint of chemical stability. Furthermore, although various materials such as boehmite (including pseudo-boehmite), gibbsite, diaspore and so on are known as an aluminum oxyhydroxide (or alumina hydrate), boehmite is more preferable among them. Details of boehmite will be described later.

A structural characteristic of such matrix layer 1 is that the matrix layer 1 has a permeability to gas and liquid, thus becoming a cause for enhancement of the utilization efficiency of the metal fine-particles 3. From the viewpoint of efficiently utilizing the high specific surface area and high activity of the metal fine-particles 3, the void proportion of the nano-composite 10 is preferably in the range of 15 to 95%. Here, the void proportion of the nano-composite 10 may be calculated using the apparent density (gross density) calculated from the area, thickness and weight of the nano-composite 10, and a density excluding the voids (true density) calculated from the inherent densities and composition ratios of the materials forming the solid framework 1a of the matrix layer 1 and the metal fine-particles 3 according to the later-described Eq. (A). When the void proportion is less than 15%, the openness to the outside environment is lowered, so that there are cases where the utilization efficiency of the metal fine-particles 3 is decreased. Meanwhile, when the void proportion exceeds 95%, the presence proportions of the solid framework 1a and the metal fine-particles 3 are lowered, so that there are cases where the mechanical strength drops and the effects (such as the LSPR effect) created by the metal fine-particles 3 are decreased.

In addition, as mentioned above, from the viewpoint of efficiently utilizing the high specific surface area and high activity of the metal fine-particles 3, the volume proportion of the fine-particles 3 in the nano-composite 10 relative to the total volume of the voids 1b in the nano-composite 10 is preferably in the range of 0.08 to 50%.

The thickness T of the matrix layer 1 varies with the particle diameter D of the metal fine-particles 3. However, in applications utilizing LSPR, the thickness T is preferably in the range of, e.g., 20 nm to 20 $\mu$m, and more preferably in the range of 30 nm to 10 $\mu$m.

In the case where the nano-composite 10 is applicable to the uses that utilize LSPR, it is possible to utilize any one of light-reflection or light-transmission LSPR. However, in the case where light-transmission LSPR is utilized, the matrix layer 1 preferably has light transmission properties in order to induce LSPR of the metal fine-particles 3, and is particularly preferably a material transmitting light having a wavelength of 380 nm or more.

The solid framework 1a include an aluminum oxyhydroxide or an alumina hydrate that easily forms a 3D network structure, but may also include, for example, silicon oxide (silica), aluminum oxide (alumina), titanium oxide, vanadium oxide, tantalum oxide, iron oxide, magnesium oxide, or zirconium oxide, etc., or an inorganic oxide that contains plural kinds of metal elements. These may be included alone or in a mixture of two or more.

(Metal Fine-particles)

In the nano-composite 10 of this embodiment, from the viewpoint of easy control over the inter-particle distance L and the particle diameter D of the metal fine-particles 3, the metal fine-particles 3 are preferably obtained by heat-reducing a metal ion as a precursor thereof. As the metal fine-particles 3 obtained in this way, a metal species such as gold (Au), silver (Ag), copper (Cu), cobalt (Co), nickel (Ni), palladium (Pd), platinum (Pt), tin (Sn), rhodium (Rh) or iridium (Ir), etc. may be used. In addition, an alloy (such as a Pt-Co alloy, etc.) of these metal species may also be used. Among the foregoing, Au, Ag, Cu, Pd, Pt, Sn, Rh and Ir may be taken as examples particularly suitable for use as metal species inducing LSPR. As metal species inducing LSPR by interacting with light in the visible region having a wavelength of 380 nm or more, Au, Ag and Cu are preferable. Especially, Au is most expected since it is hardly surface oxidized and is good in preservation stability.

The metal fine-particles 3 may be in various shapes, such as sphere, prolate spheroid, cube, truncated tetrahedron, bipyramid, regular octahedron, regular decahedron, regular icosahedron and so on. Nevertheless, a sphere shape in which the absorption spectrum of LSPR is sharp is most preferable. Here, the shape of the metal fine-particles 3 may be identified by observing with a transmission electron microscope (TEM). In addition, the mean particle diameter of the metal fine-particles 3 is defined as an area-average diameter of arbitrary 100 metal fine-particles 3 being measured. In addition, the so-called spherical metal fine-particles 3 are metal fine-particles in a shape of a sphere or a near-sphere that has a ratio of the average long diameter to the average short diameter being 1 or close to 1 (preferably 0.8 or more). Further, regarding the relationship between the long diameter and the short diameter of any individual metal fine-particle 3, it is preferred that the long diameter is less than 1.35 times the short diameter, and is more preferred that the long diameter is equal to or less than 1.25 times the short diameter. Furthermore, when the metal fine-particles 3 do not have a spherical shape but have, for example, a regular octahedral shape, the largest one among the edge lengths of a metal fine-particle 3 is taken as the long diameter of the same, the smallest one among the edge lengths is taken as the short diameter of the same, and the above long diameter is considered as the particle diameter D of the same.

As shown in the above feature b), the metal fine-particles 3 have a mean particle diameter in the range of 3 to 100 nm, with a proportion of 60% or more having particle diameters D in the range of 1 to 100 nm. Here, the mean particle diameter means the average value of the diameter (median diameter) of the metal fine-particles 3. When the proportion (number proportion relative to all the metal fine-particles) of the metal fine-particles 3 having the particle diameters D in the range of 1 to 100 nm is less than 60%, a high efficacy of LSPR is difficult to achieve. In addition, when the particle diameter D of the metal fine-particles 3 exceeds 100 nm, sufficient LSPR effect is difficult to achieve, and thus the mean particle diameter is set to be 100 nm or less. In addition, for example, for a nano-composite 10 including metal fine-particles 3 having a maximum particle diameter of about 50 to 75 nm or less, because the particle diameter distribution thereof is relatively small, it is easy to achieve a sharp absorption spectrum of LSPR. Accordingly, a nano-composite 10 including metal fine-particles 3 having a maximum particle diameter of about 50 to 75 nm or less can be a preferred embodiment even if the particle diameter distribution of the metal fine-particles 3 is not particularly limited. On the other hand, even if the nano-composite 10 includes metal fine-particles 3 having a particle diameter exceeding 75 nm, the absorption spectrum of LSPR becomes a sharp peak by decreasing the particle diameter distribution of the metal fine-particles 3. Accordingly, in this case, although the particle diameter distribution of the metal fine-particles 3 is also preferably controlled to be small, it is not particularly limited. In addition, because of the feature that the metal fine-particles 3 are dispersed with an inter-particle distance equal to or larger than the particle diameter, for example, magnetic metal fine-particles can be used as the metal fine-particles 3 to serve as magnetic bodies having excellent properties.

In a case where the metal fine-particles 3 are not spherical, the LSPR absorption spectrum tends to become broader since the apparent diameter becomes larger. Thus the particle diameter D in a case where the metal fine-particles 3 are not spherical is preferably 30 nm or less, more preferably 20 nm or less, and further preferably 10 nm or less. In addition, in a case where the metal fine-particles 3 are not spherical, it is preferred that the shapes of 80% or more, and more preferably, 90% or more of all the metal fine-particles 3 in the matrix layer 1 are substantially the same, especially in a relative manner.

Metal fine-particles 3 having particle diameters D of less than 1 nm may be present in the nano-composite 10, which are not likely to affect LSPR and cause no particular problem. Furthermore, relative to 100 weight parts of the total amount of the metal fine-particles 3 in the nano-composite 10, for example, in a case where the metal fine-particles 3 are gold fine-particles, the amount of the metal fine-particles 3 having particle diameters D of less than 1 nm is preferably set to be equal to or less than 10 weight parts, and more preferably equal to or less than 1 weight part. Here, the metal fine-particles 3 having particle diameters D of less than 1 nm may be detected by an XPS (X-ray photoelectron spectroscopy) analyzer or an EDX (energy dispersive X-ray) analyzer.

In addition, in order to achieve a LSPR effect with higher absorption spectrum intensity, the mean particle diameter of the metal fine-particles 3 is set to be at least 3 nm or more, preferably 10 nm or more and 100 nm or less, and more preferably 20 to 100 nm. In a case the mean particle diameter of the metal fine-particles 3 is less than 3 nm, the intensity of the LSPR absorption spectrum tends to become small.

In the nano-composite 10 of the present embodiment, the metal fine-particles 3 further preferably induce LSPR by interacting with light. The wavelength range for inducing LSPR varies with the particle diameter D, the particle shape, the metal species and the inter-particle distance L of the metal fine-particles 3, the refractive index of the matrix layer 1, and so on. Nevertheless, it is preferred to induce LSPR by light of a wavelength of, for example, 380 nm or more.

(State of Presence of Metal Fine-Particles)

As shown in the above feature c), in the matrix layer 1, the metal fine-particles 3 are present in a manner that they are not in contact with one another, and neighboring metal fine-particles 3 are apart from each other by a distance equal to or larger than the particle diameter of the larger one of the neighboring metal fine-particles 3. In other words, the spacing L (inter-particle distance) between neighboring metal fine-particles 3 is equal to or larger than the particle diameter $D_L$ of the larger one of the neighboring fine-particles 3 ($L \geq D_L$). In FIG. 4, the inter-particle distance L of the metal fine-particles 3 is equal to or larger than the particle diameter $D_L$ of the larger metal fine-particle 3. Accordingly, the metal fine-particles 3 are capable of efficiently exhibiting their LSPR properties. Furthermore, the relationship between the particle diameter $D_L$ of the larger one of neighboring metal fine-particles 3 and the particle diameter $D_S$ of the smaller one of the neighboring metal fine-particles 3 may be "$D_L \geq D_S$". In the nano-composite 10 of this embodiment, by heat-reducing the metal ion as a precursor of the metal fine-particles 3, thermal diffusion of the precipitated metal fine-particles 3 is easy, and the metal fine-particles 3 are dispersed inside the matrix layer 1 with an inter-particle distance L equal to or greater than the particle diameter $D_L$ of the larger one of neighboring metal fine-particles 3. In a case where the inter-particle distance L is smaller than the particle diameter $D_L$ of the larger one, interference between particles occurs at the LSPR. For example, there are cases where neighboring particles work together like a large particle to induce LSPR, so a sharp absorption spectrum cannot be made. Meanwhile, although there is no particular problem if the inter-particle distance L is large, since the inter-particle distance L of the metal fine-particles 3 in the dispersed state caused by thermal diffusion closely relates to the particle diameter D of the metal fine-particles 3 and the later-described volume fraction of the metal fine-particles 3, the upper limit for the inter-particle distance L is preferably controlled with the lower limit of the volume fraction of the metal fine-particles 3. When the inter-particle distance L is large, in other words, when the volume fraction of the metal fine-particles 3 relative to the nano-composite 10 is small, the intensity of the LSPR absorption spectrum becomes small. In such case, by increasing the thickness of the nano-composite 10, the intensity of the LSPR absorption spectrum may be increased.

In addition, the metal fine-particles 3 are 3D-dispersed in the matrix layer 1. That is, when a cross section in the thickness direction of the matrix layer 1 with a 3D network structure in the nano-composite 10 and a cross section in a direction orthogonal to the thickness direction, i.e., a cross section parallel to the surface of the matrix layer 1, are observed, as shown in FIGs. 2 and 3, a large number of metal fine-particles 3 are distributed in the vertical direction and the horizontal direction with an inter-particle distance L equal to or greater than the particle diameter $D_L$.

Further, it is preferred that 90% or more of the metal fine-particles 3 are single particles distributed with an inter-particle distance L equal to or greater than the particle diameter $D_L$. Herein, "single particle" means that each metal fine-particle 3 in the matrix layer 1 is present independently, and no aggregate of a plurality of particles (aggregated particle) is included. That is, the single particles include no aggregated particle in which plural metal fine-particles aggregate by an inter-molecular force. In addition, "aggregated particle" refers to, e.g., an aggregate formed by plural individual metal fine-particles gathering together. This is clearly confirmed by observation with a transmission electron microscope (TEM). Furthermore, though it is understood that the metal fine-particles 3 in the nano-composite 10 are, in terms of their chemical structure, metal fine-particles formed by aggregated metal atoms that are formed by heat-reduction, such metal fine-particles are considered to be formed through metal bonds between metal atoms and are distinguished from the aggregated particles formed by aggregation of plural particles. For example, when being observed with a TEM, an independent metal fine-particle 3 can be identified.

Since 90% or more of the metal fine-particles 3 are single particles as described above, the LSPR absorption spectrum is sharp and stable, thus achieving high detection accuracy. This situation means that, in other words, aggregated particles or particles dispersed with an inter-particle distance L equal to or less than the particle diameter $D_L$ account for less than 10%. In a case where such particles are present at 10% or more, the LSPR absorption spectrum gets broad or unstable, and a high detection accuracy is difficult to achieve when the nano-composite 10 is used in a device such as a sensor. In addition, when aggregated particles or the particles dispersed with an inter-particle distance L equal to or less than the particle diameter $D_L$ account for more than 10%, control of the particle diameter D also becomes extremely difficult.

In addition, the volume fraction of the metal fine-particles 3 in the matrix layer 1 is preferably 0.05 to 30% relative to the nano-composite 10. Herein, the "volume fraction" is the percentage of the total volume of the metal fine-particles 3 in a certain volume of the nano-composite 10 including the voids 1b. When the volume fraction of the metal fine-particles 3 is less than 0.05%, the intensity of the absorption spectrum of LSPR becomes considerably small. Even if the thickness of the nano-composite 10 is increased, the effects of the invention are difficult to achieve. Meanwhile, when the volume fraction exceeds 30%, because the spacing (inter-particle distance L) between neighboring metal fine-particles 3 becomes smaller than the particle diameter $D_L$ of the larger one of the neighboring metal fine-particles 3, a sharp peak of the absorption spectrum of LSPR becomes difficult to achieve.

In the nano-composite 10 of this embodiment, as shown in the above feature d), the metal fine-particles 3 are 3D-dispersed in the matrix layer 1, wherein each metal fine-particle 3 has a portion exposed in the voids 1b of the matrix layer 1. That is, in the nano-composite 10, since the metal fine-particles 3 are 3D-arranged in an efficient way with a high specific surface area, utilization efficiency of the metal fine-particles 3 may be enhanced. In addition, as each metal fine-particle 3 has a portion exposed in the voids 1b that communicate with the outside environment, the metal fine-particles 3 are also sensitive to the variation in the dielectric constant $\varepsilon_m(\lambda)$ $(=(n_m(\lambda))^2)$ ($n_m$ is the refractive index thereof) of the medium surrounding the metal fine-particles 3 and are capable of developing this characteristic. That is, the metal fine-particles 3 are capable of making the most of the characteristic that the resonance wavelength varies with the variation in the dielectric constant (or the refractive index) of the medium surrounding the metal fine-particles 3. A structural feature of such nano-composite 10 is that the nano-composite 10 is most suitable for use in, e.g., frost sensors, moisture sensors, bio-sensors, chemical sensors and so on among the applications utilizing LSPR.

In the nano-composite 10, when a cross section of the matrix layer 1 is observed using, e.g., a TEM or the like, it is seen that the metal fine-particles 3 in the matrix layer 1 overlap with one another. However, as a matter of fact, the metal fine-particles 3 are dispersed as entirely independent signal particles while maintaining therebetween a distance equal to or greater than a certain value. In addition, due to being physically or chemically immobilized by the solid framework 1a that includes an aluminum oxyhydroxide or an alumina hydrate and has a 3D network structure, the metal fine-particles 3 may be prevented from aggregating and falling off with aging, and are excellent in long-term preservability. Even in repeated use of the nano-composite 10, aggregation and falling-off of the fine-particles 3 are suppressed. Especially in a case where the solid framework 1a include an aluminum oxyhydroxide or an alumina hydrate, even under preservation at room temperature for a long time, aggregation of the metal fine-particles 3 is not recognized. Therefore, it is considered that the solid framework 1a containing an aluminum oxyhydroxide or an alumina hydrate is highly effective in chemically immobilizing the metal fine-particles 3.

<Applications of Metal Fine-Particle Dispersed Composite Material>

In the nano-composite 10 of this embodiment having the above structure, the metal fine-particles 3 are 3D-dispersed uniformly in the matrix layer 1 having a 3D network structure while maintaining an inter-particle distance L equal to or greater than a certain value. For this reason, the LSPR absorption spectrum not only is sharp, but also is very stable and excellent in reproducibility and reliability. Further, because most of the surface of the metal fine-particle 3 is exposed in the voids 1b in the matrix layer 1 that communicate with the outside environment, it is possible to sufficiently exhibit the characteristic of the metal fine-particles 3 that the resonance wavelength varies with the variation in the dielectric constant (or the refractive index) of the medium surrounding the metal fine-particles 3. Accordingly, the nano-composite 10 is suitable for use in various sensing devices such as bio-sensors, chemical sensors, moisture sensors, frost sensors, gas sensors and so on. By applying the nano-composite 10 to the sensing devices, a high-precision detection based on a simple constitution becomes possible. In addition, the nano-composite 10 may also be applied to various devices such as catalyst filters, fuel cells, air cells, water electrolysis devices, electric double layer capacitors, pollutant gas removal devices, optical recording and regenerating devices, optical information processing devices, energy enhancement devices, high-sensitivity photodiode devices, and so on.

<Fabrication Method>

Next, a method for fabricating the nano-composite 10 of this embodiment is described, which is roughly classified into (I) a method that disperses the metal fine-particles 3 in the step of forming the matrix layer 1, and (II) a method that disperses the metal fine-particles 3 in a preformed matrix layer 1. From the viewpoint of decreasing the fabrication steps of the nano-composite 10, the method (I) is preferable.

The method (I) includes the following steps Ia) to Id):

Ia) preparing a slurry containing an aluminum oxyhydroxide or an alumina hydrate for forming the solid framework 1a;
Ib) mixing the slurry with a metal compound as a raw material of the metal fine-particles 3 to prepare a coating liquid, wherein the metal compound has an amount, in terms of the metal element (in this specification, meaning the amount of the metal element contained in the metal compound being converted into the weight of the metal), in the range of 0.5 to 480 weight parts relative to 100 weight parts of the solid content of the slurry;

Ic) coating the coating liquid on a substrate and drying the same to form a coated film by; and

Id) subjecting the coated film to a heating treatment to form, from the coated film, the matrix layer 1 including the solid framework 1a having a 3D structure and voids defined by the solid framework 1a, and simultaneously to heat-reduce the metal ion of the metal compound to precipitate particle-like metal as the metal fine-particles 3.

The method (II) includes the following steps IIa) to IId):

IIa) preparing a slurry containing an aluminum oxyhydroxide or an alumina hydrate for forming the solid framework 1a;

IIb) coating the slurry on a substrate, drying and then subjecting the coated slurry to a heating treatment to form the matrix layer 1 including the solid framework 1a with a 3D network structure and the voids 1b defined by the solid framework 1a by;

IIc) impregnating the matrix layer 1 with a solution containing a metal ion as a raw material of the metal fine-particles 3, wherein the metal ion has an amount, in terms of the metal element, in the range of 0.5 to 480 weight parts relative to 100 weight parts of the solid content of the slurry; and

IId) reducing the metal ions to precipitate particle-like metal as the metal fine-particles 3 through a heating treatment after the step IIc.

Next, each step in the methods (I) and (II) is specifically described. However, parts common to both methods are explained at the same time. Here, a representative example is given in which the solid framework 1a in the matrix layer 1 are composed of boehmite (including pseudo-boehmite).

The solid framework 1a constituting the matrix layer 1 may be suitably made from a commercially available boehmite powder containing an aluminum oxyhydroxide (or alumina hydrate). For example, Boehmite (trade name) produced by Taimei Chemicals Co., Ltd., Disperal HP15 (trade name) by CNDEA Corporation, Versal™ Alumina (trade name) by Union Showa K.K., Celasule (trade name) by Kawai Lime Industry Co., Ltd., CAM9010 (trade name) by TOMOE Engineering Co., Ltd., Aluminasol 520 (trade name) by Nissan Chemical Industries, Ltd., Aluminasol-10A (trade name) by Kawaken Fine Chemicals Co., Ltd., SECO Boehmite Alumina (trade name) by SECO International Inc., and so on may be used.

The boehmite (Boehmite) used in an embodiment of the invention refers to fine-particles of an aluminum oxyhydroxide (AlOOH) or an alumina hydrate ($Al_2O_3 \cdot H_2O$) that have high crystallinity, while pseudo-boehmite refers to boehmite fine-particles that have low crystallinity. Nevertheless, both are described as boehmite in a broader sense without distinction. This boehmite powder may be produced by well-known methods such as neutralization of an aluminum salt, hydrolysis of an aluminum alkoxide, and so on. Since the boehmite powder is insoluble in water and resistant to organic solvents, acids and alkalis, it may be advantageously utilized as a component for constituting the solid framework 1a of the matrix layer 1. In addition, since the boehmite powder is characterized by having high dispersibility in an acidic aqueous solution, preparing a slurry of the boehmite powder is easy. The boehmite powder used preferably has a particle shape of a cubic shape, a needle shape, a rhombic plate shape, an intermediate shape of these shapes, or a wrinkled-sheet, etc., and has a mean particle diameter in the range of 10 nm to 2 $\mu$m. The solid framework 1a is formed by bonding the end faces or surfaces of the fine-particles to form a 3D network structure. Further, the mean particle diameter of the boehmite powder is derived by a laser diffraction method here.

The slurry containing the boehmite powder is obtained by mixing the boehmite powder with water or a polar solvent such as alcohol and then adjusting the mixed solution to acidic. In the method (I), the coating liquid is prepared by adding the metal compound as a raw material of the metal fine-particles 3 to this slurry and evenly mixing the same.

The preparation of the slurry is performed by dispersing the boehmite powder in water or a solvent such as a polar organic solvent, and the boehmite powder used is preferably made in the range of 5 to 40 weight parts and more preferably in the range of 10 to 25 weight parts, relative to 100 weight parts of the solvent. The solvent used is, for example, water, methanol, ethanol, glycerol, N,N-dimethylformamide, N,N-dimethylacetamide (DMAc), or N-methyl-2-pyrrolidone, etc. It is also possible that two or more of these solvents are used in combination. In order to improve the dispersibility of the boehmite powder, the mixed solution is desirably subjected to a dispersion treatment. The dispersion treatment may be performed by, e.g., stirring at room temperature for 5 minutes or longer, using an ultrasonic wave, and so on.

To enable even dispersion of the boehmite powder, the pH of the mixed solution is adjusted to 5 or less as needed. In this case, as a pH control agent, an organic acid such as formic acid, acetic acid, glycolic acid, oxalic acid, propionic acid, malonic acid, succinic acid, adipic acid, maleic acid, malic acid, tartaric acid, citric acid, benzoic acid, phthalic acid, isophthalic acid, terephthalic acid, glutaric acid, gluconic acid, lactic acid, aspartic acid, glutaminic acid, pimelic acid or suberic acid, an inorganic acid such as hydrochloric acid, nitric acid or phosphoric acid, or a salt of any of the above acids, for example, may be added properly. These pH control agents may be used alone or in combination of two or more. The particle diameter distribution of the boehmite powder may vary due to addition of the pH control agent, as compared with the case without addition of a pH control agent. Nevertheless, there is no particular problem.

In the method (I), the coating liquid is obtained by further adding the metal compound as a raw material of the metal fine-particles 3 in the slurry prepared as above. In this case, the amount of the metal compound added is made, in terms of the metal element, in the range of 0.5 to 480 weight parts relative to 100 weight parts of the solid content of the slurry. Furthermore, when the metal compound is added to the prepared slurry, the viscosity of the coating liquid may be increased. In such case, the optimal viscosity is desirably achieved by a proper addition of the aforementioned solvent.

The metal compound contained in the coating liquid prepared in the method (I) or the metal compound contained in the metal-ion containing solution prepared in the method (II) may be any compound containing the metal species constituting the metal fine-particles 3 with no particular limitation. The metal compound may be a salt or an organic carbonyl complex of an aforementioned metal. Examples of the salts of the metals include hydrochloride salts, sulfate salts, acetate salts, oxalate salts, citrate salts, and so on. Examples of the organic carbonyl compounds capable of forming organic carbonyl complexes of metal include β-diketones such as acetylacetone, benzoylacetone and dibenzoylmethane, and β-keto carboxylic esters such as ethyl acetoacetate.

Preferred specific examples of the metal compound include $H[AuCl_4]$, $Na[AuCl_4]$, $AuI$, $AuCl$, $AuCl_3$, $AuBr_3$, $NH_4[AuCl_4] \cdot n2H_2O$, $Ag(CH_3COO)$, $AgCl$, $AgClO_4$, $Ag_2CO_3$, $AgI$, $Ag_2SO_4$, $AgNO_3$, $Ni(CH_3COO)_2$, $Cu(CH_3COO)_2$, $CuSO_4$, $CuSO_4$, $CuSO_4$, $CuCl_2$, $CuSO_4$, $CuBr_2$, $Cu(NH_4)_2Cl_4$, $CuI$, $Cu(NO_3)_2$, $Cu(CH_3COCH_2COCH_3)_2$, $CoCl_2$, $CoCO_3$, $CoSO_4$, $Co(NO_3)_2$, $NiSO_4$, $NiCO_3$, $NiCl_2$, $NiBr_2$, $Ni(NO_3)_2$, $NiC_2O_4$, $Ni(H_2PO_2)_2$, $Ni(CH_3COCH_2COCH_3)_2$, $Pd(CH_3COO)_2$, $PdSO_4$, $PdCO_3$, $PdCl_2$, $PdBr_2$, $Pd(NO_3)_2$, $Pd(CH_3COCH_2COCH_3)_2$, $SnCl_2$, $IrCl_3$, $RhCl_3$, and so on.

To improve the strength, transparency, glossiness and so on of the matrix layer 1, a binder component may be mixed in the prepared slurry or coating liquid as needed. Suitable examples of the binder component that can be used in combination with the aluminum oxyhydroxide include: polyvinyl alcohol or modified products thereof; gum Arabic; cellulose derivatives, such as carboxymethyl cellulose, hydroxyethyl cellulose and so on; vinyl copolymer latexes, such as SBR latex, NBR latex, functional group-modified polymer latex, ethylene□vinyl acetate copolymer and so on; water-soluble cellulose; polyvinylpyrrolidone; gelatin and modified products thereof, starch and modified products thereof; casein and modified products thereof; maleic anhydride and copolymers thereof; acrylate ester copolymer; polyacrylic acid and copolymers thereof; polyamic acid (precursor of polyimide); and silane compounds, such as tetraethoxysilane, 3-aminopropyltriethoxysilane, 3-aminopropyltrimethoxysilane, N-2-(aminoethyl)-3-aminopropyltrimethoxysilane, N-2-(aminoethyl)-3-aminopropylmethyldimethoxysilane, 3-triethoxysilyl-N-(1,3-dimethylbutylidene)propylamine, N-phenyl-3-aminopropyltrimethoxysilane, and so on. These binder components may be used alone or in combination of two or more. Furthermore, with or without a metal compound, these binder components may be mixed properly, in an amount preferably in the range of 3 to 100 weight parts and more preferably 4 to 20 weight parts relative to 100 weight parts of the solid content of the slurry.

If required, it is also possible to add to the slurry or coating liquid, in addition to the binder, a dispersant, a thickener, a lubricant, a fluidity modifier, a surfactant, an defoaming agent, a water resistant agent, a releasing agent, a fluorescent whitening agent, an ultraviolet absorbent, an anti-oxidant and so on, in a range of not impairing the effects of the invention.

The method of coating the coating liquid containing the metal compound or the slurry not containing the metal compound is not particularly limited, and can be performed using, e.g., a lip coater, a knife coater, a comma coater, a blade coater, an air knife coater, a roll coater, a curtain coater, a bar coater, a gravure coater, a die coater, a spin coater, or a spray, etc.

The substrate used in the coating is not particularly limited in a case where the nano-composite 10 is used in a sensor or the like after being peeling off from the substrate, or in a case utilizing light-reflection LSPR with the nano-composite 10 being attached to the substrate. In the case utilizing light-transmission LSPR with the nano-composite 10 being attached to the substrate, the substrate is preferably light transmitting, and is, e.g., a glass substrate, or a transparent synthetic resin substrate, etc. Examples of the transparent synthetic resin include: polyimide resin, PET resin, acrylic resin, MS resin, MBS resin, ABS resin, polycarbonate resin, silicone resin, siloxane resin, epoxy resin, and so on.

After the coating liquid containing the metal compound or the slurry not containing the metal compound is coated, it is dried to form a coated film. The drying method is not particularly limited, possibly including heating at a temperature of 60 to 150°C for 1 to 60 minutes. Nevertheless, the drying is preferably performed at a temperature of 70 to 130°C.

After the coating liquid containing the metal compound or the slurry not containing the metal compound is coated and dried, it is subjected to a heating treatment preferably at 150 to 450°C and more preferably at 170 to 400°C, thereby forming the matrix layer 1. When the temperature of the heating treatment is lower than 150°C, the formation of the 3D network structure of the matrix layer 1 may not sufficiently occur. When the temperature of the heating treatment exceeds 450°C, for example, in a case where Au or Ag is used as the material of the metal fine-particles 3, melting of the metal fine-particles 3 occurs so that the resulting particle diameter D becomes larger and achieving a sufficient LSPR effect becomes difficult.

In the above method (I), it is possible to form the matrix layer 1 and at the same time form and disperse the metal fine-particles 3 through reduction of the metal ion by one heating step. In the method (II), after the matrix layer 1 is formed, the matrix layer 1 is impregnated with a solution containing a metal ion and then heated to reduce the metal ion and form and disperse the metal fine-particles 3.

In the metal ion-containing solution used in the above method (II), the metal ion is preferably contained, in terms of the metal element, in the range of 1 to 20 wt%. By limiting the concentration of the metal ion in the above range, it is possible to make the metal ion have an amount, in terms of the metal element, in the range of 0.5 to 480 weight parts relative to 100 weight parts of the solid content of the slurry.

The impregnation method in the above method (II) is not particularly limited as long as it enables at least a surface of the resulting matrix layer 1 to be in contact with the metal ion-containing solution, and may be a well-known method, such as, an immersion method, a spray method, a brush-painting method or a printing method, etc. The impregnation temperature may be 0 to 100°C, and preferably a normal temperature around 20 to 40°C. In addition, the impregnation is expected to take, for example, 5 seconds or longer, in the case of applying an immersion method.

The reduction of the metal ion and the dispersion of the precipitated metal fine-particles 3 are performed by a heating treatment preferably at 150 to 450°C and more preferably at 170 to 400°C. When the temperature of the heating treatment is lower than 150°C, the reduction of the metal ion is not sufficiently performed, and it may be difficult to make the mean particle diameter of the metal fine-particles 3 equal to or greater than the aforementioned lower limit (3 nm). In addition, when the temperature of the heating treatment is lower than 150°C, the thermal diffusion of the metal fine-particles 3 precipitated through the reduction may not sufficiently occur.

Herein, the formation of the metal fine-particles 3 through heat-reduction is described. The particle diameter D and the inter-particle distance L of the metal fine-particles 3 may be controlled by the heating temperature and heating time in the reduction step and the content of the metal ion in the matrix layer 1. The inventors have discovered that in the case where the heating temperature and heating time in the heat-reduction are constant, when the absolute amount of the metal ion in the matrix layer 1 differs, the particle diameter D of the metal fine-particles 3 being precipitated differs. In addition, it has also been discovered that in the case where a heat-reduction is performed without controlling the heating temperature and the heating time, the inter-particle distance L is smaller than the particle diameter $D_L$ of the larger one of neighboring metal fine-particles 3.

In addition, it is possible to apply the aforementioned findings, for example, to divide the thermal treatment in the reduction step into a plurality of steps for execution. For example, it is possible to perform a particle diameter control step of enabling the metal fine-particles 3 to grow to a predetermined particle diameter D at a first heating temperature, and an inter-particle distance control step of making the inter-particle distance L of the metal fine-particles 3 reach a predetermined range at a second heating temperature the same as or different from the first heating temperature. In this way, the particle diameter D and inter-particle distance L may be more precisely controlled by adjusting the first and second heating temperatures and the heating time.

Heat-reduction is adopted as the reduction method for its industrial advantages, such as that the particle diameter D and inter-particle distance L can be relatively easily controlled by controlling the reduction conditions (especially the heating temperature and the heating time), that simple equipment is applicable from laboratory scale to production scale without particular limitation, and that heat-reduction can be performed in a single-piece manner or a continuous manner without special efforts, etc.. Heat-reduction may be performed in an inert gas atmosphere such as Ar and $N_2$, in a vacuum of 1 to 5 KPa, or in the atmosphere. Vapor-phase reduction using a reductive gas such as hydrogen gas may also be utilized.

In heat-reduction, the metal ion present in the matrix layer 1 is reduced, and the metal fine-particles 3 are independently precipitated by means of thermal diffusion. The metal fine-particles 3 formed in this way maintain the inter-particle distance L equal to or larger than a certain value, and have shapes that are substantially uniform. The metal fine-particles 3 are 3D-dispersed evenly in the matrix layer 1. Especially, in the case of performing the reduction by this step, the shapes and the particle diameters D of the metal fine-particles 3 are uniformized, so that a nano-composite 10 in which the metal fine-particles 3 are evenly precipitated and dispersed in the matrix layer 1 with a substantially uniform inter-particle distance L is obtained. In addition, by controlling the structural units of the inorganic oxide constituting the matrix layer 1 or controlling the absolute amount of the metal ion and the volume fraction of the metal fine-particles 3, the particle diameter D of the metal fine-particles 3 and the distribution state of the metal fine-particles 3 in the matrix layer 1 may also be controlled.

In the way described above, the nano-composite 10 may be fabricated. Further, in a case where an inorganic oxide other than boehmite is used as the matrix layer 1, the aforementioned fabrication method may also be used.

[Variant Example of Nano-composite in the First Embodiment]

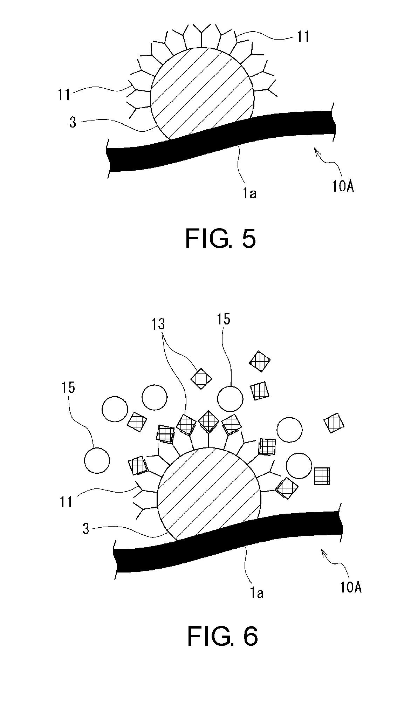

Next, a variant example of nano-composite in the first embodiment is described. In a preferred embodiment of the invention, a binding species 11 may be immobilized on a surface of the metal fine-particles 3, as shown in the magnified view in FIG. 5, for example,. In a nano-composite 10A being the variant example, the binding species 11 may be defined as a substance having: a functional group X that may be bonded to, for example, the metal fine-particles 3, and a functional group Y interacting with a specific substance such as a detection object molecule. The binding species 11 is not limited to be a single molecule, and may include a substance such as a composite one consisting of, for example, two or more constituents. The binding species 11 is immobilized on the surface of the metal fine-particles 3 through bonding between the functional group X and the metal fine-particles 3. In this case, the bonding between the functional group X and the metal fine-particles 3 refers to, e.g., chemical bonding, or physical bonding such as adsorption. In addition, the interaction between the functional group Y and the specific substance refers to, besides chemical bonding and physical bonding such as adsorption, a partial or overall change (modification or removal, etc.) of the functional group Y.

The functional group X of the binding species 11 is a function group that can be immobilized on the surface of the metal fine-particles 3 possibly by means of chemical bonding or by means of adsorption. Examples of such functional group X include: monovalent groups, such as $-SH$, $-NH_2$, $-NH_3X$ (X is a halogen atom), $-COOH$, $-Si(OCH_3)_3$, $-Si(OC_2H_5)_3$, $-SiCl_3$ and $-SCOCH_3$; and divalent groups, such as $-S_2-$ and $-S_4-$. Among these groups, the functional groups containing a sulfur atom, such as the mercapto group, the sulfide group and the disulfide group, are preferred.

In addition, the functional group Y of the binding species 11 is, for example, a substituent that may be bonded to an inorganic compound such as metal or metal oxide or an organic compound such as DNA or protein, or a leaving group that may leave due to, for example, an acid or alkali. Examples of the functional group Y capable of performing such interaction include: $-SH$, $-NH_2$, $-NR_3X$ (R is a hydrogen atom or $C_{1-6}$ alkyl, and X is a halogen atom), $-COOR$ (R is a hydrogen atom or $C_{1-6}$ alkyl), $-Si(OR)_3$ (R is $C_{1-6}$ alkyl), $-SiX_3$ (X is a halogen atom), $-SCOR$ (R is $C_{1-6}$ alkyl), $-OH$, $-CONH_2$, $-N_3$, $-CR=CHR'$ (R and R' are independently a hydrogen atom or $C_{1-6}$ alkyl), $-C{\equiv}CR$ (R is a hydrogen atom or $C_{1-6}$ alkyl), $-PO(OH)_2$, $-COR$ (R is $C_{1-6}$ alkyl), imidazolyl group, hydroquinolyl group, $-SO_3^-X$ (X is an alkali metal), N-hydroxysuccinimide group ($-NHS$), biotin group ($-Biotin$), and $-SO_2CH_2CH_2X$ (X is a halogen atom, $-OSO_2CH_3$, $-OSO_2C_6H_4CH_3$, $-OCOCH_3$, $-SO_3^-$, or pyridium).

Specific examples of the binding species 11 include $HS-(CH_2)_n-OH$ (n=11 or 16), $HS-(CH_2)_n-COOH$ (n=10, 11 or 15), $HS-(CH_2)_n-COO-NHS$ (n=10, 11 or 15), $HS-(CH_2)_n-NH_2{\cdot}HCl$ (n=10, 11 or 16), $HS-(CH_2)_{11}-NHCO-Biotin$, $HS-(CH_2)_{11}-N(CH_3)_3^+Cl^-$, $HS-(CH_2)_nSO_3^-Na^+$ (n = 10, 11 or 16), $HS-(CH_2)_{11}-PO(OH)_2$, $HS-(CH_2)_{10}-CH(OH)-CH_3$, $HS-(CH_2)_{10}-COCH_3$, $HS-(CH_2)_n-N_3$ (n=10, 11, 12, 16 or 17), $HS-(CH_2)_n-CH=CH_2$ (n=9 or 15), $HS-(CH_2)_4-C{\equiv}CH$, $HS-(CH_2)_n-CONH_2$ (n=10 or 15), $HS-(CH_2)_{11}-(OCH_2CH_2)_n-OCH_2-CONH_2$ (n=3 or 6), $HO-(CH_2)_{11}-S-S-(CH_2)_{11}-OH$, $CH_3-CO-S-(CH_2)_{11}-(OCH_2CH_2)_n-OH$ (n=3 or 6), and so on.

Other examples of the binding species 11 include heterocyclic compounds having an amino group or a mercapto group, such as 2-amino-1,3,5-triazine-4,6-dithiol, 3-amino-1,2,4-triazole-5-thiol, 2-amino-5-trifluoromethyl-1,3,4-thiadiazole, 5-amino-2-mercaptobenzimidazole, 6-amino-2-mercaptobenzothiazole, 4-amino-6-mercaptopyrazolo[3,4-d]pyrimidine, 2-amino-4-methoxybenzothiazole, 2-amino-4-phenyl-5-tetradecylthiazole, 2-amino-5-phenyl-1,3,4-thiadiazole, 2-amino-4-phenylthiazole, 4-amino-5-phenyl-4H-1,2,4-triazole-3-thiol, 2-amino-6-(methylsulfonyl)benzothiazole, 2-amino-4-methylthiazole, 2-amino-5-(methylthio)-1,3,4-thiadiazole, 3-amino-5-methylthio-1H-1,2,4-thiazole, 6-amino-1-methyluracil, 3-amino-5-nitrobenzisothiazole, 2-amino-1,3,4-thiadiazole, 5-amino-1,3,4-thiadiazole-2-thiol, 2-aminothiazole, 2-amino-4-thiazoleacetic acid, 2-amino-2-thiazoline, 2-amino-6-thiocyanate benzothiazole, DL-$\alpha$-amino-2-thiophene acetic acid, 4-amino-6-hydroxy-2-mercaptopyrimidine, 2-amino-6-purinethiol, 4-amino-5-(4-pyridyl)-4H-1,2,4-triazole-3-thiol, $N^4$-(2-amino-4-pyrimidinyl)sulfanilamide, 3-aminorhodanine, 5-amino-3-methylisothiazole, 2-amino-$\alpha$-(methoxyimino)-4-thiazoleacetic acid, thioguanine, 5-aminotetrazole, 3-amino-1,2,4-triazine, 3-amino-1,2,4-triazole, 4-amino-4H-1,2,4-triazole, 2-aminopurine, aminopyrazine, 3-amino-2-pyrazinecarboxylic acid, 3-aminopyrazole, 3-aminopyrazole-4-carbonitrile, 3-amino-4-pyrazolecarboxylic acid, 4-aminopyrazolo[3,4-d]pyrimidine, 2-aminopyridine, 3-aminopyridine, 4-aminopyridine, 5-amino-2-pyridinecarbonitrile, 2-amino-3-pyridinecarboxaldehyde, 2-amino-5-(4-pyridinyl)-1,3,4-thiadiazole, 2-aminopyrimidine, 4-aminopyrimidine and 4-amino-5-pyrimidinecarbonitrile, etc.; silane coupling agents having an amino group or a mercapto group, such as 3-aminopropyltriethoxysilane, 3-aminopropyltrimethoxysilane, N-2-(aminoethyl)-3-aminopropyltrimethoxysilane, N-2-(aminoethyl)-3-aminopropylmethyldimethoxysilane, 3-triethoxysilyl-N-(1,3-dimethylbutylidene)propylamine, N-phenyl-3-aminopropyltrimethoxysilane, 3-mercaptopropyltriethoxysilane, 3-mercaptopropyltrimethoxysilane, N-2-(mercaptoethyl)-3-mercaptopropyltrimethoxysilane, N-2-(mercaptoethyl)-3-mercaptopropylmethyldimethoxysilane, 3-triethoxysilyl-N-(1,3-dimethylbutylidene)propylmercapto and N-phenyl-3-mercaptopropyltrimethoxysilane, etc.; and so on. Moreover, these species may be used alone or in combination of two or more without particular limitation.

In addition, the molecular backbone of the binding species 11 may have a linear, branched or cyclic chemical structure including, between the functional groups X and Y, atoms selected from the group consisting of carbon atom, oxygen atom and nitrogen atom. The chemical structure may have a linear portion having 2 to 20, preferably 2 to 15, and more preferably 2 to 10 carbon atoms, and may be designed using a single molecular species or two or more molecular species. In an example of suitably applied embodiments where, for example, a detection-object molecule or the like is to be effectively detected, it is preferred that the thickness of the molecular mono-film (or molecular monolayer) formed by the binding species 11 is in the range of about 1.3 nm to 3 nm. In view of this, a binding species 11 having a $C_{11}$-$C_{20}$ alkane chain as a molecular skeleton is preferred. In such a case, for the long alkane chain immobilized on the surface of the metal fine-particle 3 via the functional group X extends vertically from the surface to form a molecular mono-film (molecular monolayer), the functional group Y suffuses the surface of the molecular mono-film (molecular monolayer). Well-known thiol compounds useful as reagents for forming self-assembly mono-films (SAM) can be suitably used as such binding species 11.

The nano-composite 10A having the aforementioned structure may serve as, for example, an affinity sensor. FIG. 6 schematically illustrates an application of the nano-composite 10A to an affinity sensor. First, the nano-composite 10A having a structure in which the binding species 11 (ligand) is bonded to the exposed part (the part exposed in the voids 1b) of the metal fine-particles 3 immobilized to the solid framework 1a is prepared. A sample containing an analyte 13 and a non-detection object substance 15 are then made to contact the nano-composite 10A in which the  binding species 11 is bonded to the metal fine-particles 3. Because the binding species 11 has a specific bindability to the analyte 13, specific bonding between the analyte 13 and the binding species 11 occurs. The non-detection object substance 15 having no specific bindability to the binding species 11 is not bonded to the binding species 11. As compared with the nano-composite 10A in which the analyte 13 is not bonded but only the binding species 11 is bonded, for the nano-composite 10A in which the analyte 13 is bonded via the binding species 11, the LSPR absorption spectrum under the light irradiation changes. That is, the color development changes. Thus, by measuring the change in the LSPR absorption spectrum, the analyte 13 in the sample may be detected with high sensitivity. The affinity sensors utilizing LSPR need not to use any labeling substance, and are applicable in various fields such as bio-sensors, gas sensors, chemical sensors and so on, as sensing means having a simple structure.

<Fabrication Method>

Next, a method for fabricating the nano-composite 10A according to the variant example of the first embodiment is described, which may be a method (I') based on the above method (I) or a method (II') based on the above method (II).

The method (I') includes the following steps Ia) to Ie):

Ia) preparing a slurry containing an aluminum oxyhydroxide or an alumina hydrate for forming the solid framework 1a;
Ib) mixing the slurry with a metal compound as a raw material of the metal fine-particles 3 to prepare a coating liquid, wherein the metal compound has an amount, in terms of the metal element (in this specification, meaning the amount of the metal element contained in the metal compound being converted into the weight of the metal), in the range of 0.5 to 480 weight parts relative to 100 weight parts of the solid content of the slurry;
Ic) coating the coating liquid on a substrate and drying the same to form a coated film;
Id) subjecting the coated film to a heating treatment to form, from the coated film, the matrix layer 1 including the solid framework 1a having a 3D network structure and voids defined by the solid framework 1a, and simultaneously to heat-reducing the metal ions of the metal compound to precipitate particle-like metal as the metal fine-particles; and
Ie) immobilizing the binding species 11 on the surface of the metal fine-particles 3 after the step Id.

The method (II') includes the following steps IIa) to IIe):

IIa) preparing a slurry containing an aluminum oxyhydroxide or an alumina hydrate for forming the solid framework 1a;
IIb) coating the slurry on a substrate, drying and then subjecting the coated slurry to a heating treatment to form the matrix layer 1 including the solid framework 1a having a 3D network structure and the voids 1b defined by the solid framework 1a;
IIc) impregnating the matrix layer 1 with a solution containing a metal ion as a raw material of the metal fine-particles 3, wherein the metal ion has an amount, in terms of the metal element, in the range of 0.5 to 480 weight parts relative to 100 weight parts of the solid content of the slurry; and
IId) reducing the metal ion to precipitate particle-like metal as the metal fine-particles 3 through another heat treatment after the step IIc; and
IIe) immobilizing the binding species 11 on the surface of the metal fine-particles 3 after the step IId.

The steps Ia) to Id) in the method (I') and the steps IIa) to IId) in the method (II') are the same as those having been mentioned in the descriptions for the above methods (I) and (II), so descriptions thereof are omitted. The step Ie) or IIe) is a step of immobilizing a binding species to obtain the nano-composite 10A by adding the binding species 11 to the metal fine-particles 3 of the nano-composite 10, and may be performed as follows.

Step of Immobilizing Binding Species:

In the step of immobilizing the binding species 11, the binding species 11 is immobilized to the surfaces of the exposed portions of the metal fine-particles 3. The step of immobilizing the binding species 11 may be performed by making the binding species 11 in contact with the surfaces of the exposed portions of the fine-particles 3. For example, it is preferred to perform a surface treatment to the metal fine-particles 3 using a treatment liquid obtained by dissolving the binding species 11 in a solvent. The solvent for dissolving the binding species 11 may be, but not limited to, water, a $C_{1-8}$ hydrocarbon alcohol such as methanol, ethanol, propanol, isopropanol, butanol, $t$-butanol, pentanol, hexanol, heptanol or octanol, etc, a $C_{3-6}$ hydrocarbon ketone such as acetone, propanone, methyl ethyl ketone, pentanone, hexanon, methyl isobutyl ketone or cyclohexanone, etc., a $C_{4-12}$ hydrocarbon ether such as diethyl ether, ethyleneglycol dimethyl ether, diethylene glycol dimethyl ether, diethylene glycol diethyl ether, diethylene glycol dibutyl ether or tetrahydrofuran, etc., a $C_{3-7}$ hydrocarbon ester such as methyl acetate, ethyl acetate, propyl acetate, butyl acetate, $\gamma$-butyrolactone or diethyl malonate, etc., a $C_{3-6}$ amide such as dimethylformamide, dimethylacetamide, tetramethylurea or hexamethylphosphoric triamide, etc., a $C_2$ sulfoxide compound such as dimethyl sulfoxide, etc., a $C_{1-6}$ halogen-containing compound such as chloromethane, bromomethane, dichloromethane, chloroform, carbon tetrachloride, dichloroethane, 1,2-dichloroethane, 1,4-dichlorobutane, trichloroethane, chlorobenzene or $o$-dichlorobenzene, etc., or a $C_{4-8}$ hydrocarbon such as butane, hexane, heptane, octane, benzene, toluene or xylene, etc.

The concentration of the binding species 11 in the treatment liquid is preferably, for example, 0.0001 to 1M (mol/L). A low concentration is advantageous from the viewpoint of less attachment of excess binding species 11 to the surface of the metal fine-particles 3. If a sufficient film formation effect generated by the binding species 11 is desired, the concentration is more preferably 0.005 to 0.05 M.

In a case where the surface of the metal fine-particles 3 is treated by the above treatment liquid, the treating method is not particularly limited as long as the treatment liquid is in contact with the surfaces of the exposed portions of the metal fine-particles 3. Nevertheless, an even contact is preferred. For example, the nano-composite 10 with the metal fine-particles 3 may be immersed in the treatment liquid, or the treatment liquid may be sprayed onto the exposed portions of the metal fine-particles 3 in the nano-composite 10 using a spray or the like. In addition, the temperature of the treatment liquid at this moment is not particularly limited, and is, for example, -20°C to 50°C. In addition, in a case of using an immersion method for the surface treatment, the immersion time is preferably 1 minute to 24 hours.

After the surface treatment is completed, it is preferred to perform a cleaning step to dissolve and remove the excess binding species 11 attached to the surface of the metal fine-particles 3 using an organic solvent. The organic solvent used therein may be one capable of dissolving the binding species 11, and examples thereof include the solvents that are used to dissolve the binding species 11 in the precedent step.

In the cleaning step, the method of cleaning the surface of the fine-particles 3 using an organic solvent is not limited. For example, the method may be achieved by immersing the metal fine-particles 3 in the organic solvent, or by spraying the organic solvent onto the surface of the metal fine-particles 3 using a spray or the like and then washing the organic solvent away. In this cleaning step, although the excess binding species 11 attached to the surface of the metal fine-particles 3 is dissolved and removed, removal of all the binding species 11 must not be done. Advantageously, the binding species 11 is cleaned and removed so that a film of the binding species 11 on the surface of the metal fine-particles 3 is approximately as thick as a monomolecular film. This method includes a step of cleaning with water before the aforementioned cleaning step, the aforementioned cleaning step, and then another step of cleaning with water. The temperature of the organic solvent in the above cleaning step is preferably 0 to 100°C and more preferably 5 to 50°C. The cleaning time is preferably in the range of 1 to 1000 seconds and more preferably 3 to 600 seconds. The amount of the used organic solvent is preferably 1 to 500 L, and more preferably 200 to 400L, per 1 $m^2$ surface area of the nano-composite 10.

In addition, if necessary, it is preferred to remove the binding species 11 attached to the surface of the solid framework 1a using an aqueous alkali solution. The concentration of the aqueous alkali solution used at this moment is preferably 10 to 500 mM (mmol/L), and the temperature of the same is preferably 0 to 50°C. For example, in cases where the solid framework 1a is immersed in an aqueous alkali solution, the immersion time is preferably set to be 5 seconds to 3 minutes.

[LSPR Inducing Substrate]

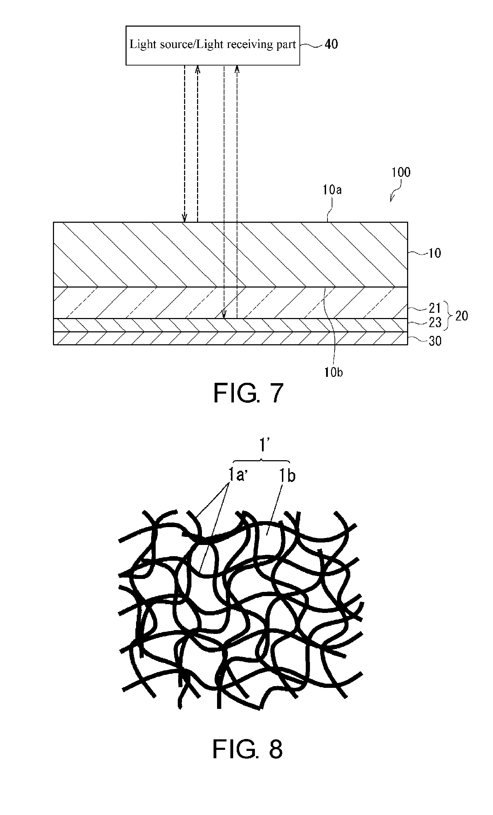

Next, a LSPR inducing substrate according to one of the preferred embodiments of applying the nano-composites 10 and 10A of the first embodiment to various devices is described. As mentioned above, the nano-composites 10 and 10A are applicable to various devices such as sensing devices. In order to increase the detection sensitivity at that situation, a LSPR inducing apparatus is provided with a light reflecting member. FIG. 7 schematically illustrates the structure of a LSPR inducing substrate 100 related to this embodiment. The LSPR inducing substrate 100 includes the nano-composite 10, a light reflecting member 20 disposed on one side of the nano-composite 10, and a protection layer 30 laminated on the light reflecting member 20. Further, although FIG. 7 shows an example of using the nano-composite 10, the nano-composite 10A may also be used instead. In addition, the protection layer 30 may have any constitution.

The light reflecting member 20 is provided with a light transmission layer 21, and a metal layer 23 laminated on the light transmission layer 21. As shown in FIG. 7, the nano-composite 10 of this embodiment includes a first surface 10A (light receiving surface) receiving light irradiated from an external light source, a light receiving part 40, as well as a second surface 10b (rear surface) formed opposite to the first surface. In addition, the light reflecting member 20 is disposed in a manner such that the light transmission layer 21 is connected to the second surface 10b.

The light transmission layer 21 is formed from a material transmitting light of a wavelength (e.g., in the range of 300 to 900 nm in cases where the metal fine-particles 3 include gold or silver) that induces LSPR. Such material is, for example, an inorganic transparent substrate of glass or quartz, etc., a transparent conductive film of indium tin oxide (ITO) or zinc oxide, etc., or a transparent synthetic resin such as polyimide resin, PET resin, acrylic resin, MS resin, MBS resin, ABS resin, polycarbonate resin, silicone resin, siloxane resin or epoxy resin, etc.

The metal layer 23 is a film of a metal material such as silver, aluminum, silicon, titanium, chromium, iron, manganese, cobalt, nickel, copper, zinc, tin or platinum, etc. Among the metal materials, aluminum is most preferred as the material of the metal layer 23 due to high optical reflectivity, high oxidation resistance, and high adhesion to the light transmission layer 21. The metal layer 23 may be formed as a film on one surface of the light transmission layer 21 by a method such as sputtering, CVD, evaporation, coating, ink-jet coating, electroless plating, or electroplating, etc.

The protection layer 30 provides protection by covering the metal layer 23 from the outside. The protection layer 30 prevents the metal layer 23 from oxidation in the heating treatment performed in the method for fabricating the nano-composite 10. Accordingly, in a case where the metal layer 23 includes a metal species difficult to be oxidized, disposing the protection layer 30 is not necessary. The protection layer 30 may be formed from a material having thermal resistance and oxidation resistance, or a material having a barrier property that suppresses oxygen permeation, etc. From such viewpoint, the protection layer 30 is made of, e.g., a metal material such as nickel, chromium or a Ni-Cr alloy, etc., an inorganic material such as glass, etc., or a highly thermo-resistant organic material such as polyimide resin or epoxy resin, etc. Among these, nickel, chromium and Ni-Cr alloy that particularly have high thermal resistance and high oxidation resistance are preferred. The protection layer 30 may be formed as a film on the surface of the metal layer 23 by a method such as sputtering, CVD, evaporation, coating, ink-jet coating, electroless plating, or electroplating, etc.

In the LSPR inducing substrate 100, from the viewpoint of increasing the detection sensitivity of the LSPR, the thickness of the nano-composite 10 is preferably set to be, for example, in the range of 30 nm to 10 $\mu$m.

In addition, although the thickness of the light transmission layer 21 is not particularly limited, it may be set to be, for example, in the range of 1 $\mu$m to 10 mm.

Although the thickness of the metal layer 23 is not particularly limited, it may be set to be, for example, in the range of 50 nm to 10 $\mu$m.

Further, in the case where the protection layer 30 is disposed, in order to provide a sufficient oxidation preventing function for the metal layer 23, the thickness thereof is preferably set to be, for example, in the range of 100 nm to 10 $\mu$m.

Due to the aforementioned constitution, the LSPR inducing substrate 100 is capable of induce excellent LSPR. As schematically shown by the dashed arrows in FIG. 7, a part of the light irradiated from the external light source/light receiving part 40 is reflected at the first surface 10a of the nano-composite 10, and the other part passes through the inside of the network structure of the nano-composite 10 and is reflected by the metal layer 23 of the light reflecting member 20. Then, these parts of reflected light are detected by a light receiving section (not shown) of the light source/ light receiving part 40 to measure the intensity and peak shift of the absorption spectrum of LSPR. In this way, in addition to the surface-reflected light from the first surface 10a of the nano-composite 10, the reflected light from the light reflecting member 20 may also be utilized, so that the intensity of the LSPR absorption spectrum becomes larger, and the detection sensitivity is considerably increased, as compared to the method of only measuring the surface-reflected light. In addition, by utilizing the reflected light from the light reflective member 20 in addition to the surface-reflected light, the entire apparatus may be downsized. Moreover, since it is possible to decrease the amount of irradiation light required for the LSPR absorption with the same intensity, measurement with high sensitivity but low power consumption is realized.

The LSPR inducing substrate 100 may be fabricated as follows. In the first method, the light reflecting member 20, which may include the protection layer 30, is used in replacement of the substrate used in the method of making the nano-composite 10. For example, a laminated object is prepared by laminating the light transmission layer 21, the metal layer 23 and the protection layer 30 in sequence. Then, for example, after the coating liquid obtained by mixing the slurry for forming the solid framework 1a with the metal compound is coated on the surface of the light transmission layer 21, or after the slurry for forming the solid framework 1 is coated on the surface of the light transmission layer 21 to form the solid framework 1a and the solid framework 1a is impregnated with the metal ion-containing solution, formation of the matrix layer 1 including the solid framework 1a and the voids 1b and precipitation of the metal fine-particles 3 is carried through a heating treatment (FIGs. 1 to 3). By using the light reflecting member 20 (possibly including the protection layer 30) as a substrate like this, the LSPR inducing substrate 100 may be fabricated with the same steps as those for fabricating the nano-composite 10. For example, in a case where the metal layer 23 includes a metal easily oxidized by heating, by disposing the protection layer 30 in advance, oxidation of the metal material of the metal layer 23 and degradation in light reflection due to the heating treatment may be effectively avoided.

In the second method of fabricating the LSPR inducing substrate 100, the nano-composite 10 and the light reflecting member 20 are respectively fabricated, and then the nano-composite 10 is arranged and fixed overlapping the surface of the light transmission layer 21 of the light reflecting member 20. In this case, because of the absence of a heating treatment to the metal layer 23 of the light reflecting member 20, the protection layer 30 may be omitted. In addition, the nano-composite 10 and the light reflecting member 20 are, for example, fixed by any means (e.g., through adhesion using an adhesive, or adhesion with pressing, etc.) to a periphery of the nano-composite 10 so as to not affect the occurrence of the LSPR.

Further, though FIG. 7 shows, as an example of the light reflecting member 20, a laminated object obtained by laminating the light transmission layer 21 and the metal layer 23, the light reflecting member 20 may be anything capable of reflecting light of the aforementioned wavelength, and may be, e.g., a mirror-finished metal plate, etc.

In addition, the nano-composite 10 and the light reflecting member 20 are not necessarily disposed in close contact with each other. The light reflecting member 20 may be disposed apart from the nano-composite 10 by any distance.

In addition, the LSPR inducing substrate 100 may include the light source/light receiving part 40 as a component. In this case, the light source/light receiving part 40 may include a light source (not shown) capable of irradiating light of a wavelength (e.g. in the range of 300 to 900 nm in cases where the metal fine-particles 3 include gold or silver) that induces LSPR of the nano-composite 10, and a light receiving part (not shown) receiving the light reflected by the surface of the nano-composite 10 or the light reflecting member 20. Furthermore, the light source and the light receiving part may be disposed separately, and are not limited to have the configuration in which the light from the light source is perpendicularly incident relative to the surface of the LSPR inducing substrate 100 (surface of nano-composite 10). The light receiving part may be arranged to receive the reflected part of light incident to the surface at any angle.

[Second Embodiment]

Next, a metal fine-particle dispersed composite of the second embodiment of the invention and a method for fabricating the same are described. First, an overview of the metal fine-particle dispersed composite is given with reference to FIGs. 8 to 10.

<Metal Fine-Particle Dispersed Composite>

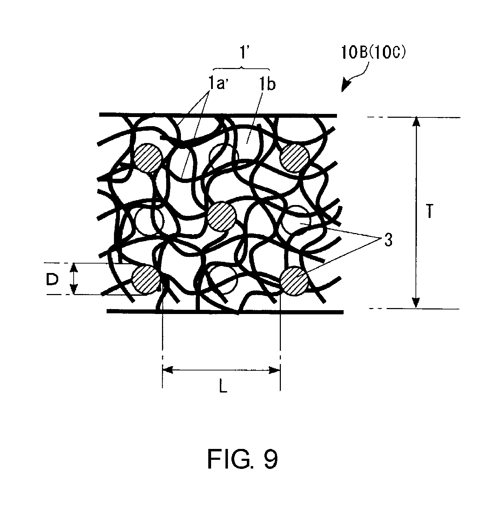

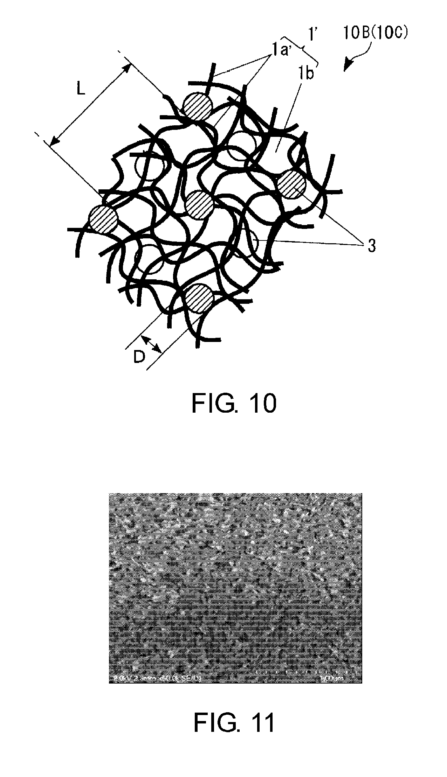

FIG. 8 schematically shows the structure of a matrix layer 1' in the metal fine-particle dispersed composites (nano-composites) 10B and 10C according to this embodiment. The nano-composite 10C is obtained by performing a later-described thermal treatment to the nano-composite 10B (see the step IIIe). FIG. 9 schematically shows the dispersed state of the metal fine-particles 3 at a cross section in the thickness direction of the nano-composites 10B and 10C. FIG. 10 schematically shows the dispersed state of the metal fine-particles 3 at a cross section in the surface direction of the nano-composites 10B and 10C.

The nano-composites 10B and 10C of this embodiment includes a matrix layer 1' including a solid framework 1a' and voids 1b defined by the solid framework 1a', and metal fine-particles 3 immobilized to the solid framework 1a'. In addition, the nano-composites 10B and 10C preferably have the following features a to d:

a) the solid framework 1a' containing a metal hydroxide or a metal oxide (e.g., an aluminum oxyhydroxide or an alumina hydrate) and forming a 3D network structure;
b) the metal fine-particles 3 having a mean particle diameter in the range of 3 to 100 nm, with a proportion of 60% or more having particle diameters D in the range of 1 to 100 nm;
c) the metal fine-particles 3 being present in a manner that they are not in contact with one another and neighboring metal fine-particles 3 are apart from each other by a distance equal to or larger than the particle diameter $D_L$ of the larger one of the neighboring metal fine-particles 3; and
d) the metal fine-particles 3 are 3D-dispersed in the matrix layer 1', wherein each metal fine-particle 3 has a portion exposed in the voids 1b of the matrix layer 1'.

Furthermore, the nano-composites 10B and 10C may also be provided with a substrate not shown, which is the same as that exemplified in the first embodiment.

(Matrix Layer)

As shown in FIG. 8, the matrix layer 1' includes a solid framework 1a' and voids 1b defined by the solid framework 1a'. As shown in the above feature a), the solid framework 1a' contains a metal hydroxide or a metal oxide and form a 3D network structure. In the following, an example is given in which the metal hydroxide or metal oxide is an aluminum oxyhydroxide or an alumina hydrate. The solid framework 1a' s an aggregate of fine inorganic filler (or crystals) of a metal oxide containing an aluminum oxyhydroxide or an alumina hydrate, and the inorganic filler is in a shape of particle, scale, plate, needle, fiber or cubic, etc. A 3D network structure including an aggregate of such inorganic filler is preferably obtained by subjecting a slurry, which is obtained by dispersing the inorganic filler of the metal oxide containing an aluminum oxyhydroxide or an alumina hydrate in a solution, to a heating treatment. In addition, the metal oxide containing an aluminum oxyhydroxide or an alumina hydrate is advantageous as a material having thermal resistance at the heat-reduction of the metal ion into the metal fine-particles 3, and is also preferred from the viewpoint of chemical stability. Furthermore, though various materials such as boehmite (including pseudo-boehmite), gibbsite, diaspore and so on are known as aluminum oxyhydroxides (or alumina hydrates), boehmite is more preferred among them. Details of boehmite will be described later.

A structural characteristic of such matrix layer 1' is that the matrix layer 1' has permeability to gas and liquid, thus becoming a cause for enhancement of the utilization efficiency of the metal fine-particles 3. From the viewpoint of efficiently utilizing the high specific surface area and high activity of the metal fine-particles 3, the void proportion of the nano-composites 10B and 10C is preferably in the range of 10 to 95% and more preferably in the range of 15 to 95%. Herein, the void proportion of the nano-composite 10B or 10C may be calculated using the apparent density (gross density) calculated from the area, thickness and weight of the nano-composite 10B or 10C, and the density excluding the voids (true density) calculated from the inherent densities and composition ratio of the materials forming the solid framework 1a' of the matrix layer 1' and the metal fine-particles 3 according to the later-described Eq. (A). When the void proportion is less than 10%, the openness to an outside environment is lowered, so there are cases where the utilization efficiency of the metal fine-particles 3 is decreased. Meanwhile, when the void proportion exceeds 95%, the presence proportions of the solid framework 1a and the metal fine-particles 3 are lowered, so there are cases where the mechanical strength drops and the effects (such as the LSPR effect) created by the metal fine-particles 3 are decreased.

In addition, as mentioned above, from the viewpoint of efficiently utilizing the high specific surface area and high activity of the metal fine-particles 3, the volume proportion of the metal fine-particles 3 in the nano-composite 10B or 10C relative to the total volume of the voids 1b in the nano-composites 10B or 10C is preferably in the range of 0.08 to 50%.

The thickness T of the matrix layer 1' varies with the particle diameter D of the metal fine-particles 3. However, in applications utilizing LSPR, the thickness T is preferably in the range of 20 nm to 20 $\mu$m and more preferably in the range of 30 nm to 10 $\mu$m, for example.

In the case where the nano-composite 10B or 10C is suitable for applications utilizing LSPR, it is possible to utilize light reflection LSPR or light transmission LSPR. However, in a case where light-transmission LSPR is utilized, the matrix layer 1' preferably has light transmittance to induce LSPR of the metal fine-particles 3, and is particularly preferably a material transmitting light of a wavelength of 380 nm or more.

The solid framework 1a' includes an aluminum oxyhydroxide or an alumina hydrate that easily form a 3D network structure, and may also include, e.g., silicon oxide (silica), aluminum oxide (alumina), titanium oxide, vanadium oxide, tantalum oxide, iron oxide, magnesium oxide, zirconium oxide, or an inorganic oxide containing plural kinds of metal elements. These may be included alone or in a mixture.

(Metal Fine-Particles)

In the nano-composite 10B or 10C of this embodiment, from the viewpoint of easy control over the inter-particle distance L and the particle diameter D of the metal fine-particles 3, the metal fine-particles 3 are preferably obtained by heat-reducing a metal ion as a precursors thereof. The metal fine-particles 3 may be the same as those described in the first embodiment.

The metal fine-particles 3 may be in various shapes, such as sphere, prolate spheroid, cube, truncated tetrahedron, bipyramid, regular octahedron, regular decahedron, regular icosahedron and so on. Nevertheless, a sphere shape of which the LSPR absorption spectrum is sharp is more preferred. Herein, the shape of the metal fine-particles 3 may be identified by observing with a TEM. In addition, the mean particle diameter of the metal fine-particles 3 is defined as the area-average diameter of arbitrary 100 metal fine-particles 3 being measured. Moreover, the so-called spherical metal fine-particles 3 are metal fine-particles in a shape of a sphere or a near-sphere and having a ratio of the average long diameter to the average short diameter being 1 or close to 1 (preferably 0.8 or more). Further, regarding the relationship between the long diameter and the short diameter of any individual metal fine-particle 3, it is preferred that the long diameter is less than 1.35 times the short diameter, and is more preferred that the long diameter is equal to or less than 1.25 times the short diameter. Furthermore, when the metal fine-particles 3 do not have a spherical shape but have, for example, a regular octahedral shape, the largest one among the edge lengths of a metal fine-particle 3 is taken as the long diameter of the same, the smallest one among the edge lengths is taken as the short diameter of the same, and the above long diameter is considered as the particle diameter D of the same.

As shown in the above feature b), the metal fine-particles 3 have a mean particle diameter in the range of 3 to 100 nm, with a proportion of 60% or more having particle diameters D in the range of 1 to 100 nm. Here, the mean particle diameter means the average value of the diameter (median diameter) of the metal fine-particles 3. When the proportion (number proportion relative to all the metal fine-particles) of the metal fine-particles 3 having the particle diameters D in the range of 1 to 100 nm is less than 60%, a high efficacy of LSPR is difficult to achieve. In addition, when the particle diameter D of the metal fine-particles 3 exceeds 100 nm, sufficient LSPR effect is difficult to achieve, and thus the mean particle diameter is set to be 100 nm or less. In addition, for example, for a nano-composite 10B or 10C including the metal fine-particles 3 having a maximum particle diameter of about 50 to 75 nm or less, because the particle diameter distribution thereof is relatively small, it is easy to achieve a sharp absorption spectrum of LSPR. Accordingly, a nano-composite 10B or 10C including metal fine-particles 3 having a maximum particle diameter of about 50 to 75 nm or less can be a preferred embodiment even if the particle diameter distribution of the metal fine-particles 3 is not particularly limited. On the other hand, even if the nano-composite 10B or 10C includes the metal fine-particles 3 having a particle diameter exceeding 75 nm, the absorption spectrum of LSPR becomes a sharp peak by decreasing the particle diameter distribution of the metal fine-particles 3. Accordingly, in this case, although the particle diameter distribution of the metal fine-particles 3 is also preferably controlled to be small, it is not particularly limited. In addition, because of the feature that the metal fine-particles 3 are dispersed with an inter-particle distance equal to or larger than the particle diameter, for example, magnetic metal fine-particles can be used as the metal fine-particles 3 to serve as magnetic bodies having excellent properties.

In a case where the metal fine-particles 3 are not spherical, the LSPR absorption spectrum tends to become broader since the apparent diameter becomes larger. Thus the particle diameter D in a case where the metal fine-particles 3 are not spherical is preferably 30 nm or less, more preferably 20 nm or less, and further preferably 10 nm or less. In addition, in a case where the metal fine-particles 3 are not spherical, it is preferred that the shapes of 80% or more, and more preferably, 90% or more of all the metal fine-particles 3 in the matrix layer 1 are substantially the same, especially in a relative manner.

Metal fine-particles 3 having particle diameters D of less than 1 nm may be present in the nano-composite 10B or 10C, which are not likely to affect LSPR and cause no particular problem. Furthermore, relative to 100 weight parts of the total amount of the metal fine-particles 3 in the nano-composite 10B or 10C, for example, in a case where the metal fine-particles 3 are gold fine-particles, the amount of the metal fine-particles 3 having particle diameters D of less than 1 nm is preferably set to be equal to or less than 10 weight parts, and more preferably equal to or less than 1 weight part. Here, the metal fine-particles 3 having particle diameters D of less than 1 nm may be detected by an XPS (X-ray photoelectron spectroscopy) analyzer or an EDX (energy dispersive X-ray) analyzer.

In addition, in order to achieve a LSPR effect with higher absorption spectrum intensity, the mean particle diameter of the metal fine-particles 3 is set to be at least 3 nm or more, preferably 5 nm or more and 100 nm or less, and more preferably 8 to 100 nm. In the case the mean particle diameter of the metal fine-particles 3 is less than 3 nm, the intensity of the LSPR absorption spectrum tends to become small.

In the nano-composite 10B or 10C of this embodiment, the metal fine-particles 3 further preferably induce LSPR by interacting with light. The wavelength range for inducing LSPR varies with the particle diameter D the particle shape, the metal species and the inter-particle distance L of the metal fine-particles 3, the refractive index of the matrix layer 1', and so on. Nevertheless, it is preferred to induce LSPR by light of a wavelength of, for example, 380 nm or more.

(State of Presence of Metal Fine-Particles)

As shown in the above feature c), in the matrix layer 1', the metal fine-particles 3 are present in a manner that they are not in contact with one another, and neighboring metal fine-particles 3 are apart from each other by a distance equal to or larger than the particle diameter of the larger one of the neighboring metal fine-particles 3. In other words, the spacing L (inter-particle distance) between neighboring metal fine-particles 3 is equal to or larger than the particle diameter $D_L$ of the larger one of the neighboring fine-particles 3 ($L \geq D_L$). The inter-particle distance L of the metal fine-particles 3 is equal to or larger than the particle diameter $D_L$ of the larger metal fine-particle 3 (FIG. 4). Accordingly, the metal fine-particles 3 are capable of efficiently exhibiting their LSPR properties. Furthermore, the relationship between the particle diameter $D_L$ of the larger one of neighboring metal fine-particles 3 and the particle diameter $D_S$ of the smaller one of the neighboring metal fine-particles 3 may be "$D_L \geq D_S$". In the nano-composite 10B or 10C of this embodiment, by heat-reducing the metal ion as a precursor of the metal fine-particles 3, thermal diffusion of the precipitated metal fine-particles 3 is easy, and the metal fine-particles 3 are dispersed inside the matrix layer 1 with an inter-particle distance L equal to or greater than the particle diameter $D_L$ of the larger one of neighboring fine-particles 3. In a case where the inter-particle distance L is smaller than the particle diameter $D_L$ of the larger one, interference between particles occurs at the LSPR. For example, there are cases where neighboring particles work together like a large particle to induce LSPR, so a sharp absorption spectrum cannot be made. Meanwhile, although there is no particular problem if the inter-particle distance L is large, since the inter-particle distances L of the metal fine-particles 3 in the dispersed state caused by thermal diffusion closely relates to the particle diameter D of the metal fine-particles 3 and the later-described volume fraction of the metal fine-particles 3, the upper limit for the inter-particle distance L is preferably controlled with the lower limit of the volume fraction of the metal fine-particles 3. When the inter-particle distance L is large, in other words, when the volume fraction of the metal fine-particles 3 relative to the nano-composite 10B or 10C is small, the intensity of the LSPR absorption spectrum becomes small. In such case, by increasing the thickness of the nano-composite 10B or 1OC, the intensity of the LSPR absorption spectrum may be increased.

In addition, the metal fine-particles 3 are 3D-dispersed in the matrix layer 1'. That is, when a cross section in the thickness direction of the matrix layer 1' with a 3D network structure in the nano-composite 10B or 10C and a cross section in a direction orthogonal to the thickness direction, i.e., a cross section parallel to the surface of the matrix layer 1', are observed, as shown in FIGs. 9 and 10, a large number of metal fine-particles 3 are distributed in the vertical direction and the horizontal direction with an inter-particle distance L equal to or greater than the particle diameter $D_L$.

Further, it is preferred that 90% or more of the metal fine-particles 3 are single particles distributed with an inter-particle distance L equal to or greater than the particle diameter $D_L$. Herein, "single particle" means that each metal fine-particle 3 in the matrix layer 1' is present independently, and no aggregate of a plurality of particles (aggregated particle) is not included. That is, the single particles include no aggregated particle in which plural metal fine-particles aggregate by an inter-molecular force. In addition, "aggregated particle" refers to, e.g., an aggregate formed by plural individual metal fine-particles gathering together. This is clearly confirmed by observation with a TEM. Further, though it is understood that the metal fine-particles 3 in the nano-composite 10B or 10C are, in terms of their chemical structure, metal fine-particles formed by aggregated metal atoms that are formed by heat-reduction, such metal fine-particles are considered to be formed through metal bonds between metal atoms and are distinguished from the aggregated particles formed by aggregation of plural particles. For example, when being observed with a TEM, an independent metal fine-particle 3 can be identified.

Since 90% or more of the metal fine-particles 3 are single particles as described above, the LSPR absorption spectrum is sharp and stable, thus achieving high detection accuracy. This situation means that, in other words, aggregated particles or the particles dispersed with an inter-particle distance L equal to or less than the particle diameter $D_L$ account for less than 10%. In a case where such particles are present at 10% or more, the LSPR absorption spectrum gets broad or unstable, and high detection accuracy is difficult to achieve when the nano-composite 10B or 10C is used in a device such as a sensor. In addition, when aggregated particles or the particles dispersed with an inter-particle distance L equal to or less than the particle diameter $D_L$ account for more than 10%, control of the particle diameter D also becomes extremely difficult.

In addition, the volume fraction of the metal fine-particles 3 in the matrix layer 1' is preferably 0.05 to 30% relative to the nano-composite 10B or 10C. Herein, the "volume fraction" is the percentage of the total volume of the metal fine-particles 3 in a certain volume of the nano-composite 10B or 10C including the voids 1b. When the volume fraction of the metal fine-particles 3 is less than 0.05%, the intensity of the LSPR absorption spectrum becomes considerably small. Even if the thickness of the nano-composite 10B or 10C is increased, the effects of the invention are difficult to achieve. Meanwhile, when the volume fraction exceeds 30%, because the spacing (inter-particle distance L) between neighboring metal fine-particles 3 becomes smaller than the particle diameter $D_L$ of the larger one of the neighboring metal fine-particles 3, a sharp peak of the absorption spectrum of LSPR becomes difficult to achieve.

In the nano-composite 10B or 10C of this embodiment, as shown in the above feature d), the metal fine-particles 3 are 3D-dispersed in the matrix layer 1', wherein each metal fine-particle 3 has a portion exposed in the voids 1b of the matrix layer 1'. That is, in the nano-composite 10B or 10C, since the metal fine-particles 3 are 3D-arranged in an efficient way with a high specific surface area, the utilization efficiency of the metal fine-particles 3 may be enhanced. In addition, as each metal fine-particle 3 has a portion exposed in the voids 1b that communicate with the outside environment, the metal fine-particles 3 are also sensitive to the variation in the dielectric constant $\varepsilon_m(\lambda)$ $(=(n_m(\lambda))^2)$ ($n_m$ is the refractive index thereof) of the medium surrounding the metal fine-particles 3 and are capable of making the most of the characteristic that the resonance wavelength varies with the variation in the dielectric constant (or refractive index) of the medium surrounding the metal fine-particles 3. A structural feature of such nano-composite 10B or 10C is that the nano-composite 10B or 10C is most suitable for use in, e.g., frost sensors, moisture sensors, bio-sensors chemical sensors and so on among the applications utilizing LSPR.

In the nano-composite 10B or 10C, when a cross section of the matrix layer 1' is observed using, e.g., a TEM or the like, it is seen that the metal fine-particles 3 in the matrix layer 1' overlap with one another. However, as a matter of fact, the metal fine-particles 3 are dispersed as entirely independent signal particles while maintaining therebetween a distance equal to or greater than a certain value. In addition, due to being physically or chemically immobilized by the solid framework 1a' having a 3D network shape, the metal fine-particles 3 may be prevented from aggregating and falling off with aging, and are excellent in long-term preservability. Even in repeated use of the nano-composite 10B or 10C, aggregation and falling-off of the metal fine-particles 3 are suppressed. For example, in a case where the solid framework 1a' includes an aluminum oxyhydroxide or an alumina hydrate, even under preservation at room temperature for a long time, aggregation of the metal fine-particles 3 is not recognized. Therefore, it is considered that the solid framework 1a' containing an aluminum oxyhydroxide or an alumina hydrate is highly effective in chemically immobilizing the metal fine-particles 3.

In the nano-composite 10B or 10C of this embodiment with the above structure, the metal fine-particles 3 are 3D-dispersed evenly in the matrix layer 1' having a 3D network structure while maintaining an inter-particle distance L equal to or greater than a certain value. For this reason, the LSPR absorption spectrum not only is sharp, but also is very stable and excellent in reproducibility and reliability. Further, because most of the surface of the metal fine-particle 3 is exposed in the voids 1b in the matrix layer 1' that communicate with the outside environment, it is possible to sufficiently  exhibit the characteristic of the metal fine-particles 3 that a resonance wavelength varies with the variation in the dielectric constant (or the refractive index) of the medium surrounding the metal fine-particles 3. Accordingly, the nano-composite 10B or 10C is suitable for use in various sensing devices such as bio-sensors, chemical sensors, moisture sensors, frost sensors and gas sensors, etc. By applying the nano-composite 10B or 10C to the sensing devices, a high-precision detection based on a simple constitution becomes possible. In addition, the nano-composite 10B or 10C may also be applied to various devices such as catalyst filters, fuel cells, air cells, water electrolysis devices, electric double layer capacitors, pollutant gas removal devices, optical recording and regenerating devices, optical information processing devices, energy enhancement devices, high sensitivity photodiode devices, and so on.

<Method for Fabricating Nano-Composite>

Next, a method for fabricating the nano-composites 10B and 10C according to the second embodiment is described. The nano-composites 10B and 10C may be fabricated according to, e.g., the following fabrication methods III and IV.

Fabrication method III:

The fabrication method III of the nano-composite 10B of this embodiment includes the following steps IIIa to IIId:

IIIa) preparing a slurry containing a metal hydroxide or a metal oxide as a raw material of the solid framework;
IIIb) mixing the slurry with a metal compound as a raw material of the metal fine-particles to prepare a coating liquid, wherein the metal compound has an amount, in terms of the metal element, in the range of 0.5 to 480 weight parts relative to 100 weight parts of the solid content of the slurry;
IIIc) coating the coating liquid on a substrate and drying the same to form a coated film; and
IIId) subjecting the coated film to a heating treatment to form, from the coated film, a matrix layer including a solid framework having a 3D network structure and voids defined by the solid framework, and simultaneously to heat-reducing the metal ion of the metal compound to precipitate particle-like metal as the metal fine-particles, so as to obtain the metal fine-particle dispersed composite.

The step IIId is performed in the presence of polyvinyl alcohol.
Next, each step in the fabrication method III is specifically described.
IIIa) The step of preparing a slurry containing a metal hydroxide or a metal oxide as a raw material of the solid framework 1a':
In this embodiment, a representative example, in which the solid framework 1a' in the matrix layer 1' is composed of boehmite (including pseudo-boehmite) containing an aluminum oxyhydroxide (or alumina hydrate), is given. The solid framework 1a' that constitute the matrix layer 1' may be suitably made of commercially available boehmite powder. For example, Boehmite (trade name) produced by Taimei Chemical Co., Ltd., Disperal HP15 (trade name) by CNDEA Corporation, Versal™ Alumina (trade name) by Union Showa K.K., Celasule (trade name) by Kawai Lime Industry Co., Ltd., CAM9010 (trade name) by TOMOE Engineering Co., Ltd., Aluminasol 520 (trade name) by Nissan Chemical Industries, Ltd., Aluminasol-10A(trade name) by Kawaken Fine Chemicals Co., Ltd., SECO Boehmite Alumina (trade name) by SECO International Inc. and so on may be used.
The boehmite (Boehmite) used in an embodiment of the invention refers to fine-particles of an aluminum oxyhydroxide (AlOOH) or an alumina hydrate ($Al_2O_3 \cdot H_2O$) that have high crystallinity, while pseudo-boehmite refers to boehmite fine-particles that have low crystallinity. Nevertheless, both are described as boehmite in a broader sense without distinction. This boehmite powder may be produced by well-known methods such as neutralization of an aluminum salt, hydrolysis of an aluminum alkoxide, and so on. Since the boehmite powder is insoluble in water and resistant to organic solvents, acids and alkalis, it may be advantageously utilized as a component for constituting the solid framework 1a' of the matrix layer 1'. In addition, since the boehmite powder is characterized by having high dispersibility in an acidic aqueous solution, preparing a slurry of the boehmite powder is easy. The boehmite powder used preferably has a particle shape of a cubic shape, a needle shape, a rhombic plate shape, an intermediate shape of these shapes, or a wrinkled-sheet, etc., and has a mean particle diameter in the range of 10 nm to 2 $\mu$m. The solid framework 1a' is formed by bonding the end faces or surfaces of the fine-particles to form a 3D network structure. Furthermore, the mean particle diameter of the boehmite powder herein is derived by a laser diffraction method.

In addition, the boehmite powder as the raw material preferably has a primary particle diameter of 200 nm or less and a secondary particle diameter in the range of 0.025 to 2 $\mu$m, wherein the secondary particle is an aggregate of plural primary particles. By using a raw-material boehmite powder with primary and secondary particle diameter in the above ranges as the main component to form the solid framework 1a' of the matrix layer 1', the dispersibility of the metal fine-particles 3 is improved. When the primary particle diameter of boehmite is greater than 200 nm, the voids 1b tend to become too large. When the secondary particle diameter is less than 0.025 $\mu$m, the 3D network structure of the matrix layer 1' becomes difficult to form. In addition, when the secondary particle diameter is greater than 2 $\mu$m, the diameter of the voids 1b (pore diameter) of the solid framework 1a' may become too large, thus lowering the intensity.

The slurry containing the boehmite powder is obtained by mixing the boehmite powder with water or a polar solvent such as alcohol and then adjusting the mixed solution to be acidic. In the step IIIa, the coating liquid is prepared by adding in this slurry the metal compound as a raw material of the metal fine-particles 3 and then evenly mixing the same.

The slurry is prepared by dispersing the boehmite powder in water or a solvent such as a polar organic solvent, and the boehmite powder used has an amount preferably in the range of 5 to 40 weight parts and more preferably in the range of 10 to 25 weight parts relative to 100 weight parts of the solvent. The solvent used is, for example, water, methanol, ethanol, glycerol, N,N-dimethylformamide, N,N-dimethylacetamide (DMAc) or N-methyl-2-pyrrolidone, etc. It is also possible to use two or more of these solvents in combination. In order to improve the dispersibility of the boehmite powder, the mixed solution is desirably subjected to a dispersion treatment, which may include, e.g., stirring at room temperature for 5 or more minutes, or using ultrasonic wave, etc.

For even dispersion of the boehmite powder, the pH of the mixed solution is adjusted to 5 or less as needed. In this case, as a pH control agent, for example, an organic acid such as formic acid, acetic acid, glycolic acid, oxalic acid, propionic acid, malonic acid, succinic acid, adipic acid, maleic acid, malic acid, tartaric acid, citric acid, benzoic acid, phthalic acid, isophthalic acid, terephthalic acid, glutaric acid, gluconic acid, lactic acid, aspartic acid, glutaminic acid, pimelic acid or suberic acid, an inorganic acid such as hydrochloric acid, nitric acid or phosphoric acid, or a salt of the acid, etc. may be added properly. The pH control agents may be used alone or in combination of two or more. The particle diameter distribution of the boehmite powder may vary due to the addition of the pH control agent as compared to the case without addition of a pH control agent. Nevertheless, there is no particular problem.

IIIb) Mixing the slurry with a metal compound as a raw material of the metal fine-particles 3 to prepare a coating liquid, wherein the metal compound has an amount, in terms of the metal element, in the range of 0.5 to 480 weight parts relative to 100 weight parts of the solid content of the slurry:
In this step, the coating liquid is obtained by further adding the metal compound as a raw material of the metal fine-particles 3 in the slurry prepared as above. In this case, the amount of the metal compound added, in terms of the metal element, is made in the range of 0.5 to 480 weight parts relative to 100 weight parts of the solid content of the slurry. Furthermore, when the metal compound is added to the prepared slurry, the viscosity of the coating liquid may be increased. In such case, the optimal viscosity is desirably achieved by proper addition of the aforementioned solvent.

The metal compound contained in the coating liquid can be one containing the metal species constituting the metal fine-particles 3, with no particular limitation. The metal compound may be a salt or an organic carbonyl complex of the above metal. Example of the metal salt include hydrochlorides, sulfate salts, acetate salts, oxalate salts, citrate salts, and so on. In addition, examples of the organic carbonyl compound forming an organic carbonyl complex with the metal species include: $\beta$-diketones such as acetylacetone, benzoylacetone and dibenzoylmethane, etc., and $\beta$-keto carboxylic esters such as ethyl acetoacetate, etc.

Preferred specific examples of the metal compound are the same as those mentioned in the description of the first embodiment.

To improve the strength, transparency, glossiness and so on of the matrix layer 1', if required, a binder component may be mixed in the slurry or the coating liquid prepared in the step IIIa or IIIb. Suitable examples of the binder component that can be used in combination with an aluminum oxyhydroxide include: gum Arabic; cellulose derivatives, such as carboxymethyl cellulose, hydroxyethyl cellulose and so on; vinyl copolymer latexes, such as SBR latex, NBR latex, functional group-modified polymer latex, ethylene□vinyl acetate copolymer and so on; water-soluble cellulose; polyvinylpyrrolidone; gelatin and modified products thereof, starch and modified products thereof; casein and modified products thereof; maleic anhydride and copolymers thereof; acrylate ester copolymer; polyacrylic acid and copolymers thereof; polyamic acid (precursor of polyimide); and silane compounds, such as tetraethoxysilane, 3-aminopropyltriethoxysilane, 3-aminopropyltrimethoxysilane, N-2-(aminoethyl)-3-aminopropyltrimethoxysilane,

N-2-(aminoethyl)-3-aminopropylmethyldimethoxysilane,

3-triethoxysilyl-N-(1,3-dimethylbutylidene)propylamine,

N-phenyl-3-aminopropyltrimethoxysilane, and so on. These binder components may be used alone or in combination of two or more. Furthermore, with or without a metal compound, these binder components may be mixed properly, in an amount preferably in the range of 30 to 200 weight parts and more preferably in the range of 40 to 100 weight parts relative to 100 weight parts of the solid content of the slurry.

If required, it is also possible to add to the slurry or coating liquid prepared by the step IIIa or IIIb, in addition to the binder, a dispersant, a thickener, a lubricant, a fluidity modifier, a surfactant, an defoaming agent, a water resistant agent, a releasing agent, a fluorescent whitening agent, an ultraviolet absorbent, an anti-oxidant and so on, in a range of not impairing the effects of the invention.

IIIc) Coating the coating liquid prepared by the step IIIb on a substrate and drying the same to form a coated film: The substrate used in the coating is not particularly limited in a case where the nano-composite 10B or 10C is used in a sensor or the like after being peeling off from the substrate, or in a case utilizing light-reflection LSPR with the nano-composite 10B or 10C being attached to the substrate. In the case utilizing light-transmission LSPR with the nano-composite 10B or 10C being attached to the substrate, the substrate is preferably light transmitting, and is, e.g., a glass substrate or a transparent synthetic resin substrate, etc. Examples of the transparent synthetic resin include: polyimide resin, PET resin, acrylic resin, MS resin, MBS resin, ABS resin, polycarbonate resin, silicone resin, siloxane resin, epoxy resin, and so on.

The method of coating the coating liquid is not particularly limited, and may be performed using, e.g., a lip coater, a knife coater, a comma coater, a blade coater, an air knife coater, a roll coater, a curtain coater, a bar coater, a gravure coater, a die coater, a spin coater, or a spray, etc.

After the coating liquid containing the metal compound is coated, it is dried to form a coated film. The drying method is not particularly limited, possibly including heating at a temperature of 60 to 150°C for 1 to 60 minutes. Nevertheless, the drying is preferably performed at a temperature of 70 to 130°C.

IIId) Subjecting the coated film to a heating treatment to form, from the coated film, the matrix layer 1' including the solid framework 1a' with a 3D network structure and the voids defined by the solid framework 1a', and simultaneously to heat-reduce the metal ion of the metal compound to precipitate particle-like metal as the metal fine-particles 3, so as to obtain the nano-composite 10B:

In the step IIId, it is possible to form the matrix layer 1' and simultaneously form and disperse the metal fine-particles 3 through the reduction of the metal ion by one heating step.

In the step IIId, by subjecting the coated film to a heating treatment preferably at 150°C or higher and more preferably at 170°C or higher, the matrix layer 1' is formed. When the temperature of the heating treatment is lower than 150°C, formation of the 3D network structure of the matrix layer 1' may not be sufficiently achieved. The upper limit of the temperature of the heating treatment is preferably in a range not affecting the control over the particle diameter and the inter-particle distance of the metal fine-particles 3 due to decomposition, melting and so on of the metal fine-particles 3, and may be set to be, e.g., 600°C or lower. Further, because of the effect of polyvinyl alcohol, even in a case where the temperature of the heating treatment is high (e.g., in the range of 450 to 600°C), the metal fine-particles formed by heat-reducing the metal ion are not enlarged, so that the particle diameter D is easily controlled.

In addition, the reduction of the metal ion and the dispersion of the precipitated metal fine-particles 3 are performed by a heating treatment preferably at 150 to 600°C, more preferably at 170 to 550°C, and further preferably at 200 to 400°C. When the temperature of the heating treatment is lower than 150°C, the reduction of the metal ion is not sufficient, and it may be difficult to make the mean particle diameter of the metal fine-particles 3 equal to or greater than the aforementioned lower limit (3 nm). In addition, when the temperature of the heating treatment is lower than 150°C, the thermal diffusion of the metal fine-particles 3 precipitated through reduction may not be sufficient in the matrix layer 1'. Further, because of the effect of polyvinyl alcohol, even in a case where the temperature of the heating treatment is high (e.g. in the range of 450 to 600°C), the metal fine-particles formed by heat-reducing the metal ion are not enlarged, and dispersion of the metal fine-particles is enabled. As described above, by performing the heating treatment at a temperature of 150°C or higher, it is possible to form the matrix layer 1' and also precipitate and disperse the metal fine-particles 3 efficiently at the same time.

In the method for fabricating the nano-composite 10B or 10C of this embodiment, the step IIId is performed in the presence of a polyvinyl alcohol. At the time of heat-reduction in the step IIId, by making a polyvinyl alcohol coexist with the metal ion, the particle diameter D of the metal fine-particles 3 may be suppressed to be small, and formation of aggregated particles may be prevented even if the amount of the metal ion in the coated film is increased. The reason is considered to be that, at the time of heat-reducing the metal ion, the polyvinyl alcohol containing a large number of -OH groups becomes an electron donor and functions as a reducing assistant to facilitate the reduction of the metal ion. As a result, more metal nuclei are formed than in the case without a polyvinyl alcohol, and then grow independently to form the metal fine-particles 3. Accordingly, by adding a polyvinyl alcohol as a reducing assistant, the LSPR absorption spectrum of the nano-composite 10B or 10C becomes sharp, and a high-precision detection becomes possible in applications to various sensing devices,.

The polyvinyl alcohol should be added prior to the heating treatment of the coated film in the step IIId. It is preferred to add the polyvinyl alcohol, for example, in the step IIIa of preparing the slurry, or in the step IIIb of preparing the coating liquid. Because polyvinyl alcohol is a water-soluble polymer, it can be easily mixed in the slurry or the coating liquid by, for example, being dissolved in water. Further, after the polyvinyl alcohol is added, it is preferred to evenly stir the slurry or coating liquid.

The polymerization degree of the polyvinyl alcohol used as a reducing assistant is preferably in the range of, for example, 10 to 5000, and more preferably in the range of 50 to 3000. In addition, the molecular weight of the polyvinyl alcohol is preferably in the range of, for example, 440 to 220000, and more preferably in the range of 2200 to 132000. If the polymerization degree or molecular weight of the polyvinyl alcohol is less than the above lower limit, at the fabrication of the nano-composite by heating, the polyvinyl alcohol may evaporate before acting as a reducing assistant. In addition, if the polymerization degree or molecular weight of the polyvinyl alcohol is excessively more than the above upper limit, the polyvinyl alcohol remarkably drops in solubility and may become difficult to be added and mixed in the slurry or coating liquid.

In addition, since the -OH groups generated by saponification effect the reduction of the metal ion, the saponification degree of the polyvinyl alcohol is preferably high, for example, 30% or more, and more preferably 50% or more.

In the reduction reaction, because one -OH group of the polyvinylalcohol can provide two electrons, corresponding to the added amount of the metal compound, the amount of the polyvinylalcohol required for the function of being a reduction assistant of the metal ion can be roughly determined. For example, the reduction of one Au ion of chloroauric acid tetrahydrate requires three electrons. Because one -OH group of the polyvinylalcohol can provide two electrons, on calculation, 3/2 mole of -OH groups of polyvinylalcohol is required for one mole of chloroauric acid tetrahydrate molecule. Accordingly, the required weight ratio (on calculation) of the used polyvinylalcohol to the metal compound can be obtained. However, because the -OH groups of the polyvinylalcohol are not only used for the reduction but also thermally decomposed, the polyvinylalcohol is preferably added in an excess amount relative to the above-calculated weight ratio. On the other hand, if the amount of the added polyvinylalcohol is overly larger than the above-calculated weight ratio, a large amount of the polyvinylalcohol will remain in the nano-composite layer 10B, and there are concerns that certain inconveniences, such as a large amount of excess exhaust gas from the composition of the polyvinylalcohol, may occur. Because of these issues, the amount of the added polyvinylalcohol functioning as a reduction assistant also depends on the saponification degree of the polyvinylalcohol. For example, when the saponification degree of the polyvinylalcohol is 88%, the amount of the added polyvinylalcohol is preferably 0.1 to 50 weight parts and more preferably 0.15 to 20 weight parts relative to 1 weight part of the metal compound.

Next, the formation of the metal fine-particles 3 through heat-reduction is described. The particle diameter D and the inter-particle distance L of the metal fine-particles 3 may be controlled with the heating temperature and heating time in the reduction step and the content of the metal ion in the matrix layer 1'. The inventors have discovered that in cases where the heating temperature and heating time in the heat-reduction are constant, when the absolute amount of the metal ion in the matrix layer 1' differs, the particle diameter D of the precipitated metal fine-particles 3 differs. It has also been discovered that in cases where the heat-reduction is performed without controlling the heating temperature or heating time, the inter-particle distance L is smaller than the particle diameter $D_L$ of the larger one of neighboring fine-particles 3. Further, it has also been discovered that by rendering a polyvinyl alcohol present at heat-reduction, the reduction of the metal ion is facilitated, and more metal nuclei are formed than in cases without using a polyvinyl alcohol, so the particle diameter D of the metal fine-particles 3 can be controlled.

In addition, it is possible to apply the aforementioned findings, for example, to divide the thermal treatment in the reduction step into a plurality of steps for execution. For example, it is possible to perform a particle diameter control step of enabling the metal fine-particles 3 to grow to a predetermined particle diameter D at a first heating temperature, and an inter-particle distance control step of making the inter-particle distance L of the metal fine-particles 3 reach a predetermined range at a second heating temperature the same as or different from the first heating temperature. In this way, the particle diameter D and inter-particle distance L may be more precisely controlled by adjusting the first and second heating temperatures and the heating time.

Heat-reduction is adopted as the reduction method for its industrial advantages, such as that the particle diameter D and inter-particle distance L can be relatively easily controlled by controlling the reduction conditions (especially the heating temperature and the heating time), that simple equipment is applicable from laboratory scale to production scale without particular limitation, and that heat-reduction can be performed in a single-piece manner or a continuous manner without special efforts, etc.. Heat-reduction may be performed in an inert gas atmosphere such as Ar and $N_2$, in a vacuum of 1 to 5 KPa, or in the atmosphere. Vapor-phase reduction using a reductive gas such as hydrogen gas may also be utilized.

In heat-reduction, the metal ion present in the matrix layer 1' is reduced, and the metal fine-particles 3 are independently precipitated by means of thermal diffusion. The metal fine-particles 3 formed in this way maintain the inter-particle distance L equal to or larger than a certain value, and have shapes that are substantially uniform. The metal fine-particles 3 are 3D-dispersed evenly in the matrix layer 1'. Especially, in the case of performing the reduction by this step, the shapes and the particle diameters D of the metal fine-particles 3 are uniformized, so that a nano-composite 10B in which the metal fine-particles 3 are evenly precipitated and dispersed in the matrix layer 1' with a substantially uniform inter-particle distance L is obtained. In addition, by controlling the structural units of the inorganic oxide constituting the matrix layer 1' or controlling the absolute amount of the metal ion and the volume fraction of the metal fine-particles 3, the particle diameter D of the metal fine-particles 3 and the distribution state of the metal fine-particles 3 in the matrix layer 1' may also be controlled.

The method for fabricating a nano-composite in this embodiment may include arbitrary step in addition to the steps IIIa to IIId. For example, the following step IIIe may further be performed after the step IIId.

IIIe) Subjecting the nano-composite 10B obtained by the step IIId to a thermal treatment at a temperature equal to or higher than the temperature at which thermal decomposition of the polyvinyl alcohol starts, so as to obtain a nano-composite 10C:

In the step IIIe, by re-heating the nano-composite 10B, the organic matter (called "polyvinyl alcohol-derived component" hereafter) derived from the remaining polyvinyl alcohol in the nano-composite 10B is removed through thermal decomposition and gasification to obtain a nano-composite 10C. In cases of using the nano-composite in sensors utilizing LSPR, because the polyvinyl alcohol-derived component that remains in the nano-composite 10B decreases the detection sensitivity, it is preferably removed. The temperature at which thermal decomposition of the polyvinyl alcohol-derived component starts is around 200°C. Hence in the step IIIe, the nano-composite 10B is heated at 200°C or higher, preferably at 300°C or higher, and more preferably at 450°C or higher at which the polyvinyl alcohol-derived component will decompose almost completely. The thermal treatment is performed preferably at a temperature in a range not causing any effect such as decomposition or melting, etc. of the solid framework 1a' and the metal fine-particles 3 that constitute the nano-composite 10B. The upper limit for the temperature of the thermal treatment may be set to be, e.g., 600°C or lower. Herein, the organic matter derived from the polyvinyl alcohol include the polyvinyl alcohol not consumed as the reduction assist, for example, a modification product or decomposition product of the polyvinyl alcohol caused by oxidation and so on (for example, conversion of the alcohol moiety to ketone) that change the structure of the polyvinyl alcohol in the heating treatment.

In addition, the heating treatment in the step IIId and the thermal treatment in the step IIIe may be performed at the same time. That is, by performing the heating treatment in one step, while particle-like metal as the metal fine-particles 3 is precipitated by heat-reducing the metal ion of the metal compound, the polyvinyl alcohol-derived component is removed through thermal decomposition and gasification. The lower limit of the temperature of the heating treatment herein is preferably set to be 200°C or higher, and more preferably 300°C or higher. The upper limit of the temperature of the heating treatment is preferably set to be 600°C or lower, and more preferably 550°C or lower.

Fabrication Method IV:

The fabrication method IV of the nano-composite 10B of this embodiment includes the following steps IVa) to IVd):

IVa) preparing a slurry containing a metal hydroxide or a metal oxide as a raw material of the solid framework 1a';
IVb) coating the slurry on a substrate, drying and then subjecting the coated slurry to a heat treatment to form the matrix layer 1' including the solid framework 1a' having a 3D network structure and voids defined by the solid framework 1a';
IVc) impregnating the matrix layer 1' with a solution containing a metal ion as a raw material of the metal fine-particles 3, wherein the metal ion has an amount, in terms of the metal eminent, in the range of 0.2 to 1100 weight parts relative to 100 weight parts of the solid content of the slurry;
IVd) precipitating particle-like metal as the metal fine-particles 3 by reducing the metal ion through a heating treatment after the step IVc;
A polyvinyl alcohol is mixed in the solution containing the metal ion in the step IVc, and the step IVd is performed in the presence of the polyvinyl alcohol.

Next, each step in the fabrication method IV is specifically described.

IVa) Preparing a slurry containing a metal hydroxide or a metal oxide as a raw material of the solid framework 1a' :

The boehmite powder as the raw material preferably has a primary particle diameter of 200 nm or less and a secondary particle diameter in the range of 0.025 to 2 $\mu$m, wherein a secondary particle is an aggregate of plural primary particles. By using the raw-material boehmite powder with primary and secondary particle diameters in the above ranges as the main component to form the solid framework 1a' of the matrix layer 1', the dispersibility of the metal fine-particles 3 is improved. If the primary particle diameter of boehmite is greater than 200 nm, the voids 1b tend to become too large. If the secondary particle diameter is less than 0.025 $\mu$m, the 3D network structure of the matrix layer 1' becomes difficult to be formed. In addition, when the secondary particle diameter is larger than 2 μm, the diameter of the voids 1b (the pore diameter) of the solid framework 1a' may become too large, thus lowering the intensity.

The slurry containing the boehmite powder is obtained by mixing the boehmite powder with water or a polar solvent such as alcohol and then adjusting the mixed solution to be acidic.

The slurry is prepared by dispersing the boehmite powder in water or a solvent such as a polar organic solvent, and the boehmite powder used is preferably made in the range of 5 to 40 weight parts and more preferably in the range of 10 to 25 weight parts relative to 100 weight parts of the solvent. The solvent used is, e.g., water, methanol, ethanol, glycerol, N,N-dimethylformamide, N,N-dimethylacetamide (DMAc) or N-methyl-2-pyrrolidone, etc. It is also possible that two or more of these solvents are used in combination. In order to improve the dispersibility of the boehmite powder, the mixed solution is desirably subjected to a dispersion treatment, which may include, e.g., stirring at room temperature for 5 minutes or longer, or using ultrasonic wave, etc.

If required, the pH of the mixed solution is adjusted to 5 or less to enable even dispersion of the boehmite powder. The pH control agents applicable in this case are the same as those mentioned in the descriptions of the fabrication method III.

To improve the strength, transparency, glossiness and so on of the matrix layer 1', if required, a binder component may be mixed in the slurry prepared by the step IVa. Suitable examples of the binder component that can be used in combination with an aluminum oxyhydroxide include: gum Arabic; cellulose derivatives, such as carboxymethyl cellulose and hydroxyethyl cellulose, etc.; vinyl copolymer latexes, such as SBR latex, NBR latex, functional group-modified polymer latex, and ethylene□vinyl acetate copolymer, etc.; water-soluble cellulose; polyvinylpyrrolidone; gelatin and modified products thereof; starch and modified products thereof; casein and modified products thereof; maleic anhydride or copolymers thereof; acrylate ester copolymers; polyacrylic acid and copolymers thereof; polyamic acid (precursor of polyimide); silane compounds, such as tetraethoxysilane, tetramethoxysilane, methyltrimethoxysilane, methyltriethoxysilane, dimethyldiethoxysilane, phenyltriethoxysilane, hexamethyldisilazane, hexyltrimethoxysilane, decyltrimethoxysilane, $n$-octyltriethoxysilane, 3-aminopropyltriethoxysilane, 3-aminopropyltrimethoxysilane, N-2-(aminoethyl)-3-aminopropyltrimethoxysilane,

N-2-(aminoethyl)-3-aminopropyltriethoxysilane,

N-2-(aminoethyl)-3-aminopropylmethyldimethoxysilane,

3-triethoxysilyl-N-(1,3-dimethylbutylidene)propylamine,

N-phenyl-3-aminopropyltrimethoxysilane,

N-(vinylbenzyl)-2-aminoethyl-3-aminopropyltrimethoxysilane hydrochloride, vinyltrichlorosilane, vinyltrimethoxysilane, vinyltriethoxysilane, vinyltris(2-methoxyethoxy)silane, vinylmethyldimethoxysilane, 2-(3,4-epoxycyclohexyl)ethyltrimethoxysilane, 3-glycidoxypropyltrimethoxysilane, 3-glycidoxypropylmethyldiethoxysilane, 3-glycidoxypropyltriethoxysilane, $p$-styryltrimethoxysilane, 3-methacryloxypropylmethyldimethoxysilane, 3-methacryloxypropyltrimethoxysilane, 3-methacryloxypropylmethyldiethoxysilane, 3-methacryloxypropyltriethoxysilane, 3-acryloxypropyltrimethoxysilane, 3-ureidopropyltriethoxysilane, 3-chloropropyltrimethoxysilane, 3-mercaptopropylmethyldimethoxysilane, 3-mercaptopropyltrimethoxysilane, bis(triethoxysilylpropyl)tetrasulfide, 3-isocyanatepropyltriethoxysilane and 3-isocyanatepropyltrimethoxysilane, etc.; and so on. These binder components may be used alone or in combination of two or more. Furthermore, these binder components may be mixed properly, in an amount preferably in the range of 30 to 200 weight parts and more preferably in the range of 40 to 100 weight parts relative to 100 weight parts of the solid content of the slurry.

Among the above binders, in order to increase the strength of the matrix layer 1', a silane compound having a coupling effect is preferable. The amount of the silane compound mixed is preferably in the range of 10 to 200 weight parts, more preferably in the range of 20 to 100 weight parts, and further preferably in the range of 30 to 80 weight parts, relative to 100 weight parts of the solid content of the slurry. By mixing the silane compound in such amount, the pencil hardness of the matrix layer 1' may be increased to, e.g., 6H or more. In this case, in this embodiment, because the matrix layer 1' is impregnated with a solution containing a metal ion and then subjected to a reduction treatment after being formed, by mixing a large amount of binder such as the above silane compound therein, the hardness of the matrix layer 1' may be sufficiently increased. That is, by adopting the impregnation method, even with incorporation of a binder in a high concentration (e.g., 30 weight parts or more of the binder relative to 100 weight parts of the solid content of the slurry), the surfaces of the resulting metal fine-particles are free from the risk of being covered by the binder. Accordingly, while the strength and durability of the matrix layer 1' are improved due to the addition of the binder in high concentration, a sharp and stable absorption spectrum is obtained without decreasing the efficacy of LSPR.

In addition, if required, it is also possible to add to the slurry prepared by the step IVa, in addition to the binder, a dispersant, a thickener, a lubricant, a fluidity modifier, a surfactant, a defoaming agent, a water resistant additive, a releasing agent, a fluorescent whitening agent, an ultraviolet absorbent, an anti-oxidant and so on, in a range not impairing the effects of the invention.

IVb) Coating the slurry on a substrate, drying and then subjecting the coated slurry to a heating treatment to form the matrix layer 1' including a solid framework having a 3D network structure and voids defined by the solid framework:
The substrate is not particularly limited in a case where the nano-composite 10B or 10C is peeled off from the substrate and used in a sensor or the like, or a case utilizing light-reflection LSPR with the nano-composite 10B or 10C being attached to the substrate. In a case utilizing light-transmission LSPR with the nano-composite 10B or 10C being attached to the substrate, the substrate is preferably light transmitting, and is, e.g., a glass substrate or a transparent synthetic resin substrate, etc. The transparent synthetic resin is, e.g., polyimide resin, PET resin, acrylic resin, MS resin, MBS resin, ABS resin, polycarbonate resin, silicone resin, siloxane resin or epoxy resin, etc.

The method of coating the slurry is not particularly limited, and is performed using, for example, a lip coater, a knife coater, a comma coater, a blade coater, an air knife coater, a roll coater, a curtain coater, a bar coater, a gravure coater, a die coater, a spin coater, or a spray, etc.

After the slurry is coated, it is dried to form a coated film. The drying method is not particularly limited, and may include, for example, heating at a temperature of 60 to 150°C for 1 to 60 minutes. Nevertheless, the drying is preferably performed at a temperature of 70 to 130°C.

In this step, by subjecting the coated film to a heating treatment preferably at 150°C or higher and more preferably at 170°C or higher, the matrix layer 1' is formed. If the temperature of the heating treatment is lower than 150°C, the formation of the 3D network structure of the matrix layer 1' may not be sufficient. The upper limit of the temperature of the heating treatment may be a heat-resistant temperature of the material constituting the matrix layer 1'. Nevertheless, to maintain the voids of the matrix layer 1', the upper limit may be set to be, for example, 600°C or lower.

IVc) Impregnating the matrix layer 1' with a solution containing a metal ion as a raw material of the metal fine-particles 3, wherein the metal ion has an amount, in terms of the metal element, in the range of 0.2 to 1100 weight parts relative to 100 weight parts of the solid content of the slurry:

In this step, the matrix layer 1' prepared in the step IVb is impregnated with a metal ion as a raw material of the metal fine-particles 3. In this case, the amount of the metal ion in terms of the metal element is in the range of 0.2 to 1100 weight parts relative to 100 weight parts of the solid content of the slurry. It is preferred to properly adjust the amount of the metal ion according to the species of the metal element. For example, in a case where the metal element is Au, the amount of the metal ion in terms of the metal element (Au) is preferably in the range of 0.6 to 1100 weight parts relative to 100 weight parts of the solid content of the slurry. If the amount of the metal ion in terms of the metal element is less than 0.2 weight part relative to 100 weight parts of the solid content of the slurry, the volume fraction of the metal fine-particles 3 is decreased and the intensity of the LSPR absorption spectrum becomes considerably low. Even if the thickness of the nano-composite 10B or 10C is increased, the effects of the invention are difficult to achieve. If the amount of the metal ion in terms of the metal element is more than 1100 weight parts relative to 100 weight parts of the solid content of the slurry, the volume fraction of the metal fine-particles 3 becomes too large, so the spacing (inter-particle distance L) between neighboring metal fine-particles 3 becomes smaller than the particle diameter $D_L$ of the larger one of the neighboring fine-particles 3, and a sharp peak of the LSPR absorption spectrum is difficult to achieve.

The metal compound providing the metal ion may be an arbitrary compound containing the metal species constituting the metal fine-particles 3, with no particular limitation. The metal compound may be a salt or an organic carbonyl complex of the above metal. Examples of the metal salt include hydrochlorides, sulfate salts, acetate salts, oxalate salts, and citrate salts, etc. Examples of the organic carbonyl compound that can form an organic carbonyl complex with the metal species include: β-diketones, such as acetylacetone, benzoylacetone and dibenzoylmethane, etc.; β-keto carboxylate esters, such as ethyl acetoacetate, etc.; and so on.

Preferred specific examples of the metal compound are the same as those mentioned in the description of the first embodiment.

The impregnation method is not particularly limited if only it enables at least a surface of the resulting matrix layer 1' to be in contact with the solution containing the metal ion, and a well-known method, such as an immersion method, a spray method, a brush-painting method or a printing method, etc., may be utilized. The impregnation temperature may be 0 to 100°C, preferably a normal temperature around 20 to 40°C. In addition, the impregnation desirably takes, for example, 5 seconds or longer, in cases applying an immersion method.

IVd) Reducing the metal ion through a heating treatment after the step IVc to precipitate particle-like metal as the metal fine-particles 3 to obtain the nano-composite 10B:

The reduction of the metal ion and the dispersion of the precipitated metal fine-particles 3 are performed by a heating treatment preferably at 150 to 600°C, more preferably at 170 to 550°C and further preferably at 200 to 400°C. Herein, if the temperature of the heating treatment is lower than 150°C, the reduction of the metal ion is insufficient, and it may be difficult to make the mean particle diameter of the metal fine-particles 3 equal to or greater than the aforementioned lower limit (3 nm). In addition, if the temperature of the heating treatment is lower than 150°C, the thermal diffusion of the metal fine-particles 3 precipitated through reduction may be insufficient in the matrix layer 1'. Further, because of the effect of the polyvinyl alcohol, even in a case where the temperature of the heating treatment is high (e.g., in the range of 450 to 600°C), the metal fine-particles formed by the heat-reduction of the metal ion are not enlarged, and dispersion of the metal fine-particles can proceed. As described above, by performing a heating treatment at a temperature of 150°C or higher, it is possible to efficiently precipitate and disperse the metal fine-particles 3 in the matrix layer 1'.

In the method for fabricating the nano-composite 10B or 10C of this embodiment, the step IVd is performed in the presence of a polyvinyl alcohol. At the heat-reduction in the step IVd, by making a polyvinyl alcohol coexist with the metal ion, the particle diameter D of the metal fine-particles 3 may be suppressed to be small, and formation of aggregated particles may be prevented even if the amount of the metal ion in the matrix layer 1' is increased,. The reason is considered to be that, at the heat-reduction of the metal ion, the polyvinyl alcohol containing a large number of -OH groups becomes an electron donor and functions as a reducing assistant to facilitate the reduction of the metal ion. As a result, more metal nuclei are formed than in the case without polyvinyl alcohol, and then grow independently to form the metal fine-particles 3. Accordingly, by adding a polyvinyl alcohol as a reducing assistant, the absorption spectrum of LSPR of the nano-composite 10B or 10C becomes sharp, and a high-precision detection becomes possible in applications to various sensing devices.

The polyvinyl alcohol should be added prior to the heat-reduction treatment in the step IVd. The polyvinyl alcohol may be added, e.g., in the stage of the solution containing the metal ion in the step IVc of impregnation with the solution containing the metal ion. By adding a polyvinyl alcohol to the solution containing the metal ion in the step IVc, the LSPR absorption spectrum is made sharp, and it is possible to improve the detection accuracy. Although the reason why the absorption spectrum of LSPR is made sharp by adding a polyvinyl alcohol to the solution containing the metal ion in the step IVc is unknown, a reasonable explanation may be provided based on the following considerations. As mentioned above, at the heat-reduction, the polyvinyl alcohol that contains a large number of -OH groups becomes an electron donor, and is considered to function as a reducing assistant to facilitate formation of metal nuclei. To fully exhibit such function, the polyvinyl alcohol is preferably present adjacent to the resulting metal fine-particles. Accordingly, it is preferred that the polyvinyl alcohol and the metal ion are in a sufficiently mixed state, and it is advantageous that the polyvinyl alcohol is added to the solution containing the metal ion to achieve a mixed state in advance. In addition, after the reduction treatment, by heating at a temperature equal to or higher than the thermal decomposition temperature of polyvinyl alcohol, the polyvinyl alcohol is gasified to disappear. Nevertheless, since polyvinyl alcohol is added to the solution containing the metal ion to achieve a sufficiently mixed state in advance, a large number of voids as vestiges of the polyvinyl alcohol adjacent to the metal fine-particles are formed. Because an exposure space of the metal fine-particles is reserved by these voids, there is apparent variation in optical characteristics caused by LSPR with respect to the change in the surrounding environment, and the efficacy of sensing characteristics is considered to be improved. Furthermore, as also shown from the aforementioned effects of the polyvinyl alcohol, in this embodiment, the polyvinyl alcohol does not function as a binder to reinforce the solid framework 1a' of the matrix layer 1'.

Because polyvinyl alcohol is a water-soluble polymer, it can be easily mixed in the metal ion-containing solution by, e.g., being dissolving in water. Further, after the polyvinyl alcohol is added, it is preferred to evenly stir the ion-containing solution.

The polymerization degree of the polyvinyl alcohol used as a reducing assistant is preferably in the range of, for example, 10 to 5000, and more preferably in the range of 50 to 3000. In addition, the molecular weight of the polyvinyl alcohol is preferably in the range of, for example, 440 to 220000, and more preferably in the range of 2200 to 132000. If the polymerization degree or molecular weight of the polyvinyl alcohol is less than the above lower limit, at the fabrication of the nano-composite by heating, the polyvinyl alcohol may possibly evaporate before acting as a reducing assistant. If the polymerization degree or molecular weight of the polyvinyl alcohol is excessively more than the above upper limit, the polyvinyl alcohol remarkably drops in solubility and may become difficult to be added and mixed in the metal ion-containing solution.

In addition, since the -OH groups formed by saponification effect the reduction of the metal ion, the saponification degree of the polyvinyl alcohol is preferably as high as, for example, 30% or more, and more preferably 50% or more.

In the reduction reaction, because one -OH group of the polyvinyl alcohol can provide two electrons, according to the content of the metal ion in the matrix layer 1' (amount of the metal compound mixed in the slurry), the amount of the polyvinyl alcohol required for the function of being a reduction assistant of the metal ion can be roughly determined. For example, the reduction of one Au ion of chloroauric acid tetrahydrate requires three electrons. Because one -OH group of the polyvinyl alcohol can provide two electrons, on calculation, 3/2 mole of -OH groups of polyvinyl alcohol is required for one mole of chloroauric acid tetrahydrate molecule. Accordingly, the required weight ratio (on calculation) of the used polyvinyl alcohol to the metal compound can be obtained. However, because the -OH groups of the polyvinyl alcohol are not only used for the reduction but also thermally decomposed, the polyvinyl alcohol is preferably added in an excess amount relative to the above-calculated weight ratio. On the other hand, if the amount of the added polyvinyl alcohol is overly larger than the above-calculated weight ratio, a large amount of the polyvinyl alcohol will remain in the nano-composite layer 10B, and there are concerns that certain inconveniences, such as a large amount of excess exhaust gas from the composition of the polyvinyl alcohol, may occur. Because of these issues, the amount of the added polyvinyl alcohol functioning as a reduction assistant also depends on the saponification degree of the polyvinyl alcohol. For example, when the saponification degree of the polyvinyl alcohol is 88%, the amount of the added polyvinyl alcohol is preferably 0.1 to 50 weight parts and more preferably 0.15 to 20 weight parts relative to 1 weight part of the metal compound.

Next, the formation of the metal fine-particles 3 through the heat-reduction is described. The particle diameter D and the inter-particle distance L of the metal fine-particles 3 may be controlled by the heating temperature and heating time in the reduction step and the content of the metal ion with which the matrix layer 1' is impregnated. The inventors have discovered that in cases where the heating temperature and heating time in heat-reduction are constant, when the absolute amount of the metal ion in the matrix layer 1' differs, the particle diameter D of the precipitated metal fine-particles 3 differs. In addition, it has also been discovered that in cases where the heat-reduction is performed without controlling the heating temperature and heating time, the inter-particle distance L is smaller than the particle diameter $D_L$ of the larger one of neighboring metal fine-particles 3. It has also been discovered that due to presence of the polyvinyl alcohol at the heat-reduction, the reduction of the metal ion is facilitated, and more metal nuclei are formed than in the case without using a polyvinyl alcohol, and the particle diameter D of the metal fine-particles 3 may be controlled.

Further, it is possible to apply the above discoveries, for example, to divide the heating treatment in the reduction step into plural steps for execution. For example, it is possible to perform a particle diameter control step enabling the metal fine-particles 3 to grow to a predetermined particle diameter D at a first heating temperature, and an inter-particle distance control step rendering the inter-particle distance L of the metal fine-particles 3 in a predetermined range at a second heating temperature the same as or different from the first heating temperature. In this way, the particle diameter D and inter-particle distance L may be further precisely controlled by adjusting the first and second heating temperatures and the heating time.

Heat-reduction is adopted as the reduction method for industrial advantages, such as that the particle diameter D and the inter-particle distance L are relatively easily controlled by controlling the reduction conditions (especially the heating temperature and the heating time), that simple equipment is applicable from laboratory scale to production scale without a particular limitation, and that heat-reduction can be performed in a single-piece manner or a continuous manner without special efforts, etc.. Heat-reduction may be performed in an inert gas atmosphere such as Ar and $N_2$, in a vacuum of 1 to 5 KPa, or in the atmosphere. Vapor-phase reduction using a reductive gas such as hydrogen gas may also be utilized.

In the heat-reduction, the metal ion in the matrix layer 1' is reduced, and the metal fine-particles 3 are precipitated independently due to thermal diffusion. The metal fine-particles 3 formed in this way maintain an inter-particle distance L equal to or greater than a certain value, and have shapes that are substantially uniform. The metal fine-particles 3 are 3D-dispersed evenly in the matrix layer 1'. Especially, in a case performing heat-reduction in the presence of a polyvinyl alcohol, because the reduction of the metal ion is facilitated, the shapes and particle diameters D of the metal fine-particles 3 are uniformized and small-sized, so that a nano-composite 10B in which most of the metal fine-particles 3 are evenly precipitated and dispersed in the matrix layer 1' with a substantially uniform inter-particle distance L is obtained. In addition, by controlling the structural units including the inorganic oxide constituting the matrix layer 1' and by controlling the absolute amount of the metal ions and the volume fraction of the metal fine-particles 3, the particle diameter D of the metal fine-particles 3 and the distribution state of the same in the matrix layer 1' may also be controlled.

The method for fabricating a nano-composite in this embodiment may include an arbitrary step in addition to the steps IVa to IVd. For example, the following step IVe may further be performed after the step IVd.

IVe) Subjecting the nano-composite 10B obtained in the step IVd to a thermal treatment at a temperature equal to or higher than the temperature at which thermal decomposition of polyvinyl alcohol starts, so as to obtain the nano-composite 10C:

In the step IVe, by re-heating the nano-composite 10B, an organic matter (called "polyvinyl alcohol-derived component" hereafter) derived from the remaining polyvinyl alcohol in the nano-composite 10B is removed through thermal decomposition and gasification to obtain a nano-composite 10C. In cases of applying the nano-composite to sensors utilizing LSPR, because the polyvinyl alcohol-derived component remaining in the nano-composite 10B decreases the detection sensitivity, it is preferably removed. The temperature at which thermal decomposition of the polyvinyl alcohol-derived component starts is around 200°C. Hence in the step IVe, the nano-composite 10B is heated at 200°C or higher, preferably at 300°C or higher, and more preferably at 450°C or higher at which the polyvinyl alcohol-derived component is substantially completely decomposed. The thermal treatment is performed preferably at a temperature in a range of not causing any effects such as decomposition, melting, and so on to the solid framework 1a' and the metal fine-particles 3 that constituting the nano-composite 10B. The upper limit of the temperature of the thermal treatment may be set to, e.g., 600°C or lower. Herein, the organic matter derived from the polyvinyl alcohol include the polyvinyl alcohol not consumed as the reduction assist, for example, a modification product or decomposition product of the polyvinyl alcohol caused by oxidation and so on (for example, conversion of the alcohol moiety to ketone) that change the structure of the polyvinyl alcohol in the heating treatment.

In addition, the heating treatment in the step IVd and the thermal treatment in the step IVe may be performed at the same time. That is, by performing the treatments in one step, while particle-like metal as the metal fine-particles 3 is precipitated by heat-reduction of the metal ion of the metal compound, the polyvinyl alcohol-derived component is removed through thermal decomposition and gasification. The lower limit of the temperature of the heating treatment herein is preferably set to 200°C or higher and more preferably 300°C or higher. The upper limit of the same is preferably set to 600°C or lower and more preferably 550°C or lower.

In the way described above, the nano-composites 10B and 10C may be fabricated. Further, in a case where a metal hydroxide or a metal oxide other than boehmite is used for the matrix layer 1', the above fabrication method may also be used.

As described above, in fabrication methods III and IV of the nano-composites 10B and 10C of this embodiment, by performing heat-reduction of the metal ion in the presence of a polyvinyl alcohol, the polyvinyl alcohol functions as a reducing assistant, and the particle diameter of the metal fine-particles 3 may be suppressed to be small. In addition, by rendering polyvinyl alcohol present at the heat-reduction, even if the amount of the metal ion in the coated film or the matrix layer 1 is increased, formation of aggregated particles may be prevented. Accordingly, the absorption spectrum of LSPR of the nano-composite 10B or 10C becomes sharp, and a high-precision detection becomes possible in applications to various sensing devices.

In addition, in the nano-composite 10B or 10C obtained by the method in this embodiment, the matrix layer 1' forms a 3D network structure including the solid framework 1a' and the voids 1b defined by the solid framework 1a'. Because the metal fine-particles 3 are 3D-dispersed in the matrix layer 1', the absorption spectrum of LSPR is large in intensity. Further, since the metal fine-particles 3 present inside the matrix layer 1' are controlled to have particle diameters in a predetermined range and are dispersed evenly while maintaining a certain inter-particle distance, the absorption spectrum of LSPR is sharp. Further, since each metal fine-particle 3 has a portion exposed in the voids 1b inside the matrix layer 1' having the network structure, it is possible to make the most of the characteristic that the resonance wavelength varies with the variation in the dielectric constant (or the refractive index) of the medium surrounding the metal fine-particles 3, and an application to the devices utilizing this characteristic also becomes possible.

The nano-composite 10B or 10C with the above structural features are suitably used not only in the field utilizing LSPR effect, but also in, e.g., catalysts and electrodes. Its application to electrochemical devices utilizing LSPR is possible, so that fuel cells, air cells, water electrolysis devices, electric double layer capacitors, gas sensors, pollutant gas removal devices and so on may be provided. In addition, since the metal fine-particles 3 in the nano-composite 10B or 10C do not aggregate but are evenly dispersed, the development in various devices, such as optical devices including those for light emission and light modulation, and electronic devices taking advantage of the above characteristic, becomes possible.

Examples

Next, the invention is specifically described according to examples, but the invention is not limited to these examples.

In the following Examples 1-1 to 1-11 and Reference Example 1-1, all sorts of measurements and evaluations are performed in the following manner unless otherwise noted.

[Measurement of Mean Particle Diameter of Metal Fine-Particles]

A measurement of a mean particle diameter of metal fine-particles was performed by cutting a cross section of a sample using a microtome (Leica Ultracut UCT Ultramicrotome, made by Leica Camera AG) to obtain an ultrathin slice and observing the same using a TEM (JEM-2000EX, made by JEOL). Moreover, because it is difficult to observe a sample fabricated on a glass substrate using the above method, the observation was performed on a sample fabricated in the same conditions on a polyimide film. In addition, the mean particle diameter of the metal fine-particles was defined as an area-average diameter.

[Measurement of Void Size of Metal Fine-Particle Dispersed Composite]

The average value of the void size (the pore diameter) of a metal fine-particle dispersed composite was obtained through a pore distribution measurement using a mercury porosimeter method.

[Measurement of Void Proportion of Metal Fine-Particle Dispersed Composite]

The void proportion of a metal fine-particle dispersed composite was calculated using an apparent density (gross density) calculated from the area, thickness and weight of the metal fine-particle dispersed composite, and the density excluding the voids (true density) calculated from the inherent densities and composition ratio of the materials that form the solid framework of the matrix layer and the metal fine-particles, according to the following Eq. (A).

$$\text{Void proportion (\%)} = (1 - \text{gross density/true density}) \times 100 \ \ .....\text{(A)}$$

[Measurement of Absorption Spectrum of Sample]

The absorption spectrum of a fabricated sample was observed using a UV-Vis-NIR spectrophotometer (U-4000, made by Hitachi, Ltd.).

[Example 1-1]

To 6 g of a boehmite powder (trade name: C-01, produced by Taimei Chemicals Co., Ltd, with a mean primary particle diameter of 20 nm, a mean secondary particle diameter of 0.1 $\mu$m and a cubic particle shape), 17 g of water and 0.5 g of acetic acid were added, and a 5-minute ultrasonic treatment was performed. Further, 17 g of ethanol and 1.25 g of chloroauric acid tetrahydrate were added, followed by a 5-minute ultrasonic treatment, thereby preparing a gold complex-containing slurry 1-1. The proportion of Au in the gold complex-containing slurry 1-1 at this moment was 10 weight parts relative to 100 weight parts of boehmite. The resulting gold complex-containing slurry 1-1 was coated on a glass substrate using a spin coater (trade name: Spincoater 1H-DX2, made by Mikasa Co., Ltd.), dried at 70°C for 3 min and at 130°C for 10 min, and then subjected to a heating treatment at 280°C for 10 min, thereby fabricating a metal gold fine-particle-dispersed nano-composite 1-1 of 1.18 $\mu$m thick that displayed a red color. The metal gold fine-particles formed in the nano-composite 1-1 were dispersed entirely independently from each other in the region from the surface portion of the nano-composite 1-1 along the thickness direction, with a distance equal to or greater than the particle diameter of the larger one of neighboring metal gold fine-particles. The characteristics of the nano-composite 1-1 include:

1) a void proportion of 58%, a mean void size of 6 nm, and a maximal void size of 35 nm;
2) a shape of the metal gold fine-particles being substantially spherical, a mean particle diameter of 33 nm, a minimal particle diameter of 15 nm, a maximal particle diameter of 60 nm; a proportion of 100% for the particles having particle diameters of 1 to 100 nm, a mean inter-particle distance of 109 nm, and a volume fraction of 0.65% and a filling proportion of 9.06 wt% for the metal gold fine-particles relative to the nano-composite 1-1; and
3) a volume fraction of 1.1% for the metal gold fine-particles in the nano-composite 1-1 relative to the total volume of the voids in the nano-composite 1.

In addition, the LSPR absorption spectrum of the metal gold fine-particles of the nano-composite 1-1 was observed to have an absorption peak with a peak top at 548 nm, a half-height width of 90 nm, and an absorbance of 0.196 at the wavelength of 600 nm, while the absorption spectrum in water was observed to have an absorption peak with a peak top at 574 nm, a half-height width of 108 nm, and an absorbance of 0.347 at the wavelength of 600 nm. The peak wavelength variation and the peak intensity variation per unit variation of the refractive index of the observed absorption peak were 78.2 nm and 0.442, respectively.

[Example 1-2]

In the same way as in Example 1-1, after a gold complex-containing slurry 1-2 was prepared, the resulting gold complex-containing slurry 1-2 was coated and dried, and then subjected to a heating treatment to fabricate a metal gold fine-particle-dispersed nano-composite 1-2 of 1.83 $\mu$m thick that displayed a red color. The metal gold fine-particles formed in the nano-composite 1-2 were dispersed entirely independently from each other in the region from the surface portion of the nano-composite 1-2 along the thickness direction, with a distance equal to or greater than the particle diameter of the larger one of neighboring metal gold fine-particles. The characteristics of the nano-composite 1-2 include:

1) a void proportion of 56%, a mean void size of 9 nm, and a maximal void size of 120 nm;
2) a shape of the metal gold fine-particles being substantially spherical; a mean particle diameter of 37 nm, a minimal particle diameter of 14 nm, a maximal particle diameter of 61 nm, a proportion of 100% for the particles having particle diameters of 1 to 100 nm, a mean inter-particle distance of 120 nm, and a volume fraction of 0.68% and a filling proportion of 9.06 wt% for the metal gold fine-particles relative to the nano-composite 1-2; and
3) a volume fraction of 1.2% for the metal gold fine-particles in the nano-composite 1-2 relative to the total volume of the voids in the nano-composite 2.

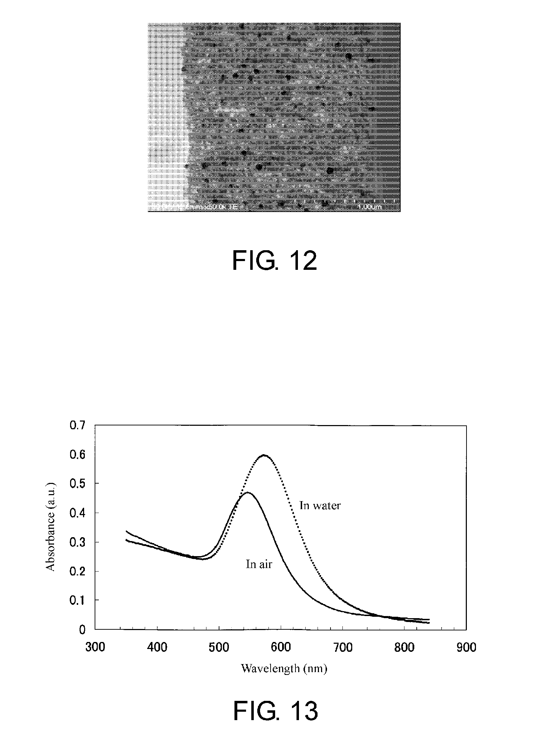

In addition, the LSPR absorption spectrum of the metal gold fine-particles in the nano-composite 1-2 was observed to have an absorption peak with a peak top at 546 nm, a half-height width of 84 nm, and an absorbance of 0.257 at the wavelength of 600 nm, while the absorption spectrum in water was observed to have an absorption peak with a peak top at 572 nm, a half-height width of 105 nm, and an absorbance of 0.517 at the wavelength of 600 nm. The peak wavelength variation and the peak intensity variation per unit variation of the refractive index of the observed absorption peak were 74.9 nm and 0.764, respectively. An image of a surface of the nano-composite 1-2 obtained by observation using a SEM and an image of a cross section of the nano-composite 1-2 obtained by observation using a TEM were shown in FIG. 11 and FIG. 12, respectively. In addition, the absorption spectra of the nano-composite 1-2 measured in air and in water was shown in FIG. 13.

[Example 1-3]

In the same way as in Example 1-1, after a gold complex-containing slurry 1-3 was prepared, the resulting gold complex-containing slurry 1-3 was coated and dried, and then subjected to a heating treatment to fabricate a metal gold fine-particle-dispersed nano-composite 1-3 of 0.81 $\mu$m thick that displayed a red color. The metal gold fine-particles formed in the nano-composite 1-3 were dispersed entirely independently from each other in the region from the surface portion of the nano-composite 1-3 along the thickness direction, with a distance equal to or greater than the particle diameter of the larger one of neighboring metal gold fine-particles. The characteristics of the nano-composite 1-3 include:

   1) a void proportion of 58%, a mean void size of 5 nm, and a maximal void size of 18 nm;
   2) a shape of the metal gold fine-particles being substantially spherical, a mean particle diameter of 31 nm, a minimal particle diameter of 18 nm, a maximal particle diameter of 73 nm; a proportion of 100% for the particles having particle diameters of 1 to 100 nm, a mean inter-particle distance of 103 nm, and a volume fraction of 0.66% and a filling proportion of 9.06 wt% for the metal gold fine-particles relative to the nano-composite 1-3; and
   3) a volume fraction of 1.1% for the metal gold fine-particles in the nano-composite 1-3 relative to the total volume of the voids in the nano-composite 3.

In addition, the LSPR absorption spectrum of the metal gold fine-particles in the nano-composite 1-3 was observed to have an absorption peak with a peak top at 552 nm, a half-height width of 94 nm, and an absorbance of 0.161 at the wavelength of 600 nm, while the absorption spectrum in water was observed to have an absorption peak with a peak top at 582 nm, a half-height width of 122 nm, and an absorbance of 0.247 at the wavelength of 600 nm. The peak wavelength variation and the peak intensity variation per unit variation of the refractive index of the observed absorption peak were 89.7 nm and 0.224, respectively.

[Example 1-4]

Except that 11.25 g instead of 1.25 g of chloroauric acid tetrahydrate in Example 1-1 was used, in the same way as in Example 1-1, after a gold complex-containing slurry 1-4 was prepared, the resulting gold complex-containing slurry 1-4 was coated and dried, and then subjected to a heating treatment to fabricate a metal gold fine-particle-dispersed nano-composite 1-4 of 1.10 $\mu$m thick that displayed a red color. The proportion of Au in the gold complex-containing slurry 1-4 at this moment was 90 weight parts relative to 100 weight parts of boehmite. In addition, the metal gold fine-particles formed in the nano-composite 1-4 were dispersed entirely independently from each other in the region from the surface portion of the nano-composite 1-4 along the thickness direction, with a distance equal to or greater than the particle diameter of the larger one of neighboring metal gold fine-particles. The characteristics of the nano-composite 1-4 include:

   1) a void proportion of 64%, a mean void size of 6 nm, and a maximal void size of 20 nm;
   2) a shape of metal gold fine-particles being substantially spherical, a mean particle diameter of 91 nm, a minimal particle diameter of 28 nm, a maximal particle diameter of 167 nm, a proportion of 64% for the particles having particle diameters of 1 to 100 nm, a mean inter-particle distance of 114 nm, and a volume fraction of 4.5% and a filling proportion of 47.28 wt% for the metal gold fine-particles relative to the nano-composite 1-4; and
   3) a volume fraction of 7.0% for the metal gold fine-particles in the nano-composite 1-4 relative to the total volume of the voids in the nano-composite 1-4.

In addition, the LSPR absorption spectrum of the metal gold fine-particles of the nano-composite 1-4 was observed to have an absorption peak with a peak top at 562 nm, a half-height width of 162 nm, and an absorbance of 1.132 at the wavelength of 600 nm, while the absorption spectrum in water was observed to have an absorption peak with a peak top at 586 nm, a half-height width of 216 nm, and an absorbance of 1.215 at the wavelength of 600 nm. The peak wavelength variation and the peak intensity variation per unit variation of the refractive index of the observed absorption peak were 69.2 nm and 0.226, respectively.

[Example 1-5]

Except that 33.75 g instead of 1.25 g of chloroauric acid tetrahydrate in Example 1-1 was used, in the same way as in Example 1-1, after a gold complex-containing slurry 1-5 was prepared, the resulting gold complex-containing slurry 1-5 was coated and dried, and then subjected to a heating treatment to fabricate a metal gold fine-particle-dispersed nano-composite 1-5 of 0.60 $\mu$m thick that displayed a red color. The proportion of Au in the gold complex-containing slurry 1-5 at this moment was 270 weight parts relative to 100 weight parts of boehmite. In addition, the metal gold fine-particles formed in the nano-composite 1-5 were dispersed entirely independently from each other in the region from the surface portion of the nano-composite 1-5 along the thickness direction, with a distance equal to or greater than the particle diameter of the larger one of neighboring metal gold fine-particles. The characteristics of the nano-composite 1-5 include:

1) a void proportion of 81%, a mean void size of 6 nm; a maximal void size of 55 nm;
2) a shape of the metal gold fine-particles being substantially spherical, a mean particle diameter of 63 nm, a minimal particle diameter of 26 nm, a maximal particle diameter of 95 nm, a proportion of 100% for the particles having particle diameters of 1 to 100 nm, a mean inter-particle distance of 70 nm, and a volume fraction of 5.6% and a filling proportion of 72.9 wt% for the metal gold fine-particles relative to the nano-composite 1-5; and
3) a volume fraction of 6.9% for the metal gold fine-particles in the nano-composite 1-5 relative to the total volume of the voids in the nano-composite 5.

In addition, the LSPR absorption spectrum of the metal gold fine-particles in the nano-composite 1-5 was observed to have an absorption peak with a peak top at 540 nm, a half-height width of 114 nm, and an absorbance of 0.351 at the wavelength of 600 nm, while the absorption spectrum in water was observed to have an absorption peak with a peak top at 574 nm, a half-height width of 160 nm, and an absorbance of 0.414 at the wavelength of 600 nm. The peak wavelength variation and the peak intensity variation per unit variation of the refractive index of the observed absorption peak were 101.3 nm and 0.185, respectively.

[Example 1-6]

To 6 g of boehmite powder (trade name: C-01, produced by Taimei Chemicals Co., Ltd., with a mean primary particle diameter of 20 nm, a mean secondary particle diameter of 0.1 $\mu$m and a cubic particle shape), 11.5 g of water and 0.5 g of acetic acid were added, and a 5-min ultrasonic treatment was performed. Then, 22.6 g of ethanol and 0.60 g of $\gamma$-aminopropyltriethoxysilane were added and stirred, 1.25 g of chloroauric acid tetrahydrate was added, and then a 5-min ultrasonic treatment was performed to prepare a gold complex-containing slurry 1-6.
In the same way as in Example 1-1, the resulting gold complex-containing slurry 1-6 was coated and dried, and then subjected to a heating treatment to fabricate a metal gold fine-particle-dispersed nano-composite 1-6 of 2.85 $\mu$m thick that displayed a red color. The metal gold fine-particles formed in the nano-composite 1-6 were dispersed entirely independently from each other in the region from the surface portion of the nano-composite 1-6 along the thickness direction, with a distance equal to or greater than the particle diameter of the larger one of neighboring metal gold fine-particles. The characteristics of the nano-composite 1-6 include:

1) a void proportion of 58%, a mean void size of 8 nm, and a maximal void size of 110 nm;
2) a shape of the metal gold fine-particles being substantially spherical, a mean particle diameter of 35 nm, a minimal particle diameter of 12 nm, a maximal particle diameter of 55 nm, a proportion of 100% for the particles having particle diameters of 1 to 100 nm, a mean inter-particle distance of 117 nm, and a volume fraction of 0.66% and a filling proportion of 8.84 wt% for the metal gold fine-particles relative to the nano-composite 1-6; and
3) a volume fraction of 1.1% for the metal gold fine-particles in the nano-composite 1-6 relative to the total volume of the voids in the nano-composite 1-6.

In addition, the LSPR absorption spectrum of the metal gold fine-particles of the nano-composite 1-6 was observed to have an absorption peak with a peak top at 543 nm, a half-height width of 94 nm, and an absorbance of 0.339 at the wavelength of 600 nm, while the absorption spectrum in water was observed to have an absorption peak with a peak top at 558 nm, a half-height width of 100 nm, and an absorbance of 0.456 at the wavelength of 600 nm. The peak wavelength variation and the peak intensity variation per unit variation of the refractive index of the observed absorption peak were 46.5 nm and 0.352, respectively.

[Example 1-7]

To 6 g of boehmite powder (trade name: C-01, produced by Taimei Chemicals Co., Ltd., with a mean primary particle diameter of 20 nm; a mean secondary particle diameter of 0.1 $\mu$m, and a cubic particle shape), 17 g of water and 0.5 g of acetic acid were added, and a 5-min ultrasonic treatment was performed. Then, 17 g of ethanol was added, and then a 5-min ultrasonic treatment was performed to prepare a slurry 1-7. The resulting slurry 1-7 was coated on a glass substrate using a spin coater (trade name: Spincoater 1H-DX2, made by Mikasa Co., Ltd.), dried at 70°C for 3 min and at 130°C for 10 min, and then subjected to a heating treatment at 280°C for 10 min to fabricate a matrix layer 1-7 of 1.55 $\mu$m thick.

The matrix layer 1-7 was immersed in a 2.5 wt% aqueous solution of chloroauric acid tetrahydrate for 10 minutes to be impregnated with the same. Then, the excess aqueous solution of chloroauric acid tetrahydrate was removed by air blow and a heating treatment was performed at 280°C for 10 min to fabricate a metal gold fine-particle-dispersed nano-composite 1-7 that displayed a red color. The proportion of Au at this moment was about 3 weight parts relative to 100 weight parts of boehmite. The metal gold fine-particles formed in the nano-composite 1-7 were dispersed entirely independently from each other in the region from the surface portion of the nano-composite 1-7 along the thickness direction, with a distance equal to or greater than the particle diameter of the larger one of neighboring metal gold fine-particles. The characteristics of the nano-composite 1-7 include:

1) a void proportion of 60%, a mean void size of 6 nm, and a maximal void size of 16 nm;
2) a shape of the metal gold fine-particles being substantially spherical, a mean particle diameter of 30 nm, a minimal particle diameter of 8 nm, a maximal particle diameter of 52 nm, a proportion of 100% for the particles having particle diameters of 1 to 100 nm, a mean inter-particle distance of 167 nm, and a volume fraction of 0.18% and a filling proportion of 2.79 wt% for the metal gold fine-particles relative to the nano-composite 1-7; and
3) a volume fraction of 0.3% for the metal gold fine-particles in the nano-composite 1-7 relative to the total volume of the voids in the nano-composite 1-7.

In addition, the LSPR absorption spectrum of the metal gold fine-particles of the nano-composite 1-7 was observed to have an absorption peak with a peak top at 540 nm, a half-height width of 85 nm, and an absorbance of 0.102 at the wavelength of 600 nm, while the absorption spectrum in water was observed to have an absorption peak with a peak top at 560 nm, a half-height width of 99 nm, and an absorbance of 0.142 at the wavelength of 600 nm. The peak wavelength variation and the peak intensity variation per unit variation of the refractive index of the observed absorption peak were 57.6 nm and 0.115, respectively.

[Example 1-8]

Except that the immersion was performed in a 10 wt% aqueous solution of chloroauric acid tetrahydrate for 10 min instead of in the 2.5 wt% aqueous solution of chloroauric acid tetrahydrate for 10 min as in Example 1-7, in the same way as in Example 1-7, a metal gold fine-particle-dispersed nano-composite 1-8 displaying a red color was fabricated. The proportion of Au at this moment was about 11 weight parts relative to 100 weight parts of boehmite. The metal gold fine-particles formed in the nano-composite 1-8 were dispersed entirely independently from each other in the region from the surface portion of the nano-composite 1-8 along the thickness direction, with a distance equal to or greater than the particle diameter of the larger one of neighboring metal gold fine-particles. The characteristics of the nano-composite 1-8 include:

1) a void proportion of 60%, a mean void size of 6 nm, and a maximal void size of 16 nm;
2) a shape of the metal gold fine-particles being substantially spherical, a mean particle diameter of 43 nm, a minimal particle diameter of 14 nm, a maximal particle diameter of 65 nm, a proportion of 100% for the particles having particle diameters of 1 to 100 nm, a mean inter-particle distance of 139 nm, and a volume fraction of 0.71% and a filling proportion of 10.29 wt% for the metal gold fine-particles relative to the nano-composite 1-8; and
3) a volume fraction of 1.2% for the metal gold fine-particles in the nano-composite 1-8 relative to the total volume of the voids in the nano-composite 1-8.

In addition, the LSPR absorption spectrum of the metal gold fine-particles of the nano-composite 1-8 was observed to have an absorption peak with a peak top at 552 nm, a half-height width of 96 nm, and an absorbance of 0.295 at the wavelength of 600 nm, while the absorption spectrum in water was observed to have an absorption peak with a peak top at 582 nm, a half-height width of 116 nm, and an absorbance of 0.523 at the wavelength of 600 nm. The peak wavelength variation and the peak intensity variation per unit variation of the refractive index of the observed absorption peak were 86.5 nm and 0.691, respectively.

[Example 1-9]

<Fabrication of LSPR Inducing Substrate 1-1>

To 6 g of boehmite powder (trade name: C-01, produced by Taimei Chemicals Co., Ltd., with a mean primary particle diameter of 20 nm, a mean secondary particle diameter of 0.1 $\mu$m, and a cubic particle shape), 17 g of water and 0.5 g of acetic acid were added, and a 5-min ultrasonic treatment was performed. Then, 17 g of ethanol, 0.6 g of 3-aminopropyltriethoxysilane and 1.25 g of chloroauric acid tetrahydrate were added, and then a 5-min ultrasonic treatment was performed to prepare a gold complex-containing slurry 1-9. The proportion of Au in the gold complex-containing slurry 1-9 at this moment was 10 weight parts relative to 100 weight parts of boehmite.

Next, the resulting gold complex-containing slurry 1-9 was coated on a glass face of a substrate (12 cm square) having a three-layer structure of Ni-Cr alloy film of 193 nm thick/Ag film of 233 nm thick/transparent glass substrate of 0.7 mm thick using a spin coater (trade name: Spincoater 1H-DX2, made by Mikasa Co., Ltd.), dried at 70°C for 3 min and at 130°C for 10 min, and then subjected to a heating treatment at 280°C for 10 min to fabricate a LSPR inducing substrate 1-1 containing a metal gold fine-particle-dispersed nano-composite 1-9 of 1.80 $\mu$m thick that displayed a red color.

The metal gold fine-particles formed in the nano-composite 1-9 were dispersed entirely independently from each other in the region from the surface portion of the nano-composite 1-9 along the thickness direction, with a distance equal to or greater than the particle diameter of the larger one of neighboring metal gold fine-particles. The characteristics of the nano-composite 1-9 include:

1) a void proportion of 58%, a mean void size of 8 nm, and a maximal void size of 110 nm;
2) a shape of the metal gold fine-particles being substantially spherical, a mean particle diameter of 34 nm, a minimal particle diameter of 12 nm, a maximal particle diameter of 54 nm, a proportion of 100% for the particles having particle diameters of 1 to 100 nm, a mean inter-particle distance of 117 nm, and a volume fraction of 0.66% and a filling proportion of 8.84 wt% for the metal gold fine-particles relative to the nano-composite 1-9; and
3) a volume fraction of 1.1% for the metal gold fine-particles in the nano-composite 1-9 relative to the total volume of the voids in the nano-composite 1-9.

In addition, the reflection absorption spectrum of the LSPR of the metal gold fine-particles in the LSPR inducing substrate 1-1 was observed to have an absorption peak with a peak top at 565 nm, a half-height width of 157 nm, and an absorbance of 0.510 at the wavelength of 600 nm, while the absorption spectrum in water was observed to have an absorption peak with a peak top at 603 nm, a half-height width of 204 nm, and an absorbance of 0.768 at the wavelength of 600 nm. The peak wavelength variation and the peak intensity variation per unit variation of the refractive index of the observed absorption peak were 115.2 nm and 0.782, respectively.

[Example 1-10]

<Fabrication of LSPR Inducing Substrate 1-2>

Except that a substrate (12 cm square) having a two-layer structure of A1 film of 190 nm thick/transparent glass substrate of 0.7 mm thick was used instead of the substrate (12 cm square) having a three-layer structure of Ni-Cr alloy film of 193 nm thick/Ag film of 233 nm thick/transparent glass substrate of 0.7 mm thick, in the same way as in Example 1-9, a LSPR inducing substrate 1-2 was fabricated.

The reflection absorption spectrum of the LSPR of the metal gold fine-particles in the LSPR inducing substrate 1-2 was observed to have an absorption peak with a peak top at 564 nm, a half-height width of 163 nm, and an absorbance of 0.421 at the wavelength of 600 nm, while the absorption spectrum in water was observed to have an absorption peak with a peak top at 594 nm, a half-height width of 204 nm, and an absorbance of 0.638 at the wavelength of 600 nm. The peak wavelength variation and the peak intensity variation per unit variation of the refractive index of the observed absorption peak were 90.9 nm and 0.651, respectively.

Reference Example 1-1

Except that a transparent glass substrate of 0.7 mm thick was used instead of the substrate (12 cm square) having a three-layer structure of Ni-Cr alloy film of 193 nm thick/Ag film of 233 nm thick/transparent glass substrate of 0.7 mm thick, in the same way as in Example 1-9, a nano-composite 1-10 was fabricated. The reflection absorption spectrum of LSPR of the metal gold fine-particles in the nano-composite 1-10 was observed to have an absorption peak with a peak top at 572 nm, a half-height width of 154 nm, and an absorbance of 0.079 at the wavelength of 600 nm, while the absorption spectrum in water was observed to have an absorption peak with a peak top at 572 nm, a half-height width of 242 nm, and an absorbance of 0.100 at the wavelength of 600 nm. The peak wavelength variation and the peak intensity variation per unit variation of the refractive index of the observed absorption peak were 0 nm and 0.064, respectively.

Preparation Example 1-1

3 mg of powder reagent of N-succinimidyl biotin (trade name: Biotin Sulfo-OSu, produced by Dojindo Laboratories) was dissolved in 3 ml of phosphate buffered saline (a mixed aqueous solution of 150 mM of sodium chloride, 7.5 mM of disodium hydrogen phosphate and 2.9 mM of sodium dihydrogen phosphate) to prepare a biotin solution 1-1 of 1 mg/ml.

Preparation Example 1-2

1 mg of a powder reagent of avidin (trade name: Avidin from egg white, produced by Nacalai Tesque) was dissolved in 10 ml of phosphate buffered saline (a mixed aqueous solution of 150 mM of sodium chloride, 7.5 mM of disodium hydrogen phosphate and 2.9 mM of sodium dihydrogen phosphate) to prepare an avidin solution 1-2 of 1.47 $\mu$M.

[Example 1-11]

The nano-composite 1-2 obtained in Example 1-2 was immersed in a 0.1 mM (0.1 mmol/L) ethanol solution of amino undecanethiol hydrochloride as a binding species and treated at 23°C for 2 hours, and was then cleaned with ethanol and dried to prepare a nano-composite 1-11a.

Next, the nano-composite 1-11a was immersed in the biotin solution 1-1 of Preparation Example 1-1 and treated at 23°C for 2 hours, and was then cleaned with a phosphate buffered saline and then immersed in a phosphate buffered saline to fabricate a nano-composite 1-11 b in which N-succinimidyl biotin was further immobilized by the binding species of the nano-composite 1-11a. The absorption spectrum of 1-11b in the phosphate buffered saline was observed to have an absorption peak with a peak top at 574 nm and an absorbance of 0.505 at the wavelength of 600 nm.

The above nano-composite 1-11b was immersed in the avidin solution 1-2 of Preparation Example 1-2 and treated by stirring at 23°C for 2 hours, and was then cleaned with a phosphate buffered saline and immersed in a phosphate buffered saline to obtain a nano-composite 1-11c in which avidin was absorbed on the biotin part of the binding species in the nano-composite 1-11b. The absorption spectrum of 1-11c in phosphate buffered saline was observed to have an absorption peak with a peak top of 577 nm and an absorbance of 0.529 at the wavelength of 600 nm.

In the following Examples 2-1 to 2-10, Reference Examples 2-1 to 2-10, Examples 3-1 to 3-2, and Reference Examples 3-1 to 3-3, all sorts of measurements and evaluations are performed in the following manner unless otherwise noted.

[Measurement of Mean Particle Diameter of Metal Fine-Particles]

The measurement of a mean particle diameter of metal fine-particles was made by breaking a sample into pieces and dispersing them in ethanol, dripping the resulting dispersion liquid to a metallic mesh including a carbon-supporting film to obtain a substrate, and observing the same using a TEM (JEM-2000EX, made by JEOL). In addition, the mean particle diameter of the metal fine-particles was defined as an area-average diameter.

[Measurement of Void Size of Metal Fine-Particle Dispersed Composite]

The average value of the void size (pore diameter) of a metal fine-particle dispersed composite was obtained by a pore distribution measurement using a mercury porosimeter method.

[Measurement of Void Proportion of Metal Fine-Particle Dispersed Composite]

The void proportion of a metal fine-particle dispersed composite was calculated from the apparent density (gross density) calculated from the area, thickness and weight of the metal fine-particle dispersed composite, and the density excluding the voids (real density) calculated from the inherent densities and the composition ratio of the materials forming the solid framework of the matrix layer and the metal fine-particles, according to the following expression (A).

$$\text{Void proportion (\%)} = (1\text{-gross density/real density}) \times 100 \quad .....(A)$$

[Measurement of Absorption Spectrum of Sample]

The absorption spectrum of a fabricated nano-composite sample was observed using an instantaneous multi-channel photo-detector (MCPD-3700, made by Otsuka Electronics Co., Ltd.).

[Example 2-1]

To 0.6 g of boehmite powder (trade name: C-01, produced by Taimei Chemicals Co., Ltd., with a mean primary particle diameter of 20 nm, a mean secondary particle diameter of 0.1 $\mu$m, and a cubic particle shape), 2.84 g of water and 0.05 g of acetic acid were added, and a 5-min ultrasonic treatment was performed. Then, 3.40 g of ethanol, 0.06 g of 3-aminopropyltriethoxysilane, 0.125 g of polyvinyl alcohol (with an average molecular weight of 22000, a polymerization degree of 500, and a saponification degree of 88%) dissolved in 0.50 g of pure water, and 0.125 g of chloroauric acid tetrahydrate were added, and then a 5-min ultrasonic treatment was performed to prepare a gold complex-containing slurry 2-1. The proportion of Au in the gold complex-containing slurry 2-1 at this moment was 10 weight parts relative to 100 weight parts of boehmite. In addition, there was 8.24 mol of hydroxyl group in the mixed polyvinyl alcohol relative to 1 mol of chloroauric acid tetrahydrate.

Next, the resulting gold complex-containing slurry 2-1 was coated on a glass substrate of 0.7 mm thick using a spin coater (trade name: Spincoater 1H-DX2, made by Mikasa Co., Ltd.), dried at 70°C for 3 min and at 130°C for 10 min, and then subjected to a heating treatment at 280°C for 10 minutes to fabricate a metal gold fine-particle-dispersed nano-composite 2-1A of 1.61 $\mu$m thick that displayed a reddish purple color. The metal gold fine-particles formed in the nano-composite 2-1A were dispersed entirely independently from each other in the region from the surface portion of the nano-composite 2-1A along the thickness direction, with a distance equal to or greater than the particle diameter of the larger one of neighboring metal gold fine-particles. The characteristics of the nano-composite 2-1A include:

1) a void proportion of 54.2%;
2) a shape of the metal gold fine-particles being substantially spherical, a mean particle diameter of 10.2 nm, a minimal particle diameter of 2 nm, a maximal particle diameter of 36 nm, a proportion of 100% for the particles having particle diameters of 1 to 100 nm, a mean inter-particle distance of 34.3 nm, and a volume fraction of 0.63% and a filling proportion of 8.56 wt% for the metal gold fine-particles relative to the nano-composite 2-1A; and
3) a volume fraction of 1.16% for the gold fine-particles in the nano-composite 2-1A relative to the total volume of the voids in the nano-composite 2-1A:.

In addition, the reflection absorption spectrum of the LSPR of the metal gold fine-particles in the nano-composite 2-1A was observed to have an absorption peak with a peak top at 549 nm, a half-height width of 114 nm, and an absorbance of 0.467 at the peak top, while the absorption spectrum in water was observed to have an absorption peak with a peak top at 559 nm, a half-height width of 126 nm, and an absorbance of 0.469 at the peak top. The peak wavelength variation and the peak intensity variation per unit variation of the refractive index of the observed absorption peak were 32.1 nm and 0.001, respectively.

By further subjecting the resulting nano-composite 2-1A to a heating treatment at 500°C for 1 hour, a metal gold fine-particle-dispersed nano-composite 2-1B displaying a red color was fabricated. The metal gold fine-particles formed in the nano-composite 2-1B were dispersed entirely independently from each other in the region from the surface portion of the nano-composite 2-1B along the thickness direction, with a distance equal to or greater than the particle diameter of the larger one of neighboring metal gold fine-particles. The characteristics of the nano-composite 2-1B include:

1) a void proportion of 58.0%;
2) a shape of the metal gold fine-particles being substantially spherical, a mean particle diameter of 11.9 nm, a minimal particle diameter of 3 nm, a maximal particle diameter of 40 nm, a proportion of 100% for the particles having particle diameters of 1 to 100 nm, a mean inter-particle distance of 40.0 nm, and a volume fraction of 0.63% and a filling proportion of 8.87 wt% for the metal gold fine-particles relative to the nano-composite 2-1B; and
3) a volume fraction of 1.09% for the gold fine-particles in the nano-composite 2-1B relative to the total volume of the voids in the nano-composite 2-1B.

In addition, the reflection absorption spectrum of the LSPR of the metal gold fine-particles in the nano-composite 2-1B was observed to have an absorption peak with a peak top at 535 nm, a half-height width of 88 nm, and an absorbance of 0.359 at the peak top, while the absorption spectrum in water was observed to have an absorption peak with a peak top at 550 nm, a half-height width of 101 nm, and an absorbance of 0.446 at the peak top. The peak wavelength variation and the peak intensity variation per unit variation of the refractive index of the observed absorption peak were 43.2 nm and 0.259, respectively.

[Example 2-2]

To 0.6 g of boehmite powder (trade name: C-01, produced by Taimei Chemicals Co., Ltd., with a mean primary particle diameter of 20 nm, a mean secondary particle diameter of 0.1 $\mu$m, and a cubic particle shape), 2.28 g of water and 0.05 g of acetic acid were added, and a 5-min ultrasonic treatment was performed. Then, 3.40 g of ethanol, 0.06 g of 3-aminopropyltriethoxysilane, 0.25 g of polyvinyl alcohol (with an average molecular weight of 22000, a polymerization degree of 500, and a saponification degree of 88%) dissolved in 1.00 g of pure water and 0.25 g of chloroauric acid tetrahydrate were added, and then a 5-minute ultrasonic treatment was performed to prepare a gold complex-containing slurry 2-2. The proportion of Au in the gold complex-containing slurry 2-2 at this moment was 20 weight parts relative to 100 weight parts of boehmite. In addition, there was 8.24 mol of hydroxyl group in the mixed polyvinyl alcohol relative to 1 mol of chloroauric acid tetrahydrate.

In the same way as in Example 2-1, the resulting gold complex-containing slurry 2-2 was coated and dried, and then subjected to a heating treatment at 280°C for 10 min to fabricate a metal gold fine-particle-dispersed nano-composite 2-2A of 1.61 $\mu$m thick that displayed a dark reddish purple color. The metal gold fine-particles formed in the nano-composite 2-2A were dispersed entirely independently from each other in the region from the surface portion of the nano-composite 2-2A along the thickness direction, with a distance equal to or greater than the particle diameter of the larger one of neighboring metal gold fine-particles. The characteristics of the nano-composite 2-2A include:

1) a void proportion of 49.6%;
2) a shape of the metal gold fine-particles being substantially spherical, a mean particle diameter of 14.4 nm, a minimal particle diameter of 5 nm, a maximal particle diameter of 3 7 nm, a proportion of 100% for the particles having particle diameters of 1 to 100 nm, a mean inter-particle distance of 35.5 nm, and volume fraction of 1.26% and a filling proportion of 15.26 wt% for the metal gold fine-particles relative to the nano-composite 2-2A; and
3) a volume fraction of 2.54% for the gold fine-particles in the nano-composite 2-2A relative to the total volume of the voids in the nano-composite 2-2A.

In addition, the reflection absorption spectrum of the LSPR of the metal gold fine-particles of the nano-composite 2-2A was observed to have an absorption peak with a peak top at 551 nm, a half-height width of 98 nm, and an absorbance of 1.106 at the peak top, while the absorption spectrum in water was observed to have an absorption peak with a peak top at 556 nm, a half-height width of 102 nm, and an absorbance of 1.149 at the peak top. The peak wavelength variation and the peak intensity variation per unit variation of the refractive index of the observed absorption peak were 16.1 nm and 0.136, respectively.

By further subjecting the resulting nano-composite 2-2A to a heating treatment at 500°C for 1 hour, a metal gold fine-particle-dispersed nano-composite 2-2B displaying a red color was fabricated. The metal gold fine-particles formed in the nano-composite 2-2B were dispersed entirely independently from each other in the region from the surface portion of the nano-composite 2-2B along the thickness direction, with a distance equal to or greater than the particle diameter of the larger one of neighboring metal gold fine-particles. The characteristics of the nano-composite 2-2B include:

1) a void proportion of 57.4%;
2) a shape of the metal gold fine-particles being substantially spherical, a mean particle diameter of 17.9 nm, a minimal particle diameter of 6 nm, a maximal particle diameter of 40 nm, a proportion of 100% for the particles having particle diameters of 1 to 100 nm, a mean inter-particle distance of 44.1 nm, and a volume fraction of 1.26% and a filling proportion of 16.3 wt% for the metal gold fine-particles relative to the nano-composite 2-2B; and
3) a volume fraction of 2.19% for the gold fine-particles in the nano-composite 2-2B relative to the total volume of the voids in the nano-composite 2-2B.

In addition, the reflection absorption spectrum of the LSPR of the metal gold fine-particles in the nano-composite 2-2B was observed to have an absorption peak with a peak top at 529 nm, a half-height width of 75 nm, and an absorbance of 0.741 at the peak top, while the absorption spectrum in water was observed to have an absorption peak with a peak top at 539 nm, a half-height width of 81 nm, and an absorbance of 0.981 at the peak top. The peak wavelength variation and the peak intensity variation per unit variation of the refractive index of the observed absorption peak were 30.5 nm and 0.709, respectively.

[Example 2-3]

To 18 g of boehmite powder (trade name: C-01, produced by Taimei Chemicals Co., Ltd., with a mean primary particle diameter of 20 nm, a mean secondary particle diameter of 0.1 $\mu$m, and a cubic particle shape), 78.72 g of water and 3.28 g of acetic acid were added, and then a mechanical stirring (rotation speed: 400 rpm, 3 hours) was performed to prepare a boehmite dispersed liquid 2-3. Next, relative to 3.5 g of the boehmite dispersed liquid 2-3, 2.33 g of ethanol, 0.394 g of polyvinyl alcohol (with an average molecular weight of 22000, a polymerization degree of 500, and a saponification degree of 88%) dissolved in 1.575 g of pure water, 0.063 g of 3-aminopropyltriethoxysilane, and 0.263 g of chloroauric acid tetrahydrate dissolved in 2 g of ethanol were added to prepare a gold complex-containing slurry 2-3. In the preparation of the slurry 2-3, every time the respective reagents were added, a stirring bar-based stirring (rotation speed: 1000 rpm, 5 min) was performed. The proportion of Au in the gold complex-containing slurry 2-3 at this moment was 20 weight parts relative to 100 weight parts of boehmite. In addition, there was 12.3 mol of hydroxyl group in the mixed polyvinyl alcohol relative to 1 mol of chloroauric acid tetrahydrate.

Next, the resulting gold complex-containing slurry 2-3 was coated on a glass substrate of 0.7 mm thick using a spin coater (trade name: Spincoater 1H-DX2, made by Mikasa Co., Ltd.), dried at 70°C for 3 min and at 130°C for 10 min, and then subjected to heat treatments at 280°C for 10 min and at 500°C for 1 hour to fabricate a metal gold fine-particle-dispersed nano-composite 2-3A of 1.52 $\mu$m thick that displayed a red color. The metal gold fine-particles formed in the nano-composite 2-3A were dispersed entirely independently from each other in the region from the surface portion of the nano-composite 2-3A along the thickness direction, with a distance equal to or greater than the particle diameter of the larger one of neighboring metal gold fine-particles. The characteristics of the nano-composite 2-3A include:

1) a void proportion of 66.1 %, a mean void size of 24 nm, and a maximal void size of 50 nm;
2) a shape of the metal gold fine-particles being substantially spherical, a mean particle diameter of 6 nm, a minimal particle diameter of 2 nm, a maximal particle diameter of 42 nm, a proportion of 100% for the particles having particle diameters of 1 to 100 nm, a mean inter-particle distance of 16.5 nm, and a volume fraction of 1.0% and a filling proportion of 16.3 wt% for the metal gold fine-particles relative to the nano-composite 2-3A; and
3) a volume fraction of 1.5% for the gold fine-particles in the nano-composite 2-3A relative to the total volume of the voids in the nano-composite 2-3A.

In addition, the reflection absorption spectrum of the LSPR of the metal gold fine-particles in the nano-composite 2-3A was observed to have an absorption peak with a peak top at 524 nm, a half-height width of 70.1 nm, and an absorbance of 0.573 at the peak top, while the absorption spectrum in water was observed to have an absorption peak with a peak top at 541 nm, a half-height width of 83.1 nm, and an absorbance of 0.908 at the peak top. The peak wavelength variation and the peak intensity variation per unit variation of the refractive index of the observed absorption peak were 52.3 nm and 1.009, respectively.

[Example 2-4]

Except that boehmite powder (trade name: SECO-140, produced by SECO, with a mean primary particle diameter of 14 nm, a mean secondary particle diameter of 0.17 $\mu$m, and a needle-like particle shape) was used, in the same way as in Example 2-3, a gold complex-containing slurry 2-4 was prepared. The gold complex-containing slurry 2-4 was coated and dried, and then subjected to a heating treatment to fabricate a metal gold fine-particle-dispersed nano-composite 2-4A of 1.63 $\mu$m thick that displayed a red color. The metal gold fine-particles formed in the nano-composite 2-4A were dispersed entirely independently from each other in the region from the surface portion of the nano-composite 2-4A along the thickness direction, with a distance equal to or greater than the particle diameter of the larger one of neighboring metal gold fine-particles. The characteristics of the nano-composite 2-4A include:

1) a void proportion of 67.9%, a mean void size of 16 nm, and a maximal void size of 30 nm;
2) a shape of the metal gold fine-particles being substantially spherical, a mean particle diameter of 10 nm, a minimal particle diameter of 4 nm, a maximal particle diameter of 57 nm, a proportion of 100% for the particles having particle diameters of 1 to 100 nm, a mean inter-particle distance of 27.3 nm, and a volume fraction of 0.95% and a filling proportion of 16.3 wt% for the metal gold fine-particles relative to the nano-composite 2-4A; and
3) a volume fraction of 1.4% for the gold fine-particles in the nano-composite 2-4A relative to the total volume of the voids in the nano-composite 2-4A.

In addition, the reflection absorption spectrum of the LSPR of the metal gold fine-particles in the nano-composite 2-4A was observed to have an absorption peak with a peak top at 526 nm, a half-height width of 74.0 nm, and an absorbance of 0.574 at the peak top, while the absorption spectrum in water was observed to have an absorption peak with a peak top at 542 nm, a half-height width of 87.0 nm, and an absorbance of 0.862 at the peak top. The peak wavelength variation and the peak intensity variation per unit variation of the refractive index of the observed absorption peak were 47.7 nm and 0.862, respectively.

[Example 2-5]

Except that boehmite powder (trade name: SECO-100, produced by SECO, with a mean primary particle diameter of 10 nm, a mean secondary particle diameter of 0.15 $\mu$m, and a needle-like particle shape) was used, in the same way as in Example 2-3, a gold complex-containing slurry 2-5 was prepared. The gold complex-containing slurry 2-5 was coated and dried, and then subjected to a heating treatment to fabricate a metal gold fine-particle-dispersed nano-composite 2-5A of 1.76 $\mu$m thick that displayed a red color. The metal gold fine-particles formed in the nano-composite 2-5A were dispersed entirely independently from each other in the region from the surface portion of the nano-composite 2-5A along the thickness direction, with a distance equal to or greater than the particle diameter of the larger one of neighboring metal gold fine-particles. The characteristics of the nano-composite 2-5A include:

1) a void proportion of 65.6%, a mean void size of 12 nm, and a maximal void size of 20 nm;
2) a shape of the metal gold fine-particles being substantially spherical, a mean particle diameter of 10 nm, a minimal particle diameter of 4 nm, a maximal particle diameter of 67 nm, a proportion of 100% for the particles having particle diameters of 1 to 100 nm, a mean inter-particle distance of 29.3 nm, and a volume fraction of 1.02% and a filling proportion of 16.3 wt% for the metal gold fine-particles relative to the nano-composite 2-5A; and
3) a volume fraction of 1.6% for the gold fine-particles in the nano-composite 2-5A relative to the total volume of the voids in the nano-composite 2-5A.

In addition, the reflection absorption spectrum of the LSPR of the metal gold fine-particles in the nano-composite 2-5A was observed to have an absorption peak with a peak top at 525 nm, a half-height width of 68.5 nm, and an absorbance of 0.67 at the peak top, while the absorption spectrum in water was observed to have an absorption peak with a peak top at 540 nm, a half-height width of 79.2 nm, and an absorbance of 1.008 at the peak top. The peak wavelength variation and the peak intensity variation per unit variation of the refractive index of the observed absorption peak were 46.5 nm and 1.03, respectively.

[Example 2-6]

Except that boehmite powder (trade name: SECO-080, produced by SECO, with a mean primary particle diameter of 8 nm, a mean secondary particle diameter of 0.12 $\mu$m, and a needle-like particle shape) was used, in the same way as in Example 2-3, a gold complex-containing slurry 2-6 was prepared. The resulting gold complex-containing slurry 2-6 was coated and dried, and then subjected to a heating treatment to fabricate a metal gold fine-particle-dispersed nano-composite 2-6A of 1.78 $\mu$m thick that displayed a red color. The metal gold fine-particles formed in the nano-composite 2-6A were dispersed entirely independently from each other in the region from the surface portion of the nano-composite 2-6A along the thickness direction, with a distance equal to or greater than the particle diameter of the larger one of neighboring metal gold fine-particles. The characteristics of the nano-composite 2-6A include:

1) a void proportion of 64.1%, a mean void size of 9 nm, and a maximal void size of 30 nm;
2) a shape of the metal gold fine-particles being substantially spherical, a mean particle diameter of 10 nm, a minimal particle diameter of 4 nm, a maximal particle diameter of 48 nm, a proportion of 100% for the particles having particle diameters of 1 to 100 nm, a mean inter-particle distance of 27.2 nm, and a volume fraction of 1.06% and a filling proportion of 16.3 wt% for the metal gold fine-particles relative to the nano-composite 2-6A; and
3) a volume fraction of 1.7% for the gold fine-particles in the nano-composite 2-6A relative to the total volume of the voids in the nano-composite 2-6A.

In addition, the reflection absorption spectrum of the LSPR of the metal gold fine-particles in the nano-composite 2-6A was observed to have an absorption peak with a peak top at 527 nm, a half-height width of 73.8 nm, and an absorbance of 0.757 at the peak top, while the absorption spectrum in water was observed to have an absorption peak with a peak top at 540 nm, a half-height width of 83.2 nm, and an absorbance of 1.082 at the peak top. The peak wavelength variation and the peak intensity variation per unit variation of the refractive index of the observed absorption peak were 39.1 nm and 0.997, respectively.

[Example 2-7]

To 18 g of boehmite powder (trade name: SECO-045D, produced by SECO, with a mean primary particle diameter of 5 nm, a mean secondary particle diameter of 0.025 μm, and a needle-like particle shape), 82 g of water was added, and then a mechanical stirring (rotation speed: 400 rpm, 3 hours) was performed to prepare a boehmite dispersed liquid 2-7. Next, relative to 2 g of the boehmite dispersed liquid 2-7, 0.068 g of acetic acid, 3.08 g of ethanol, 2.54 g of water, 0.225 g of polyvinyl alcohol (with an average molecular weight of 22000, a polymerization degree of 500, and a saponification degree of 88%) dissolved in 0.9 g of pure water, 0.036 g of 3-aminopropyltriethoxysilane, and 0.15 g of chloroauric acid tetrahydrate dissolved in 2 g of ethanol were added to prepare a gold complex-containing slurry 2-7. Further, in the preparation of the slurry 2-7, every time the respective reagents were added, a stirring bar-based stirring (rotation speed: 1000 rpm, 5 min) was performed. The proportion of Au in the gold complex-containing slurry 2-7 at this moment was 20 weight parts relative to 100 weight parts of boehmite. In addition, there was 12.3 mol of hydroxyl group in the mixed polyvinyl alcohol relative to 1 mol of chloroauric acid tetrahydrate.

Next, the resulting gold complex-containing slurry 2-7 was coated on a glass substrate of 0.7 mm thick using a spin coater (trade name: Spincoater 1H-DX2, made by Mikasa Co., Ltd.), dried at 70°C for 3 min and at 130°C for 10 min, and then subjected to heat treatments at 280°C for 10 min and at 500°C for 1 hour to fabricate a metal gold fine-particle-dispersed nano-composite 2-7A of 1.56 μm thick that displayed a red color. The metal gold fine-particles formed in the nano-composite 2-7A were dispersed entirely independently from each other in the region from the surface portion of the nano-composite 2-7A along the thickness direction, with a distance equal to or greater than the particle diameter of the larger one of neighboring metal gold fine-particles. The characteristics of the nano-composite 2-7A include:

1) a void proportion of 49.4%, a mean void size of 8 nm, and a maximal void size of 500 nm or more;
2) a shape of the metal gold fine-particles being substantially spherical, a mean particle diameter of 12 nm, a minimal particle diameter of 4 nm, a maximal particle diameter of 29 nm, a proportion of 100% for the particles having particle diameters of 1 to 100 nm, a mean inter-particle distance of 26.8 nm, and a volume fraction of 1.5% and a filling proportion of 16.3 wt% for the metal gold fine-particles relative to the nano-composite 2-7A; and
3) a volume fraction of 3.0% for the gold fine-particles in the nano-composite 2-7A relative to the total volume of the voids in the nano-composite 2-7A.

In addition, the reflection absorption spectrum of the LSPR of the metal gold fine-particles in the nano-composite 2-7A was observed to have an absorption peak with a peak top at 535 nm, a half-height width of 72.7 nm, and an absorbance of 0.581 at the peak top, while the absorption spectrum in water was observed to have an absorption peak with a peak top at 542 nm, a half-height width of 75.3 nm, and an absorbance of 0.701 at the peak top. The peak wavelength variation and the peak intensity variation per unit variation of the refractive index of the observed absorption peak were 21.8 nm and 0.423, respectively.

[Example 2-8]

Except boehmite powder (trade name: SECO-045U, produced by SECO, with a mean primary particle diameter of 5 nm, a mean secondary particle diameter of 0.025 μm, and a needle-like particle shape) was used, in the same way as in Example 2-7, a boehmite dispersed liquid 2-8 was prepared. Next, relative to 3.5 g of the boehmite dispersed liquid 2-8, 0.119 g of acetic acid, 2.445 g of ethanol, 0.394 g of a polyvinyl alcohol (with an average molecular weight of 22000, a polymerization degree of 500, and a saponification degree of 88%) dissolved in 1.575 g of pure water, 0.06 g of 3-aminopropyltriethoxysilane, and 0.263 g of chloroauric acid tetrahydrate were added to prepare a gold complex-containing slurry 2-8. Further, in the preparation of the slurry 2-8, every time the respective reagents were added, a stirring bar-based stirring (rotation speed: 1000 rpm, 5 min) was performed. The proportion of Au in the gold complex-containing slurry 2-8 at this moment was 20 weight parts relative to 100 weight parts of boehmite. In addition, there was 12.3 mol of hydroxyl group in the mixed polyvinyl alcohol relative to 1 mol of chloroauric acid tetrahydrate.

Next, the resulting gold complex-containing slurry 2-8 was coated on a glass substrate of 0.7 mm thick using a spin coater (trade name: Spincoater 1H-DX2, made by Mikasa Co., Ltd.), dried at 70°C for 3 min and at 130°C for 10 min, and then subjected to heat treatments at 280°C for 10 min and at 500°C for 1 hour to fabricate a metal gold fine-particle-dispersed nano-composite 2-8A of 1.65 μm thick that displayed a red color. The metal gold fine-particles formed in the nano-composite 2-8A were dispersed entirely independently from each other in the region from the surface portion of the nano-composite 2-8A along the thickness direction, with a distance equal to or greater than the particle diameter of the larger one of neighboring metal gold fine-particles. The characteristics of the nano-composite 2-8A include:

1) a void proportion of 52.0%, a mean void size of 8 nm, and a maximal void size of 500 nm or more;
2) a shape of the metal gold fine-particles being substantially spherical, a mean particle diameter of 10 nm, a minimal particle diameter of 3 nm, a maximal particle diameter of 34 nm, a proportion of 100% for the particles having particle diameters of 1 to 100 nm, a mean inter-particle distance of 24.0 nm, and a volume fraction of 1.42% and a filling proportion of 16.3 wt% for the metal gold fine-particles relative to the nano-composite 2-8A; and

3) a volume fraction of 2.7% for the gold fine-particles in the nano-composite 2-8A relative to the total volume of the voids in the nano-composite 2-8A.

In addition, the reflection absorption spectrum of the LSPR of the metal gold fine-particles in the nano-composite 2-8A was observed to have an absorption peak with a peak top at 533 nm, a half-height width of 68.5 nm, and an absorbance of 0.873 at the peak top, while the absorption spectrum in water was observed to have an absorption peak with a peak top at 544 nm, a half-height width of 75.2 nm, and an absorbance of 1.193 at the peak top. The peak wavelength variation and the peak intensity variation per unit variation of the refractive index of the observed absorption peak were 35 nm and 0.984, respectively.

[Example 2-9]

To 72 g of an aqueous solution of 25 wt% boehmite (trade name: Nanoboehmite b, produced by Kawai Lime Industry Co., Ltd., with an average long diameter of primary particles of 100 nm, an average short diameter of primary particles of 15 nm, a mean secondary particle diameter of 0.35 $\mu$m, and a needle-like particle shape), 24.72 g of water and 3.28 g of acetic acid were added, then a mechanical stirring (rotation speed: 400 rpm, 3 hours) was performed to prepare a boehmite dispersed liquid 2-9. Next, relative to 2 g of the boehmite dispersed liquid 2-9, 2.95 g of ethanol, 2.47 g of water, 0.225 g of polyvinyl alcohol (with an average molecular weight of 22000, a polymerization degree of 500, and a saponification degree of 88%) dissolved in 0.9 g of pure water, 0.036 g of 3-aminopropyltriethoxysilane, and 0.15 g of chloroauric acid tetrahydrate dissolved in 2 g of ethanol were added to prepare a gold complex-containing slurry 2-9. Further, in the preparation of the slurry 2-9, every time the respective reagents were added, a stirring bar-based stirring (rotation speed: 1000 rpm, 5 min) was performed. The proportion of Au in the gold complex-containing slurry 2-9 at this moment was 20 weight parts relative to 100 weight parts of boehmite. In addition, there was 12.3 mol of hydroxyl group in the mixed polyvinyl alcohol relative to 1 mol of chloroauric acid tetrahydrate.

Next, the resulting gold complex-containing slurry 2-9 was coated on a glass substrate of 0.7 mm thick using a spin coater (trade name: Spincoater 1H-DX2, made by Mikasa Co., Ltd.), dried at 70°C for 3 min and at 130°C for 10 min, and then subjected to heat treatments at 280°C for 10 min and at 500°C for 1 hour to fabricate a metal gold fine-particle-dispersed nano-composite 2-9A of 1.73 $\mu$m thick that displayed a red color. The metal gold fine-particles formed in the nano-composite 2-9A were dispersed entirely independently from each other in the region from the surface portion of the nano-composite 2-9A along the thickness direction, with a distance equal to or greater than the particle diameter of the larger one of neighboring metal gold fine-particles. The characteristics of the nano-composite 2-9A include:

1) a void proportion of 55.8%, a mean void size of 8 nm, and a maximal void size of 20 nm;
2) a shape of the metal gold fine-particles being substantially spherical, a mean particle diameter of 7 nm, a minimal particle diameter of 3 nm, a maximal particle diameter of 32 nm, a proportion of 100% for the particles having particle diameters of 1 to 100 nm, a mean inter-particle distance of 17.3 nm, and a volume fraction of 1.31% and a filling proportion of 16.3 wt% for the metal gold fine-particles relative to the nano-composite 2-9A; and
3) a volume fraction of 2.4% for the gold fine-particles in the nano-composite 2-9A relative to the total volume of the voids in the nano-composite 2-9A.

In addition, the reflection absorption spectrum of the LSPR of the metal gold fine-particles in the nano-composite 2-9A was observed to have an absorption peak with a peak top at 524 nm, a half-height width of 69.8 nm, and an absorbance of 0.712 at the peak top, while the absorption spectrum in water was observed to have an absorption peak with a peak top at 537 nm, a half-height width of 72.5 nm, and an absorbance of 1.023 at the peak top. The peak wavelength variation and the peak intensity variation per unit variation of the refractive index of the observed absorption peak were 39.1 nm and 0.959, respectively.

[Example 2-10]

Except that an aqueous solution of 25 wt% boehmite (trade name: BMJ, produced by Kawai Lime Industry Co., Ltd., with a mean primary particle diameter of 100 nm, a mean secondary particle diameter of 0.106 $\mu$m, and a plate-like particle shape) was used, in the same way as in Example 2-7, a boehmite dispersed liquid 2-10 was prepared. Next, relative to 3.5 g of the boehmite dispersed liquid 2-10, 2.33 g of ethanol, 0.394 g of a polyvinyl alcohol (with an average molecular weight of 22000, a polymerization degree of 500, and a saponification degree of 88%) dissolved in 1.575 g of pure water, 0.063 g of 3-aminopropyltriethoxysilane, and 0.263 g of chloroauric acid tetrahydrate dissolved in 2 g of ethanol were added to prepare a gold complex-containing slurry 2-10. Further, in the preparation of the slurry 2-10, every time the respective reagents were added, a stirring bar-based stirring (rotation speed: 1000 rpm, 5 min) was performed. The proportion of Au in the gold complex-containing slurry 2-10 at this moment was 20 weight parts relative to 100 weight parts of boehmite. In addition, there was 12.3 mol of hydroxyl group in the mixed polyvinyl alcohol relative to 1 mol of chloroauric acid tetrahydrate.

Next, the resulting gold complex-containing slurry 2-10 was coated on a glass substrate of 0.7 mm thick using a spin coater (trade name: Spincoater 1H-DX2, made by Mikasa Co., Ltd.), dried at 70°C for 3 min and at 130°C for 10 min, and then subjected to heat treatments at 280°C for 10 min and at 500°C for 1 hour to fabricate a metal gold fine-particle-dispersed nano-composite 2-10A of 1.65 $\mu$m thick that displayed a red color. The metal gold fine-particles formed in the nano-composite 2-10A were dispersed entirely independently from each other in the region from the surface portion of the nano-composite 2-10A along the thickness direction, with a distance equal to or greater than the particle diameter of the larger one of neighboring metal gold fine-particles. The characteristics of the nano-composite 2-10A include:

1) a void proportion of 53.7%, a mean void size of 24 nm, and a maximal void size of 500 nm or more;
2) a shape of the metal gold fine-particles being substantially spherical, a mean particle diameter of 16 nm, a minimal particle diameter of 2 nm, a maximal particle diameter of 39 nm, a proportion of 100% for the particles having particle diameters of 1 to 100 nm, a mean inter-particle distance of 37.9 nm, and a volume fraction of 1.37% and a filling proportion of 16.3 wt% for the metal gold fine-particles relative to the nano-composite 2-10A; and
3) a volume fraction of 2.6% for the gold fine-particles in the nano-composite 2-10A relative to the total volume of the voids in the nano-composite 2-10A.

In addition, the reflection absorption spectrum of the LSPR of the metal gold fine-particles in the nano-composite 2-10A was observed to have an absorption peak with a peak top at 517 nm, a half-height width of 70.1 nm, and an absorbance of 0.60 at the peak top, while the absorption spectrum in water was observed to have an absorption peak with a peak top at 532 nm, a half-height width of 73.2 nm, and an absorbance of 0.907 at the peak top. The peak wavelength variation and the peak intensity variation per unit variation of the refractive index of the observed absorption peak were 44.9 nm and 0.948, respectively.

[Reference Example 2-1]

To 0.6 g of boehmite powder (trade name: C-01, produced by Taimei Chemicals Co., Ltd., with a mean primary particle diameter of 20 nm, a mean secondary particle diameter of 0.1 $\mu$m, and a cubic particle shape), 1.70 g of water and 0.05 g of acetic acid were added, and a 5-min ultrasonic treatment was performed. Then, 1.70 g of ethanol, 0.06 g of 3-aminopropyltriethoxysilane, and 0.125 g of chloroauric acid tetrahydrate were added, and then a 5-min ultrasonic treatment was performed to prepare a gold complex-containing slurry 2R-1. The proportion of Au in the gold complex-containing slurry 2R-1 at this moment was 10 weight parts relative to 100 weight parts of boehmite.

In the same way as in Example 2-1, the resulting gold complex-containing slurry 2R-1 was coated and dried, and then subjected to a heating treatment at 280°C for 10 min to fabricate a metal gold fine-particle-dispersed nano-composite 2R-1 of 1.53 $\mu$m thick that displayed a red color. The metal gold fine-particles formed in the nano-composite 2R-1 were dispersed entirely independently from each other in the region from the surface portion of the nano-composite 2R-1 along the thickness direction, with a distance equal to or greater than the particle diameter of the larger one of neighboring metal gold fine-particles. The characteristics of the nano-composite 2R-1 include:

1) a void proportion of 58%, a mean void size of 8 nm, and a maximal void size of 110 nm;
2) a shape of the metal gold fine-particles being substantially spherical, a mean particle diameter of 46.8 nm, a minimal particle diameter of 28 nm, a maximal particle diameter of 65 nm, a proportion of 100% for the particles having particle diameters of 1 to 100 nm, a mean inter-particle distance of 157.4 nm, and a volume fraction of 0.63% and a filling proportion of 8.87 wt% for the metal gold fine-particles relative to the nano-composite 2R-1; and
3) a volume fraction of 1.09% for the gold fine-particles in the nano-composite 2R-1 relative to the total volume of the voids in the nano-composite 2R-1.

In addition, the reflection absorption spectrum of the LSPR of the metal gold fine-particles in the nano-composite 2R-1 was observed to have an absorption peak with a peak top at 558 nm, a half-height width of 127 nm, and an absorbance of 0.373 at the peak top, while the absorption spectrum in water was observed to have an absorption peak with a peak top at 588 nm, a half-height width of 164 nm, and an absorbance of 0.406 at the peak top. The peak wavelength variation and the peak intensity variation per unit variation of the refractive index of the observed absorption peak were 90.0 nm and 0.103, respectively.

[Reference Example 2-2]

To 0.6 g of boehmite powder (trade name: C-01, produced by Taimei Chemicals Co., Ltd., with a mean primary particle diameter of 20 nm, a mean secondary particle diameter of 0.1 μm, and a cubic particle shape), 1.70 g of water and 0.05 g of acetic acid were added, and a 5-min ultrasonic treatment was performed. Then, 1.70 g of ethanol, 0.06 g of 3-aminopropyltriethoxysilane, and 0.25 g of chloroauric acid tetrahydrate were added, and then a 5-min ultrasonic treatment was performed to prepare a gold complex-containing slurry 2R-2. The proportion of Au in the gold complex-containing slurry 2R-2 at this moment was 20 weight parts relative to 100 weight parts of boehmite.

In the same way as in Example 2-1, the resulting gold complex-containing slurry 2R-2 was coated and dried, and then subjected to a heating treatment at 280°C for 10 min to fabricate a metal gold fine-particle-dispersed nano-composite 2R-2 of 1.59 μm thick that displayed a reddish purple color. The metal gold fine-particles formed in the nano-composite 2R-2 were dispersed entirely independently from each other in the region from the surface of the nano-composite 2R-2 along the thickness direction, with a distance equal to or greater than the particle diameter of the larger one of neighboring metal gold fine-particles. The characteristics of the nano-composite 2R-2 include:

1) a void proportion of 57.4%;
2) a shape of the metal gold fine-particles being substantially spherical, a mean particle diameter of 67.3 nm, a minimal particle diameter of 37 nm, a maximal particle diameter of 110 nm, a proportion of 88.2% for the particles having particle diameters of 1 to 100 nm, a mean inter-particle distance of 165.8 nm, and a volume fraction of 1.26% and a filling proportion of 16.3 wt% for the metal gold fine-particles relative to the nano-composite 2R-2; and
3) a volume fraction of 2.19% for the gold fine-particles in the nano-composite 2R-2 relative to the total volume of the voids in the nano-composite 2R-2.

In addition, the reflection absorption spectrum of the LSPR of the metal gold fine-particles in the nano-composite 2R-2 was observed to have an absorption peak with a peak top at 575 nm, a half-height width of 142 nm, and an absorbance of 0.707 at the peak top, while the absorption spectrum in water was observed to have an absorption peak with a peak top at 604 nm, a half-height width of 179 nm, and an absorbance of 0.788 at the peak top. The peak wavelength variation and the peak intensity variation per unit variation of the refractive index of the observed absorption peak were 60.8 nm and 0.236, respectively.

[Reference Example 2-3]

Except that a polyvinyl alcohol was not added, a gold complex-containing slurry 2R-3 was prepared in the same way as in Example 2-3. In the same way as in Example 2-3, the resulting gold complex-containing slurry 2R-3 was coated and dried, and then subjected to a heating treatment to fabricate a metal gold fine-particle-dispersed nano-composite 2R-3 of 1.53 μm thick that displayed a reddish purple color. The metal gold fine-particles formed in the nano-composite 2R-3 were dispersed entirely independently from each other in the region from the surface portion of the nano-composite 2R-3 along the thickness direction, with a distance equal to or greater than the particle diameter of the larger one of neighboring metal gold fine-particles. The characteristics of the nano-composite 2R-3 include:

1) a void proportion of 66.1 %, a mean void size of 24 nm, and a maximal void size of 50 nm;
2) a shape of the metal gold fine-particles being substantially spherical, a mean particle diameter of 88.6 nm, a minimal particle diameter of 63.5 nm, a maximal particle diameter of 119.3 nm, a proportion of 80% for the particles having particle diameters of 1 to 100 nm, a mean inter-particle distance of 242.6 nm, a volume fraction of 1.0% and a filling proportion of 16.3 wt% for the metal gold fine-particles relative to the nano-composite 2R-3; and
3) a volume fraction of 1.5% for the gold fine-particles in the nano-composite 2R-3 relative to the total volume of the voids in the nano-composite 2R-3:.

In addition, the reflection absorption spectrum of the LSPR of the metal gold fine-particles in the nano-composite 2R-3 was observed to have an absorption peak with a peak top at 588 nm, a half-height width of 183.1 nm, and an absorbance of 0.338 at the peak top, while the absorption spectrum in water was observed to have an absorption peak with a peak top at 625 nm, a half-height width of 241.6 nm, and an absorbance of 0.347 at the peak top. The peak wavelength variation and the peak intensity variation per unit variation of the refractive index of the observed absorption peak were 113.2 nm and 0.026, respectively.

[Reference Example 2-4]

Except that polyvinyl alcohol was not added, a gold complex-containing slurry 2R-4 was prepared in the same way as in Example 2-4. In the same way as in Example 2-4, the resulting gold complex-containing slurry 2R-4 was coated and dried, and then subjected to a heating treatment to fabricate a metal gold fine-particle-dispersed nano-composite 2R-4 of 1.53 μm thick that displayed a reddish purple color. The metal gold fine-particles formed in the nano-composite 2R-4 were dispersed entirely independently from each other in the region from the surface portion of the nano-composite 2R-4 along the thickness direction, with a distance equal to or greater than the particle diameter of the larger one of neighboring metal gold fine-particles. The characteristics of the nano-composite 2R-4 include:

1) a void proportion of 67.9%, a mean void size of 16 nm, and a maximal void size of 30 nm;
2) a shape of the metal gold fine-particles being substantially spherical, a mean particle diameter of 54.6 nm, a minimal particle diameter of 36.5 nm, a maximal particle diameter of 81.4 nm, a proportion of 100% for the particles having particle diameters of 1 to 100 nm, a mean inter-particle distance of 153.1 nm, and a volume fraction of 0.95% and a filling proportion of 16.3 wt% for the metal gold fine-particles relative to the nano-composite 2R-4; and
3) a volume fraction of 1.4% for the gold fine-particles in the nano-composite 2R-4 relative to the total volume of the voids in the nano-composite 2R-4:.

In addition, the reflection absorption spectrum of the LSPR of the metal gold fine-particles in the nano-composite 2R-4 was observed to have an absorption peak with a peak top at 553 nm, a half-height width of 107.8 nm, and an absorbance of 0.54 at the peak top, while the absorption spectrum in water was observed to have an absorption peak with a peak top at 584 nm, a half-height width of 142.9 nm, and an absorbance of 0.643 at the peak top. The peak wavelength variation and the peak intensity variation per unit variation of the refractive index of the observed absorption peak were 95.9 nm and 0.304, respectively.

[Reference Example 2-5]

Except that a polyvinyl alcohol was not added, a gold complex-containing slurry 2R-5 was prepared in the same way as in Example 2-5. In the same way as in Example 2-5, the resulting gold complex-containing slurry 2R-5 was coated and dried, and then subjected to a heating treatment to fabricate a metal gold fine-particle-dispersed nano-composite 2R-5 of 1.51 μm thick that displayed a reddish purple color. The metal gold fine-particles formed in the nano-composite 2R-5 were dispersed  entirely independently from each other in the region from the surface portion of the nano-composite 2R-5 along the thickness direction, with a distance equal to or greater than the particle diameter of the larger one of neighboring metal gold fine-particles. The characteristics of the nano-composite 2R-5 include:

1) a void proportion of 65.6%, a mean void size of 12 nm, and a maximal void size of 20 nm;
2) a shape of the metal gold fine-particles being substantially spherical, a mean particle diameter of 100.2 nm, a minimal particle diameter of 10.5 nm, a maximal particle diameter of 268.1 nm, a proportion of 57% for the particles having particle diameters of 1 to 100 nm, a mean inter-particle distance of 272.4 nm, and a volume fraction of 1.02% and a filling proportion of 16.3 wt% for the metal gold fine-particles relative to the nano-composite 2R-5; and
3) a volume fraction of 1.6% for the gold fine-particles in the nano-composite 2R-5 relative to the total volume of the voids in the nano-composite 2R-5.

In addition, the reflection absorption spectrum of the LSPR of the metal gold fine-particles in the nano-composite 2R-5 was observed to have an absorption peak with a peak top at 595 nm, a half-height width of 200.0 nm, and an absorbance of 0.456 at the peak top, while the absorption spectrum in water was observed to have an absorption peak with a peak top at 634 nm, a half-height width of 250.6 nm, and an absorbance of 0.484 at the peak top. The peak wavelength variation and the peak intensity variation per unit variation of the refractive index of the observed absorption peak were 122.2 nm and 0.087, respectively.

[Reference Example 2-6]

Except that a polyvinyl alcohol was not added, a gold complex-containing slurry 2R-6 was prepared in the same way as in Example 2-6. In the same way as in Example 2-6, the resulting gold complex-containing slurry 2R-6 was coated and dried, and then subjected to a heating treatment to fabricate a metal gold fine-particle-dispersed nano-composite 2R-6 of 1.34 μm thick that displayed a reddish purple color. The metal gold fine-particles formed in the nano-composite 2R-6 were dispersed entirely independently from each other in the region from the surface portion of the nano-composite 2R-6 along the thickness direction, with a distance equal to or greater than the particle diameter of the larger one of neighboring metal gold fine-particles. The characteristics of the nano-composite 2R-6 include:

1) a void proportion of 64.1%, a mean void size of 9 nm, and a maximal void size of 30 nm;

2) a shape of the metal gold fine-particles being substantially spherical, a mean particle diameter of 81.1 nm, a minimal particle diameter of 5.0 nm, a maximal particle diameter of 122.8 nm, a proportion of 50% for the particles having particle diameters of 1 to 100 nm, a mean inter-particle distance of 216.1 nm, and a volume fraction of 1.06% and a filling proportion of 16.3 wt% for the metal gold fine-particles relative to the nano-composite 2R-6; and

3) a volume fraction of 1.7% for the gold fine-particles in the nano-composite 2R-6 relative to the total volume of the voids in the nano-composite 2R-6.

In addition, the reflection absorption spectrum of the LSPR of the metal gold fine-particles in the nano-composite 2R-6 was observed to have an absorption peak with a peak top at 613 nm, a half-height width of 202.6 nm, and an absorbance of 0.615 at the peak top, while the absorption spectrum in water was observed to have an absorption peak with a peak top at 658 nm, a half-height width of 309.1 nm, and an absorbance of 0.638 at the peak top. The peak wavelength variation and the peak intensity variation per unit variation of the refractive index of the observed absorption peak were 137.8 nm and 0.09, respectively.

[Reference Example 2-7]

Except that polyvinyl alcohol was not added, a gold complex-containing slurry 2R-7 was prepared in the same way as in Example 2-7. In the same way as in Example 2-7, the resulting gold complex-containing slurry 2R-7 was coated and dried, and then subjected to a heating treatment to fabricate a metal gold fine-particle-dispersed nano-composite 2R-7 of 1.32 μm thick. The metal gold fine-particles formed in the nano-composite 2R-7 were dispersed entirely independently from each other in the region from the surface portion of the nano-composite 2R-7 along the thickness direction, with a distance equal to or greater than the particle diameter of the larger one of neighboring metal gold fine-particles. The characteristics of the nano-composite 2R-7 include:

1) a void proportion of 49.4%, a mean void size of 8 nm, and a maximal void size of 500 nm or more;
2) a shape of the metal gold fine-particles being substantially spherical, a mean particle diameter of 379.8 nm, a minimal particle diameter of 14.3 nm, a maximal particle diameter of 1077.3 nm, a proportion of 30% for the particles having particle diameters of 1 to 100 nm, a mean inter-particle distance of 862.2 nm, and a volume fraction of 1.5% and a filling proportion of 16.3 wt% for the metal gold fine-particles relative to the nano-composite 2R-7; and
3) a volume fraction of 3.0% for the gold fine-particles in the nano-composite 2R-7 relative to the total volume of the voids in the nano-composite 2R-7.

In addition, the reflection absorption spectrum of the LSPR of the metal gold fine-particles in the nano-composite 2R-7 was difficult to measure.

[Reference Example 2-8]

Except that a polyvinyl alcohol was not added, a gold complex-containing slurry 2R-8 was prepared in the same way as in Example 2-8. In the same way as in Example 2-8, the resulting gold complex-containing slurry 2R-8 was coated and dried, and then subjected to a heating treatment to fabricate a metal gold fine-particle-dispersed nano-composite 2R-8 of 1.45 μm thick. The nano-composite 2R-8 had a void proportion of 52.0%, an average void size of 8 nm, and a maximum void size of 500 nm or more. The metal gold fine-particles of the nano-composite 2R-8 include many aggregates so that the particle diameter thereof and the reflection absorption spectrum of the LSPR therefrom were difficult to measure.

[Reference Example 2-9]

Except that a polyvinyl alcohol was not added, a gold complex-containing slurry 2R-9 was prepared in the same way as in Example 2-9. In the same way as in Example 2-9, the resulting gold complex-containing slurry 2R-9 was coated and dried, and then subjected to a heating treatment to fabricate a metal gold fine-particle-dispersed nano-composite 2R-9 of 1.63 μm thick. The metal gold fine-particles formed in the nano-composite 2R-9 were dispersed entirely independently from each other in the region from the surface portion of the nano-composite 2R-9 along the thickness direction, with a distance equal to or greater than the particle diameter of the larger one of neighboring metal gold fine-particles. The characteristics of the nano-composite 2R-9 include:

1) a void proportion of 55.8%, a mean void size of 8 nm, and a maximal void size of 20 nm;
2) a shape of the metal gold fine-particles being substantially spherical, a mean particle diameter of 128.1 nm, a minimal particle diameter of 19.8 nm, a maximal particle diameter of 415.5 nm, a proportion of 71% for the particles having particle diameters of 1 to 100 nm, a mean inter-particle distance of 309.9 nm, and a volume fraction of 1.31 % and a filling proportion of 16.3 wt% for the metal gold fine-particles relative to the nano-composite 2R-9; and

3) a volume fraction of 2.4% for the gold fine-particles in the nano-composite 2R-9 relative to the total volume of the voids in the nano-composite 2R-9.

In addition, the reflection absorption spectrum of the LSPR of the metal gold fine-particles in the nano-composite 2R-9 was difficult to measure.

[Reference Example 2-10]

Except that a polyvinyl alcohol was not added, a gold complex-containing slurry 2R-10 was prepared in the same way as in Example 2-10. In the same way as in Example 2-10, the resulting gold complex-containing slurry 2R-10 was coated and dried, and then subjected to a heating treatment to fabricate a metal gold fine-particle-dispersed nano-composite 2R-10 of 1.73 $\mu$m thick. The metal gold fine-particles formed in the nano-composite 2R-10 were dispersed entirely independently from each other in the region from the surface portion of the nano-composite 2R-10 along the thickness direction, with a distance equal to or greater than the particle diameter of the larger one of neighboring metal gold fine-particles. The characteristics of the nano-composite 2R-10 include:

1) a void proportion of 53.7%, a mean void size of 24 nm, and a maximal void size of 500 nm or more;

2) a shape of the metal gold fine-particles being substantially spherical, a mean particle diameter of 65.4 nm, a minimal particle diameter of 8.4 nm, a maximal particle diameter of 176.8 nm, a proportion of 89% for the particles having particle diameters of 1 to 100 nm, a mean inter-particle distance of 154.9 nm, and a volume fraction of 1.37% and a filling proportion of 16.3 wt% for the metal gold fine-particles relative to the nano-composite 2R-10; and

3) a volume fraction of 2.6% for the gold fine-particles in the nano-composite 2R-10 relative to the total volume of the voids in the nano-composite 2R-10.

In addition, the reflection absorption spectrum of the LSPR of the metal gold fine-particles in the nano-composite 2R-10 was observed to have an absorption peak with a peak top at 590 nm, a half-height width of 206.5 nm, and an absorbance of 0.529 at the peak top, while the absorption spectrum in water was observed to have an absorption peak with a peak top at 599 nm, a half-height width of 231.2 nm, and an absorbance of 0.535 at the peak top. The peak wavelength variation and the peak intensity variation per unit variation of the refractive index of the observed absorption peak were 27.6 nm and 0.011, respectively.

As compared to Reference Examples 2-1 to 2-10, in Examples 2-1 to 2-10 where a polyvinyl alcohol was present at the heat-reduction, the particle diameters of the metal fine-particles in the nano-composites were decreased. Also, the reflection absorption spectrum of LSPR had a small half-height width and a sharp shape.

[Example 3-1]

To 1.2 g of boehmite powder (trade name: C-01, produced by Taimei Chemicals Co., Ltd., with a mean primary particle diameter of 20 nm, a mean secondary particle diameter of 0.1 $\mu$m, and a cubic particle shape), 2.84 g of water and 0.1 g of acetic acid were added, and a 5-min ultrasonic treatment was performed. Then, 3.96 g of ethanol, 1.6 g of a silane coupling agent aqueous solution (solid content concentration: 30 wt%) were added, and then a 5-min ultrasonic treatment was performed to prepare a slurry 3-1. The proportion of the solid content of the silane coupling agent in the slurry 3-1 at this moment was 40 weight parts relative to 100 weight parts of boehmite.

Next, the resulting slurry 3-1 was coated on a glass substrate of 0.7 mm thick using a spin coater (trade name: Spincoater 1H-DX2, made by Mikasa Co., Ltd.), dried at 70°C for 3 min and at 130°C for 10 min, and then subjected to a heating treatment at 280°C for 10 min to fabricate a substrate 3-1A of 1.8 $\mu$m thick. The pencil hardness of the coated surface of the substrate 3-1A was 6H.

Onto the coated surface of the resulting substrate 3-1A, a solution obtained by mixing and dissolving, in 2.5 g of ethanol, 0.25 g of chloroauric acid tetrahydrate and 0.25 g of a polyvinyl alcohol (with an average molecular weight of 22000, a polymerization degree of 500, and a saponification degree of 88%) dissolved in 2.25 g of pure water was coated using a spin coater (trade name: Spincoater 1H-DX2, made by Mikasa Co., Ltd.) at a rotation speed of 1000 rpm, dried at 70°C for 3 min and at 130°C for 10 min, and then subjected to heating treatments at 280°C for 10 min and at 500°C for 1 hour to fabricate a metal gold fine-particle dispersed nano-composite 3-1A that displayed an orange color. The metal gold fine-particles formed in the nano-composite 3-1A were dispersed entirely independently from each other in the region from the surface portion of the nano-composite 3-1A along the thickness direction, with a distance equal to or greater than the particle diameter of the larger one of neighboring metal gold fine-particles. The characteristics of the nano-composite 3-1A include:

1) a shape of the metal gold fine-particles being substantially spherical;

2) a mean particle diameter of 9.1 nm, a minimal particle diameter of 4.6 nm, and a maximal particle diameter of 25.5 nm;

3) a proportion of 100% for the particles having particle diameters of 1 to 100 nm;

4) a volume fraction of 0.73% and a filling proportion of 7.73 wt% for the metal gold fine-particles relative to the nano-composite 3-1A.

In addition, the reflection absorption spectrum of the LSPR of the metal gold fine-particles in the nano-composite 3-1A was observed to have an absorption peak with a peak top at 524 nm, a half-height width of 79 nm, and an absorbance of 0.807 at the peak top, while the absorption spectrum in water was observed to have an absorption peak with a peak top at 534 nm, a half-height width of 82 nm, and an absorbance of 0.966 at the peak top. The peak wavelength variation and the peak intensity variation per unit variation of the refractive index of the observed absorption peak were 30.3 nm and 0.479, respectively. Furthermore, when the nano-composite 3-1A was placed in the atmosphere, a change in the color of the surface of the nano-composite 3-1A on which water was dripped was clear and could be definitely confirmed by eyes.

[Example 3-2]

To 2.4 g of boehmite powder (trade name: C-01, produced by Taimei Chemicals Co., Ltd., with a mean primary particle diameter of 20 nm, a mean secondary particle diameter of 0.1 $\mu$m, and a cubic particle shape), 5.68 g of water and 0.2 g of acetic acid were added, and a 5-min ultrasonic treatment was performed. Then, 8.24 g of ethanol, 3.2 g of s silane coupling agent aqueous solution (solid content concentration: 30 wt%), and 0.672 g of concentrated hydrochloric acid were added, and then a 5-min ultrasonic treatment was performed to prepare a slurry 3-2. The proportion of the solid content of the silane coupling agent in the slurry 3-2 was 40 weight parts relative to 100 weight parts of boehmite.

Next, the resulting slurry 3-2 was coated on a glass substrate of 0.7 mm thick using a spin coater (trade name: Spincoater 1H-DX2, made by Mikasa Co., Ltd.), dried at 70°C for 3 min and at 130°C for 10 min, and then subjected to a heating treatment at 280°C for 10 min to fabricate a substrate 3-2A of 1.8 $\mu$m thick. The pencil hardness of the coated surface of the substrate 3-2A was 9H.

Onto the coated surface of the resulting substrate 3-2A, a solution obtained by mixing and dissolving, in 6 g of ethanol, 0.6 g of chloroauric acid tetrahydrate and 0.6 g of a polyvinyl alcohol (with an average molecular weight of 22000, a polymerization degree of 500, and a saponification degree of 88%) dissolved in 5.4 g of pure water was coated and dried in the same way as in Example 3-1, and then subjected to heating treatments at 280°C for 10 min and at 500°C for 1 hour to fabricate a metal gold fine-particle dispersed nano-composite 3-2A that displayed a red color. The metal gold fine-particles formed in the nano-composite 3-2A were dispersed entirely independently from each other in the region from the surface portion of the nano-composite 3-2A along the thickness direction, with a distance equal to or greater than the particle diameter of the larger one of neighboring metal gold fine-particles. The characteristics of the nano-composite 3-2A include:

1) a shape of the metal gold fine-particles being substantially spherical;

2) a mean particle diameter of 10.8 nm, a minimal particle diameter of 3.6 nm, and a maximal particle diameter of 55.2 nm;

3) a proportion of 100% for the particles having particle diameters of 1 to 100 nm;

4) a volume fraction of 0.61 % and a filling proportion of 6.46 wt% for the metal gold fine-particles relative to the nano-composite 3-2A.

In addition, the reflection absorption spectrum of the LSPR of the metal gold fine-particles in the nano-composite 3-2A was observed to have an absorption peak with a peak top at 526 nm, a half-height width of 76 nm, and an absorbance of 0.715 at the peak top, while the absorption spectrum in water was observed to have an absorption peak with a peak top at 541 nm, a half-height width of 86 nm, and an absorbance of 0.935 at the peak top. The peak wavelength variation and the peak intensity variation per unit variation of the refractive index of the observed absorption peak were 45.5 nm and 0.667, respectively. Further, when the nano-composite 3-2A was placed in the atmosphere, a change in the color of the surface of the nano-composite 3-2A on which water was dripped was clear and could be definitely confirmed visually.

[Reference Example 3-1]

To 1.2 g of boehmite powder (trade name: C-01, produced by Taimei Chemicals Co., Ltd., with a mean primary particle diameter of 20 nm, a mean secondary particle diameter of 0.1 $\mu$m, and a cubic particle shape), 2.84 g of water and 0.1 g of acetic acid were added, and a 5-min ultrasonic treatment was performed. Then, 3.96 g of ethanol, 1.6 g of a silane coupling agent aqueous solution (solid content concentration: 30 wt%), and 0.25 g of chloroauric acid tetrahydrate were added, and then a 5-min ultrasonic treatment was performed to prepare a slurry 3-3. The proportion of Au in the slurry 3-3 at this moment was 10 weight parts relative to 100 weight parts of boehmite.

Next, the resulting slurry 3 was coated on a glass substrate of 0.7 mm thick using a spin coater (trade name: Spincoater 1H-DX2, made by Mikasa Co., Ltd.), dried at 70°C for 3 min and at 130°C for 10 min, and then subjected to heat treatments at 280°C for 10 min and at 500°C for 1 hour to fabricate a metal gold fine-particle dispersed nano-composite 3-3A of 1.8 μm thick that displayed a reddish purple color. The metal gold fine-particles formed in the nano-composite 3-3A were dispersed entirely independently from each other in the region from the surface portion of the nano-composite 3-3A along the thickness direction, with a distance equal to or greater than the particle diameter of the larger one of neighboring metal gold fine-particles. The characteristics of the nano-composite 3-3A include:

1) a shape of the metal gold fine-particles being substantially spherical;
2) a mean particle diameter of 6.8 nm, a minimal particle diameter of 3.3 nm, and a maximal particle diameter of 17.5 nm;
3) a proportion of 100% for the particles having particle diameters of 1 to 100 nm;
4) a volume fraction of 0.63% and a filling proportion of 6.67 wt% for the metal gold fine-particles relative to the nano-composite 3-3A.

In addition, the reflection absorption spectrum of the LSPR of the metal gold fine-particles in the nano-composite 3-3A was observed to have an absorption peak with a peak top at 528 nm, a half-height width of 88 nm, and an absorbance of 0.589 at the peak top, while the absorption spectrum in water was observed to have an absorption peak with a peak top at 531 nm, a half-height width of 83 nm, and an absorbance of 0.530 at the peak top. The peak wavelength variation and the peak intensity variation per unit variation of the refractive index of the observed absorption peak were 9.1 nm and 0.179, respectively. Further, when the nano-composite 3-3A was placed in the atmosphere, a change in the color of the surface of the nano-composite 3-3A on which water was dripped was almost none and could not be confirmed visually.

[Reference Example 3-2]

Onto the coated surface of the substrate 3-1A obtained in Example 3-1, a solution obtained by dissolving 0.25 g of chloroauric acid tetrahydrate in 4.75 g of ethanol, which was a 5 wt% aqueous solution of chloroauric acid tetrahydrate, was coated in the same way as in Example 3-1, dried at 70°C for 3 min and at 130°C for 10 min, and then subjected to a heating treatment at 280°C for 10 min to fabricate a metal gold fine-particle dispersed nano-composite 3-4A that displayed a reddish purple color. The metal gold fine-particles formed in the nano-composite 3-4A were dispersed entirely independently from each other in the region from the surface portion of the nano-composite 3-4A along the thickness direction, with a distance equal to or greater than the particle diameter of the larger one of neighboring metal gold fine-particles. The characteristics of the nano-composite 3-4A include:

1) a shape of the metal gold fine-particles being substantially spherical;
2) a mean particle diameter of 7.8 nm, a minimal particle diameter of 3.0 nm, and a maximal particle diameter of 23.2 nm;
3) a proportion of 100% for the particles having particle diameters of 1 to 100 nm;
4) a volume fraction of 0.49% and a filling proportion of 5.19 wt% for the metal gold fine-particles relative to the nano-composite 3-4A.

In addition, the reflection absorption spectrum of the LSPR of the metal gold fine-particles in the nano-composite 3-4A was observed to have an absorption peak with a peak top at 542 nm, a half-height width of 102 nm, and an absorbance of 0.560 at the peak top, while the absorption spectrum in water was observed to have an absorption peak with a peak top at 547 nm, a half-height width of 105 nm, and an absorbance of 0.499 at the peak top. The peak wavelength variation and the peak intensity variation per unit variation of the refractive index of the observed absorption peak were 15.2 nm and 0.185, respectively. Further, when the nano-composite 3-4A was placed in the atmosphere, a change in the color of the surface of the nano-composite 3-4A on which water was dripped was almost none and could not be confirmed visually.

[Reference Example 3-3]

Onto the coated surface of the substrate 3-1A obtained in Example 3-1, a solution obtained by dissolving 0.5 g of chloroauric acid tetrahydrate in 4.5 g of ethanol, which was a 10 wt% aqueous solution of chloroauric acid tetrahydrate, was coated in the same way as in Example 3-1, dried at 70°C for 3 min and at 130°C for 10 min, and then subjected to a heating treatment at 280°C for 10 min to fabricate a metal gold fine-particle dispersed nano-composite 3-5A that displayer a reddish purple color. When the nano-composite 3-5A was placed in the atmosphere, a change in the color of the surface of the nano-composite 3-5A on which water was dripped was almost none and could be confirmed visually.

As compared to Reference Examples 3-1 to 3-3, in Examples 3-1 and 3-2 where a polyvinyl alcohol was present at the heat-reduction, the reflection absorption spectrum of LSPR had a large absorbance at the peak top, and the absorption spectrum was sharp.

Description of Reference Characters

1, 1': Matrix layer; 1a, 1a': Solid framework; 1b: Void; 3: Metal fine-particle; 10, 10A, 10B, 10C: Nano-composite; 11: Binding species

**Claims**

1. A metal fine-particle dispersed composite, comprising a matrix layer comprising a solid framework and voids defined by the solid framework, and metal fine-particles immobilized to the solid framework, and having the following features a to d:

   a) the solid framework containing an aluminum oxyhydroxide or an alumina hydrate and forming a three-dimensional network structure;
   b) the metal fine-particles having a mean particle diameter in a range of 3 to 100 nm, with a proportion of 60% or more having particle diameters in a range of 1 to 100 nm;
   c) the metal fine-particles being present in a manner that the metal fine-particles are not in contact with one another and neighboring metal fine-particles are apart from each other by a distance equal to or larger than the particle diameter of a larger one of the neighboring metal fine-particles;
   d) the metal fine-particles being dispersed three-dimensionally in the matrix layer, wherein each metal fine-particle has a portion exposed in the voids of the matrix layer.

2. The metal fine-particle dispersed composite of claim 1, wherein a void proportion is in a range of 15 to 95%.

3. The metal fine-particle dispersed composite of claim 1, wherein a volume fraction of the metal fine-particles relative to the metal fine-particle dispersed composite is in a range of 0.05 to 30%.

4. The metal fine-particle dispersed composite of claim 1, wherein the metal fine-particles comprise Au, Ag or Cu.

5. The metal fine-particle dispersed composite of claim 1, wherein the metal fine-particles generate a localized surface plasmon resonance when interacting with light of a wavelength of 380 nm or more.

6. The metal fine-particle dispersed composite of any one of claims 1 to 5, wherein a binding species having a functional group interacting with a specific substance is further immobilized on a surface of the metal fine-particles.

7. A localized surface plasmon resonance (LSPR) inducing substrate, comprising
   the metal fine-particle dispersed composite according to any one of claims 1 to 6; and
   a light reflecting member disposed on one side of the metal fine-particle dispersed composite.

8. The LSPR inducing substrate of claim 7, wherein
   the metal fine-particle dispersed composite comprises
   a first surface receiving light irradiated from a light source; and
   a second surface formed opposite to the first surface; and
   the light reflecting member is disposed connected to the second surface.

9. The LSPR inducing substrate of claim 7 or 8, wherein the light reflecting member comprises
   a light transmission layer; and
   a metal layer laminated on the light transmission layer.

10. The LSPR inducing substrate of any one of claims 7 to 9, wherein the light reflecting member further comprises a protection layer covering the metal layer.

**11.** The LSPR inducing substrate of claim 10, wherein the protection layer comprises a Ni-Cr alloy.

**12.** A method for fabricating a metal fine-particle dispersed composite, wherein the metal fine-particle dispersed composite comprises a matrix layer comprising a solid framework and voids defined by the solid framework, and metal fine-particles immobilized to the solid framework, the method comprising the following steps Ia to Id:

Ia) preparing a slurry containing an aluminum oxyhydroxide or an alumina hydrate for forming the solid framework;
Ib) mixing the slurry with a metal compound as a raw material of the metal fine-particles to prepare a coating liquid, wherein the metal compound has an amount, in terms of the metal element, in a range of 0.5 to 480 weight parts relative to 100 weight parts of a solid content of the slurry;
Ic) coating the coating liquid on a substrate and drying the coating liquid to form a coated film;
Id) subjecting the coated film to a heating treatment to form, from the coated film, the matrix layer comprising the solid framework having a three-dimensional network structure and voids defined by the solid framework, and simultaneously to heat-reduce a metal ion of the metal compound to precipitate particle-like metal as the metal fine-particles.

**13.** The method of claim 12, further comprising, after the step Id,

Ie) immobilizing, on a surface of the metal fine-particles, a binding species having a functional group interacting with a specific substance.

**14.** A method for fabricating a metal fine-particle dispersed composite, wherein the metal fine-particle dispersed composite comprises a matrix layer comprising a solid framework and voids defined by the solid framework, and metal fine-particles immobilized to the solid framework, the method comprising the following steps IIa to IId:

IIa) preparing a slurry containing an aluminum oxyhydroxide or an alumina hydrate for forming the solid framework;
IIb) coating the slurry on a substrate, drying and then subjecting the coated slurry to a heating treatment to form the matrix layer comprising the solid framework having a three-dimensional network structure and voids defined by the solid framework;
IIc) impregnating the matrix layer with a solution containing a metal ion as a raw material of the metal fine-particles, wherein the metal ion has an amount, in terms of the metal element, in a range of 0.5 to 480 weight parts relative to 100 weight parts of a solid content of the slurry;
IId) reducing the metal ion to precipitate particle-like metal as the metal fine-particles, through a heating treatment after the step IIc.

**15.** The method of claim 14, further comprising, after the step IId,
IIe) immobilizing, on a surface of the metal fine-particles, a binding species having a functional group interacting with a specific substance.

**16.** A method for fabricating a metal fine-particle dispersed composite, wherein the metal fine-particle dispersed composite comprises a matrix layer comprising a solid framework and voids defined by the solid framework, and metal fine-particles immobilized to the solid framework, the method comprising the following steps IIIa to IIId:

IIIa) preparing a slurry containing a metal hydroxide or a metal oxide as a raw material of the solid framework;
IIIb) mixing the slurry with a metal compound as a raw material of the metal fine-particles to prepare a coating liquid, wherein the metal compound has an amount, in terms of the metal element, in a range of 0.5 to 480 weight parts relative to 100 weight parts of a solid content of the slurry;
IIIc) coating the coating liquid on a substrate and drying the coating liquid to form a coated film; and
IIId) subjecting the coated film to a heating treatment to form, from the coated film, the matrix layer comprising the solid framework having a three-dimensional network structure and voids defined by the solid framework, and simultaneously to heat-reduce a metal ion of the metal compound to precipitate particle-like metal as the metal fine-particles, so as to obtain the metal fine-particle dispersed composite;

and being **characterized in that** the step IIId is performed in presence of a polyvinyl alcohol.

**17.** The method of claim 16, wherein the polyvinyl alcohol is added in the step IIIa of preparing the slurry.

**18.** The method of claim 16, wherein the polyvinyl alcohol is added in the step IIIb of preparing the coating liquid.

**19.** The method of any one of claims 16 to 18, wherein the polyvinyl alcohol is used in a range of 0.1 to 50 weight parts relative to 1 weight part of the metal compound.

**20.** The method of any one of claims 16 to 19, wherein the polyvinyl alcohol has a polymerization degree in a range of 10 to 5000.

**21.** The method of any one of claims 16 to 20, wherein the polyvinyl alcohol has a saponification degree of 30% or more.

**22.** The method of any one of claims 16 to 21, further comprising a step IIIe: heating the metal fine-particle dispersed composite at a temperature equal to or higher than a temperature at which thermal decomposition of the polyvinyl alcohol starts.

**23.** A metal fine-particle dispersed composite fabricated by the method of any one of claims 16 to 22.

**24.** A method for fabricating a metal fine-particle dispersed composite, wherein the metal fine-particle dispersed composite comprises a matrix layer comprising a solid framework and voids defined by the solid framework, and metal fine-particles immobilized to the solid framework, the method comprising the following steps IVa to IVd:

IVa) preparing a slurry containing a metal hydroxide or a metal oxide as a raw material of the solid framework;
IVb) coating the slurry on a substrate, drying and then subjecting the coated slurry to a heating treatment to form the matrix layer comprising the solid framework having a three-dimensional network structure and voids defined by the solid framework;
IVc) impregnating the matrix layer with a solution containing a metal ion as a raw material of the metal fine-particles, wherein the metal ion has an amount, in terms of the metal element, in a range of 0.2 to 1100 weight parts relative to 100 weight parts by of a solid content of the slurry; and
IVd) reducing the metal ion through a heating treatment after the step IVc to precipitate particle-like metal as the metal fine-particles;

and being **characterized in that** a polyvinyl alcohol is mixed in the solution containing the metal ion of the step IVc and the step IVd is performed in presence of a polyvinyl alcohol.

**25.** The method of claim 24, wherein the polyvinyl alcohol is used in a range of 0.1 to 50 weight parts relative to 1 weight part of a metal compound which is a raw material of the metal ion.

**26.** The method of claim 24 or 25, wherein the polyvinyl alcohol has a polymerization degree in a range of 10 to 5000.

**27.** The method of any one of claims 24 to 26, wherein the polyvinyl alcohol has a saponification degree of 30% or more.

**28.** The method of any one of claims 24 to 27, further comprising a step IVe: heating the metal fine-particle dispersed composite at a temperature equal to or higher than a temperature at which thermal decomposition of the polyvinyl alcohol starts.

**29.** The method of any one of claims 24 to 28, wherein the slurry contains a silane compound in a range of 10 to 200 weight parts relative to 100 weight parts of a solid content of the slurry.

**30.** A metal fine-particle dispersed composite fabricated by the method of any one of claims 24 to 29.

FIG. 1

FIG. 2

FIG. 3

FIG. 4

FIG. 5

FIG. 6

EP 2 674 744 A1

| Light source/Light receiving part |  40

FIG. 7

FIG. 8

FIG. 9

FIG. 10

FIG. 11

FIG. 12

FIG. 13

## INTERNATIONAL SEARCH REPORT

| | International application No. |
|---|---|
| | PCT/JP2012/052331 |

**A. CLASSIFICATION OF SUBJECT MATTER**
*G01N21/27*(2006.01)i, *B82Y30/00*(2011.01)i, *G01N21/01*(2006.01)i, *G01N33/532*
(2006.01)i, *G01N33/543*(2006.01)i

According to International Patent Classification (IPC) or to both national classification and IPC

**B. FIELDS SEARCHED**

Minimum documentation searched (classification system followed by classification symbols)
G01N21/00-21/74, G01N33/48-33/98, B22F1/00-8/00, B22F9/00-9/30,
C22C1/04-1/05, C22C33/02, G02B5/00-5/136

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
|---|---|---|
| X | WO 2010/140714 A1 (Sumitomo Chemical Co., Ltd.), | 1-5,12,14, 16-30 |
| Y | 09 December 2010 (09.12.2010), & JP 2011-68122 A & JP 2011-68982 A & WO 2010/140713 A1 | 6-11,13,15 |
| Y | JP 2005-049297 A (National Institute of Advanced Industrial Science and Technology), 24 February 2005 (24.02.2005), paragraphs [0010] to [0016], [0026] to [0035]; fig. 1 to 6 (Family: none) | 6-11,13,15 |

☒ Further documents are listed in the continuation of Box C. ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 20 April, 2012 (20.04.12) | 01 May, 2012 (01.05.12) |

| Name and mailing address of the ISA/ Japanese Patent Office | Authorized officer |
|---|---|
| Facsimile No. | Telephone No. |

Form PCT/ISA/210 (second sheet) (July 2009)

**INTERNATIONAL SEARCH REPORT**

International application No.

PCT/JP2012/052331

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2009-115492 A  (Canon Inc.),<br>28 May 2009 (28.05.2009),<br>paragraphs [0014] to [0019], [0056] to [0080];<br>fig. 1 to 6<br>& US 2010/0233825 A1     & WO 2009/057804 A1 | 6-11,13,15 |
| Y | JP 5-301319 A  (Mitsui Toatsu Chemicals, Inc.),<br>16 November 1993 (16.11.1993),<br>paragraph [0028]; fig. 1<br>(Family: none) | 10,11 |
| Y | JP 2006-173502 A  (Nikon Corp.),<br>29 June 2006 (29.06.2006),<br>paragraphs [0012], [0016] to [0017]; fig. 1<br>(Family: none) | 10,11 |
| A | WO 2010/134592 A1  (Konica Minolta Holdings, Inc.),<br>25 November 2010 (25.11.2010),<br>entire text; all drawings<br>(Family: none) | 1-30 |
| A | JP 2006-514286 A  (Intel Corp.),<br>27 April 2006 (27.04.2006),<br>entire text; all drawings<br>& US 2004/0161369 A1     & US 2004/0135997 A1<br>& EP 1595120 A           & WO 2004/074790 A1<br>& DE 60330809 D          & CN 1745291 A<br>& AT 453853 T            & AU 2003282750 A | 1-30 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000356587 A
- JP 2005171306 A
- JP 2008531447 PCT
- JP 4110930 PCT

**Non-patent literature cited in the description**

- **KUEI-JUNG CHAO et al.** Preparation and characterization of highly dispersed gold fine-particles within channels of mesoporous silica. *Catalysis Today,* 2004, vol. 97 (1), 49-53